# EUROPEAN PATENT SPECIFICATION

(11) **EP 3 040 340 B1**
(45) Date of publication and mention of the grant of the patent: **14.11.2018**
(21) Application number: 16155811.9
(22) Date of filing: 20.07.2004
(51) Int. Cl.: C07H 19/10, A61K 31/7068, A61P 35/00

(54) **CHEMICAL COMPOUNDS**
CHEMISCHE VERBINDUNGEN
COMPOSÉS CHIMIQUES

(30) Priority: 21.07.2003 GB 0317009
(43) Date of publication of application: 06.07.2016
(62) Divisional of application: 04743483.2
(73) Proprietor: NuCana plc, London EC4N 6AF (GB)
(72) Inventor: McGUIGAN, Christopher, Vale of Glamorgan Wales CF5 6LG (GB)
(74) Representative: HGF Limited

(56) References cited:
- US-A1- 2003 109 697
- ABRAHAM T W ET AL: "Synthesis and biological activity of aromatic amino acid phosphoramidates of 5-fluoro-2'-deoxyuridine and 1-beta-arabinofuranosylcytosine: evidence of phosphoramidase activity", JOURNAL OF MEDICINAL CHEMISTRY, AMERICAN CHEMICAL SOCIETY, US, vol. 39, 1 January 1996 (1996-01-01), pages 4569-4575, XP002147029, ISSN: 0022-2623, DOI: 10.1021/JM9603680

## Description

### Chemical Compounds

The present invention relates to nucleotide derivatives and their use in the treatment of cancer.

Nucleoside analogues such as fluorodeoxyuridine (1), cytarabine (2) and gemcitabine (3) are well established as anticancer agents. They function as inhibitors, of DNA synthesis after activation to their 5'-phosphate form.

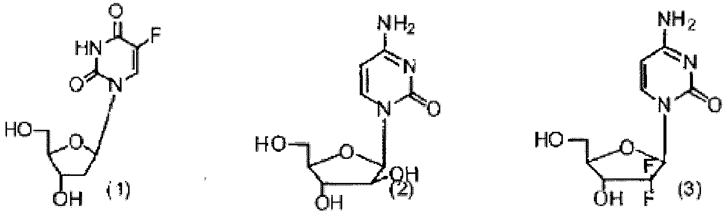

The free bioactive phosphate forms do not in general represent useful drugs due to their poor membrane permeation. In an effort to circumvent this a number of phosphate pro-drug approaches have been reported [Rosowsky et al, J. Med. Chem., 1982, 25, 171-8; Hong et al, J. Med. Chem., 1985, 28, 171-8; Kodama et al, Jpn. J. Cancer Res., 1989, 80, 679-85; Hong et al, 1979, 22, 1428-32; Ji et al, J. Med. Chem., 1990, 33, 2264-70; Jones et al, Nucleic Acids Res., 1989, 17, 7195-7201; Hunston et al, J. Med. Chem., 1984, 27, 440-4; Lorey et al, Nucleosides Nucleotides, 1997, 16, 1307-10; Farquhar et al, J. Med. Chem., 1983, 26, 1153-8; Shuto et al, Nucleosides Nucleotides, 1992, 11, 437-46; Le Bec et aI, Tet. Letts., 1991, 32, 6553-6; Phelps et al, J. Med. Chem., 1980, 23, 1229-32].

In general the phosphate prodrugs have biological properties and therapeutic activities that are similar to, or somewhat lower than, the parent nucleoside analogue.

We have carried out extensive work in this area from an antiviral perspective, largely on dideoxy nucleosides, and have reported a phosphoramidate approach which has been widely adopted for the delivery of bio-active phosphates of antiviral nucleosides.

An example is the phosphoramidate (4) derived from anti-HIV d4T (5).

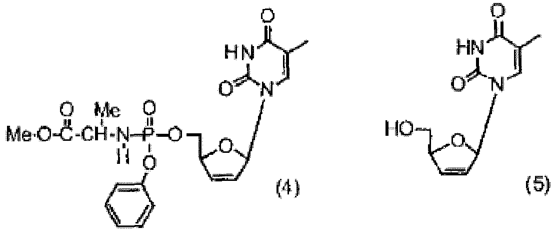

We observed the effect of variations in the ester [McGuigan et al,AVCC, 1998, 9, 473-9], amino acid [McGuigan et al, Antiviral Res., 1997, 35, 195-204; AVCC, 2000, 11, 111-6], and aryl [Siddiqui et al, J. Med. Chem., 1999, 42, 393-9] regions of the phosphoramidate, as well as the effect of amino acid stereochemistry [McGuigan et al, AVCC, 1996, 7, 184-8], phosphate stereochemistry [Allender et al, Analytica Chim. Acta, 2001, 435, 107-13] and nucleoside [Balzarini et al, BBRC, 1996, 225, 363-9; McGuigan et al, BioOrg. Med, Chem. Lett., 1996, 6, 2369-62; McGuigan et al, Bioorg. Med. Chem. Lett., 2000, 10, 645-7].

This work has lead to the optimal description of phenyl methoxyalaninyl phosphoramidate as the prototype pro-moiety for the intracellular delivery of bioactive nucleotides [Balzarini et al, PNAS, 1996, 93, 7295-9; McGuigan et al, J. Med. Chem., 1996, 39, 1748-53].

Lackey et al [Bioehem Pharmacol., 2001, 61, 179-89] have reported the application of our phosphoramidate pro-drug method for antiviral nucleosides to the anti-herpetic agent bromovinyl-2'-deoxyuridine (BVDU) (6). In particular, they have found that the phenyl methoxyalaninyl phosphoramidate (7) has significant anti-cancer activity. This is in marked contrast to the parent (antiviral) nucleoside (6).

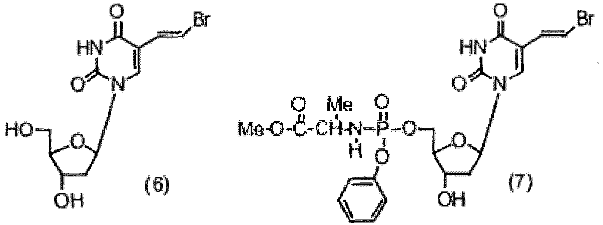

Limited SAR has been presented by this group, although in their patent applications [WO0239952, EP1200455, CA2317505, US6339151, EP116797, AU2451601] they claim a series of general variations in the base, and phosphate regions. However, based on our prior art, the phenyl methoxyalaninyl phosphoramidate (7) would be anticipated to be amongst the most optimal of structure.

Surprisingly, it has now been found that other derivatives of oxyamino acid-phosphoramidate nucleoside analogues are significantly more potent in the treatment of cancer than the phenyl methoxyalaninyl phosphoramidate (7).

US 2003/109697 relates to phosphoramidate derivatives of nucleotides and their use in treating cells or tissue involved in a pathology characterized by hyper-proliferative cells such as cancer, infectious disease, autoimmune disorders and inflammatory conditions/diseases.

ABRAHAM T. W. et. al.: "Synthesis and Biological Activity of Aromatic Amino Acid Phosphoramidates of 5-fluoro-2'-deoxyuridine and 1-β-Arabinofuranosylcytosine: Evidence of Phosphoramidase Activity", JOURNAL OF MEDICINAL CHEMISTRY, AMERICAN CHEMICAL SOCIETY, US, vol. 39, 1996, pages 4569-4575, XP002147029, ISSN: 0022-2623, DOI: 10.1021/JM9603680. This document relates to phosphoramidates of arabinofuranosylcytoside and FUdR for antitumor activity against L1210 mouse lymphocytic leukemia cells and CCRF-CEM human T-cell lymphoblastic leukaemia cells.

According to the first aspect of the present invention, there is provided a compound of formula I: 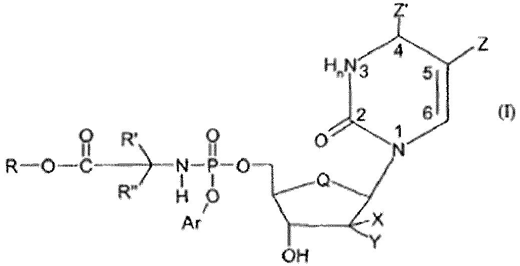 wherein:
R is selected from a C₁-C₁₆ branched or unbranched acyclic alkyl group, a C₃-C₁₂ cyclic alkyl group, a phenyl group or a naphthyl group and a benzyl group; and wherein R is unsubstituted;
R' and R" are independently selected from H, methyl (-CH₃), benzyl (-CH₂C₆H₅) and secondary butyl (-CH₂CH(CH₃)₂, or, R' and R" together with the C atom to which they are attached, provide a C₅₋₆ ring;
Q is -O-;
X and Y are both F;
Ar is naphthyl or phenyl; and one, two, three or four substituents, which may be the same or different, may be present on Ar and are selected from the group comprising halogen, - NO₂;-NH₂; -C₁₋₃alkyl; -C₁-C₃alkoxy; -SC₁₋₃alkyl; -CN; -COC₁₋₃alkyl; and -CO₂C₁₋₃alkyl; Z is H;
n is 0, Z' is -NH₂ and a double bond exists between position 3 and position 4; or a pharmaceutically acceptable salt, ester or salt of such ester of a compound on formula I.

Reference in the present specification to an alkyl group means a branched or unbranched, cyclic or acyclic, saturated or unsaturated (e.g. alkenyl or alkynyl) hydrocarbyl radical. Where cyclic, the alkylene group is preferably C₃ to C₁₂, more preferably C₅ to C₁₀, more preferably C₅ to C₇. Where acyclic, the alkyl group is preferably C₁ to C₁₆, more preferably C₁ to C₆.

Reference in the present specification to an aryl group means an aromatic group containing 5 to 14 ring atoms, for example phenyl or naphthyl. The aromatic group may be a heteroaromatic group containing one, two, three or four, preferably one, heteroatoms selected, independently, from the group consisting of O, N and S. Examples of such heteroaromatic groups include pyridyl, pyrrolyl, furanyl and thiophenyl. Preferably, the aryl group comprises phenyl or substituted phenyl.

The alkyl and aryl groups may be substituted or unsubstituted. Where substituted, there will generally be one to three substituents present, preferably one substituent. Substituents may include halogen atoms, by which is meant F, Cl, Br and I atoms, and halomethyl groups such as CF₃ and CCl₃; oxygen containing groups such as oxo, hydroxy, carboxy, carboxyC₁₋₆alkyl, alkoxy, alkoyl, alkoyloxy, aryloxy, aryloyl and aryloyloxy; nitrogen containing groups such as amino, C₁₋₆alkylamino, diC₁₋₆alkylamino, cyano, azide and nitro; sulphur containing groups such as thiol, C₁₋₆alkylthiol, sulphonyl and sulphoxide; heterocyclic groups which may themselves be substituted; alkyl groups as defined above, which may themselves be substituted; and aryl groups as defined above, which may themselves be substituted, such as phenyl and substituted phenyl. Substituents on said heterocyclic, alkyl and aryl groups are as defined immediately above.

Reference in the present specification to alkoxy and aryloxy groups means, respectively, alkyl-O- (for example where alkyl is C₁ to C₁₆, preferably C₁ to C₆) and aryl-O- (for example where aryl is a 5 to 14 membered aromatic mono- or bifused ring moiety, optionally containing 1, 2, 3 or 4 heteroatoms selected, independently, from O, S and N, preferably aryl is phenyl).

Reference in the present specification to alkoyl and aryloyl groups means, respectively, alkyl-CO- (for example where alkyl is C₁ to C₁₆, preferably C₁ to C₆) and aryl-CO- (for example where aryl is a 5 to 14 membered aromatic mono or bifused ring moiety, optionally containing 1, 2, 3 or 4 heteroatoms selected, independently, from O, S and N, preferably aryl is phenyl).

Reference in the present specification to alkoyloxy and aryloyloxy means, respectively, alkyl-CO-O (for example where alkyl is C₁ to C₁₆, preferably C₁ to C₆) and aryl-CO-O (for example where aryl is a 5 to 14 membered mono- or bifused aromatic ring system, optionally containing 1, 2, 3 or 4 heteroatoms selected, independently, from O, S and N, preferably aryl is phenyl).

Reference in the present specification to heterocyclic groups means groups containing one or more, pyrrolyl, imidazolyl, pyraziolyl, thiazolyl, isothiazolyl, oxazolyl, pyrrolidinyl, pyrrolinyl, imidazolidinyl, imidazolinyl, pyrazolidinyl, tetrahydrofuranyl, pyranyl, pyronly, pyridyl, pyrazinyl, pyridazinyl, piperidyl, piperazinyl, morpholinyl, thionaphthyl, benzofuranyl, isobenzofuryl, indolyl, oxyindolyl, isoindolyl, indazolyl, indolinyl, 7-azaindolyl, isoindazolyl, benzopyranyl, coumarinyl, isocoumarinyl, quinolyl, isoquinolyl, naphthridinyl, cinnolinyl, quinazolinyl, pyridopyridyl, benzoxazinyl, quinoxadinyl, chromenyl, chromanyl, isochromanyl and carbolinyl.

The group Ar comprises a substituted or unsubstituted aryl group, wherein the term "aryl group" and the possible substitution of said group is as defined herein. Preferably, Ar is a substituted or unsubstituted phenyl group. Particularly preferred substituents are electron withdrawing groups such as halogen (preferably chlorine or fluorine), trihalomethyl (preferably trifluoromethyl), cyano and nitro groups. For example, Ar can be phenyl, 3,5-dichloro-phenyl, p-trifluoromethyl-phenyl, p-cyano-phenyl, or p-nitro-phenyl.

Suitably, R is a C₁₋₁₆ primary or secondary alkyl group, a C₅₋₇ carbocyclic aryl group or a C₁₋₆alkylC₅₋₁₁aryl group. More suitably, R is a C₁₋₁₀ alkyl group, a phenyl group or C₁₋₃ alkylC₅₋₇ aryl group. Preferably R is unsubstituted.

Preferably, R is methyl (-CH₃), ethyl (-C₂H₅), *n-* or *i*- propyl (-C₃H₇), *n*- or *i-* butyl (-C₄H₉) or benzyl (-CH₂C₆H₅). Most preferably, R is benzyl. Particularly, R is preferably benzyl when one of R' and R" is H and one of R' and R" is methyl (-CH₃).

Alternatively, preferably, R' and R" are, independently, H, methyl (-CH₃), secondary butyl (-CH₂-CH-(CH₃)₂), benzyl (-CH₂C₆H₅), or, together with the C atom to which they are attached, provide a C₅₋₆ ring.

Preferred compounds include those where R' and R" are both methyl, one of R' and R" is H and one of R' and R" is methyl, and R' and R", together with the C atom to which they are attached, provide a pentyl ring.

When R' and R" arc different, the C atom to which they are attached is chiral. The present compounds can be L or D or a mixture of stereoisomers. Preferably they are L.

It will be appreciated that the moiety -O-C(O)-CR'R"-NH- corresponds to a carboxy-protected α-amino acid, R' and R" can thus correspond to the side chains of a naturally occurring amino acid.

For example, when one of R' and R" is H and one of R' and R" is Me or PhCH₂, the moiety corresponds to alanine or phenylalanine, respectively.

Preferably, the stereochemistry at the asymmetric centre -CR'R" corresponds to an L-amino acid. The stereochemistry at the asymmetric centre -CR'R" can, however, correspond to a D-amino acid, Alternatively, mixtures of of compounds can be employed having asymmetric centres corresponding to L and D amino acids.

In the present specification by "naturally occurring amino acid" we mean Alanine, Arginine, Asparagine, Aspartic Acid, Cysteine, Cystine, Glycine, Glutamic Acid, Glutamine, Histidine, Hydroxylysine, Hydroxyproline, Isoleucine, Leucine, Lysine, Methionine, Phenylalanine, Proline, Serine, Threonine, Tryptophan, Tyrosine and Valine.

The present invention is not, however, limited to compounds having a moiety corresponding to a naturally occurring amino acid. The present invention specifically includes compounds having a moiety which corresponds to a non-naturally occurring amino acid, such as, for example, those where R'=R"=alkyl, or, where together with the C atom to which they are attached, R' and R" provide a cyclic moiety. Preferably with respect to the compound of formula I, the moiety ROCOCR'R"NH- corresponds to or is derived from a non-naturally occurring amino acid.

Z is H, Q is O, n is 0, X and Y are each F, and thus the base moiety of the compound of formula I corresponds to that of gemcitabine i.e. compound (3) above.

Compounds of formula 1 are those wherein n is 0 and X and Y are F. Compounds of formula I are those wherein n is 0, X and Y are F, Q is O and Z is H, thus corresponding to phosphoramidated gemcitabine.

Preferably, Ar is an optionally substituted phenyl.

One, two, three or four substituents, which may be the same or different, may be present on Ar and are selected from the group comprising halogen, which may -F, -Cl, -Br or -I;-NO₂; -NH₂; optionally substituted -C₁₋₃alkyl; optionally substituted -C₁₋₃alkoxy, preferably methoxy (-OCH₃); optionally substituted -SC₁₋₃alkyl; -CN; optionally substituted -COC₁₋₃alkyl; and optionally substituted -CO₂C₁₋₃alkyl. The optional substitutents are one or more up to six, preferably three, members selected from the group comprising halogen which may be F, Cl, Br and I and NO₂. Preferred substituents on Ar include F, Cl, CF₃, and NO₂.

The substituents may be at any position on the ring moiety, Where the ring moiety is C₆ ie phenyl, a single substituent at the 2 (*ortho*) or 4 (*para*) position is preferred. Where Ar is phenyl, a single substituent at the 4 position is more preferred.

Preferably, Ar is an optionally substituted phenyl moiety. More preferably, Ar is selected form the group comprising: Ph-, *p*CF₃C₆H₄-, *p*FC₆H₄-, *p*NO₂C₆H₄-, *p*ClC₆H₄- and *o*ClC₆H₄-.

Surprisingly, modifying the ester moiety in compound (7) has been found to show a marked increase in potency with respect to cancer cell lines. A compound of the present disclosure is the benzyl ester (8). It has surprisingly been found that the benzyl ester (8) is very significantly more potent against several cancer cell lines than the methyl ester (7):

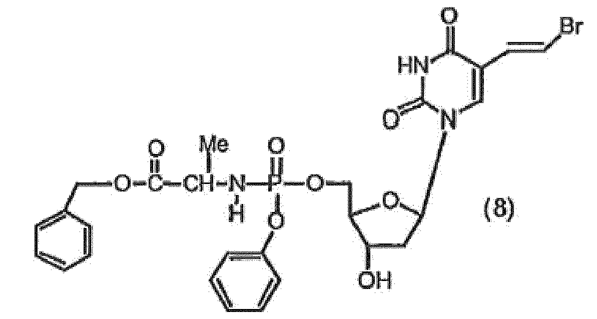

Compound (8) inhibits the growth of colon cancer cell line HT115 by 50% at 1.4 µM, whilst (7) requires a concentration of 244 µM; (8) is thus 174 times more potent. Compound (8) is also 8 times more potent than (7) versus prostate cancer cell line PC-3 (19 µM vs. 155 µM).

The degree of potency enhancement for (8) vs. (7) is surprising based on the prior art. Thus, comparing the equivalent phosphoramidates of d4T reveals a ca 4-fold potency boost of (10) over (9) [McGuigan et al, AVCC, 1998, 9, 473-9].

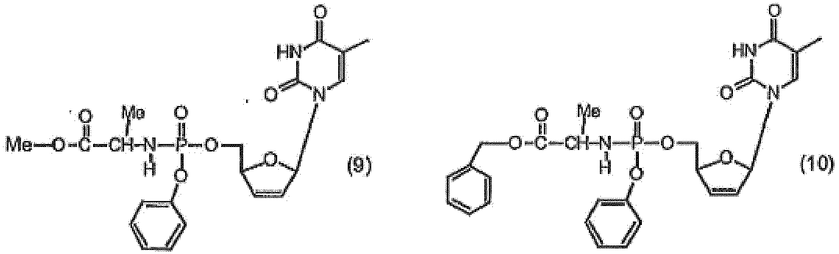

This would imply that the benzyl phosphoramidate motif in (10) is ca 4-fold more efficient at the intracellular delivery of the bio-active free phosphate forms of d4T than is the methyl ester (9). A person skilled in the art would anticipate a similar degree of enhancement for the benzyl phosphoramidate of BVDU (8) over the methyl ester (7) whilst we observed an almost 200-fold enhancement for colon cancer as noted above.

Surprising efficacy of modifications in the amino acid and aryl moieties of the BVDU phosporamidate has also been found in compounds of the present disclosure.

Thus, compound (11) has simultaneous modification in these two regions, being the p-trifluoromethylphenyl benzyl [α,α-dimethylglycinyl]phosphoramidate.

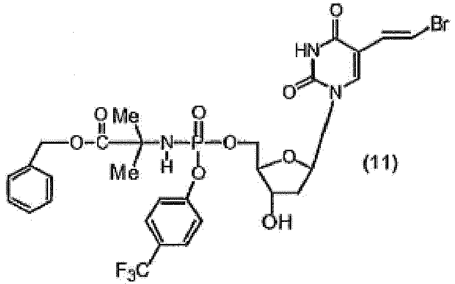

Compound 11 shows high potency against a range of cancer cell types and is significantly and surprisingly more potent than (7). Thus, for breast cancer (11) is 60-fold more active (1.3µM vs 79 µM), and for prostate cancer (11) is 254-fold more potent (0.61 µM vs. 155 µM). Against colon cancer, (11) is 35-fold more potent (7 µM vs 244 µM). Again, the degree of enhancement of the analogue (11) vs. (7) is surprising based on prior art. Thus, comparing (12) [dimethyl glycine modification] and (13) [p-CF₃phenyl modification] to (9) shows no significant difference in potency.

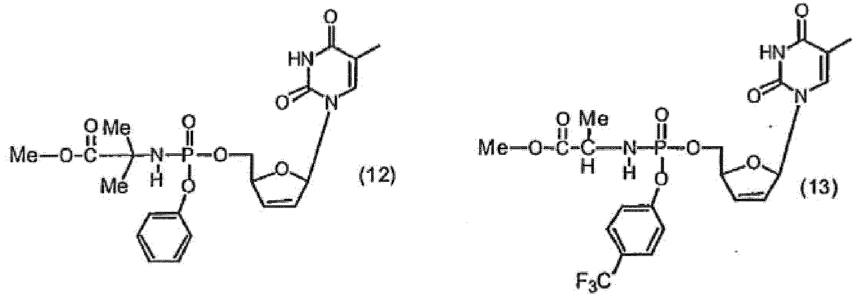

Thus 50% effective doses vs HIV-1 for (9), (12) and (13) are: 0.075, 0.29, and 0.01 µM respectively; within experimental error, (12) and (13) are identical in potency to (9). Thus a person skilled in the art would have predicted that (11) would show little enhancement over (7) as opposed to the 35 to 254-fold enhancements noted above.

Thus, compounds of the present disclosure and having variations in one or more of the ester (R), amino acid (R', R") and aryl (Ar) region of the phosphoramidate structure compared to phenyl methoxyalaninyl phosphoramidate can give surprising and substantial potency boosts of pro-tides derived from BVDU against a range of cancer cell types.

According to a further aspect of the present invention there is provided a compound having formula I according to the present invention for use in a method of treatment, preferably in the prophylaxis or treatment of cancer.

According to a further aspect of the present disclosure there is provided a method of phrophylaxis or treatment of cancer comprising administration to a patient in need of such treatment an effective dose of a compound having formula I according to the present invention.

According to a further aspect of the present disclosure there is provided use of a compound having formula I of the present invention in the manufacture of a medicament for use in the treatment or prophlylaxis of cancer.

According to a further aspect of the present invention there is provided a pharmaceutical composition comprising a compound having formula I of the present invention in combination with a pharmaceutically acceptable excipient, carrier or diluent.

According to a further aspect of the present invention there is provided a method of preparing a pharmaceutical composition comprising the step of combining a compound having formula I of the present invention with a pharmaceutically acceptable excipient, carrier or diluent.

The present invention is particularly applicable for the treatment of a patient having breast cancer, colon cancer or prostate cancer. Examples of such cancers include breast MDA MB231, colon HT115 and prostate PC-3.

The compound having formula I or pharmaceutical composition according to the present invention can be administered to a patient, which may be human or animal by any suitable means.

The medicaments employed in the present invention can be administered by oral or parenteral routes, including intravenous, intramuscular, intraperitoneal, subcutaneous, transdermal, airway (aerosol), rectal, vaginal and topical (including buccal and sublingual) administration.

For oral administration, the compounds of the invention will generally be provided in the form of tablets or capsules, as a powder or granules, or as an aqueous solution or suspension.

Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate, and lactose, while cornstarch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostcarate or glyceryl distearate, to delay absorption in the gastrointestinal tract.

Capsules for oral use include hard gelatin capsules in which the active ingredient is mixed with a solid diluent, and soft gelatin capsules wherein the active ingredient is mixed with water or an oil such as peanut oil, liquid paraffin or olive oil.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

For intramuscular, intraperitoneal, subcutaneous and intravenous use, the compounds of the invention will generally be provided in sterile aqueous solutions or suspensions, buffered to an appropriate pH and isotonicity. Suitable aqueous vehicles include Ringer's solution and isotonic sodium chloride. Aqueous suspensions according to the invention may include suspending agents such as cellulose derivatives, sodium alginate, polyvinylpyrrolidone and gum tragacanth, and a wetting agent such as lecithin. Suitable preservatives for aqueous suspensions include ethyl and n-propyl p-hydroxybenzoate.

The compounds of the invention may also be presented as liposome formulations.

In general a suitable dose will be in the range of 0.1 to 300 mg per kilogram body weight of the recipient per day. A preferred lower dose is 0.5 mg per kilogram body weight of recpient per day, a more preferred lower dose is 6 mg per kilogram body weight of recipient per day, an even more preferred lower dose is 10 mg per kilogram body weight per recipient per day. A suitable dose is preferably in the range of 6 to 150 mg per kilogram body weight per day, and most preferably in the range of 15 to 100 mg per kilogram body weight per day. The desired dose is preferably presented as two, three, four, five or six or more sub-doses administered at appropriate intervals throughout the day. These sub-doses may be administered in unit dosage forms, for example, containing 10 to 1500 mg, preferably 20 to 1000 mg, and most preferably 50 to 700 mg of active ingredient per unit dosage form.

According to a further aspect of the present invention there is provided a process for the preparation of a compound having formula I according to the present invention, the process comprising reacting of a compound of formula (III): 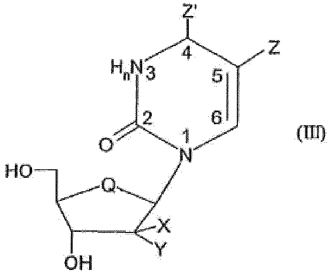 with a compound of formula (IV): 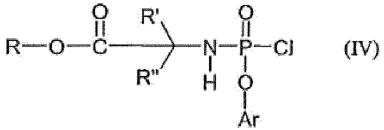 wherein Ar,n Q, R, R', R", X, Y, Z' and Z have the meanings described above with respect to formula (I).

Embodiments of the present invention will now be described, by way of example only, with reference to the following examples, experimental procedures and experimental data.

Data are presented for a range of structures against tumour cell types representing a range of common cancers in man with un-met clinical need: breast MDA MB231, colon HT115, prostate PC-3. Data from these assays are presented as Table I.

### Experimental Procedure

For the absence of doubt, compounds below labelled as "Illustrative Examples" do not form part of the invention.

### General methods

The following anhydrous solvents and reagents were bought from Aldrich with sure stopper: dichloromethane (DCM), diethyl ether (Et₂O), tetrahydrofuran THF), N-methylimidazole (NMI), methanol (MeOH), dimethylformamide (DMF), 1,4-dioxane. triethylamine was dried on molecular sieves of 4 Angstrom.

### Thin Layer Chromatography

Thin layer chromatography (TLC) was performed on commercially available Merck Kieselgel 60 F₂₅₄ plates and separated components were visualized using ultraviolet light (254 nm and 366 nm).

### Column Chromatography

Columns were performed using (Kieselgel 60, 35-70µm, Fluka) as the stationary phase, Samples were applied as a concentrated solution in the same eluent, or pre-adsorbed onto silica gel.

### NMR Spectroscopy

¹H, ¹³C and ³¹P-NMR were recorded on a Bruker Avance DPX300 spectrometer with operating frequencies of 300MHz, 75MHz and 121MHz respectively. ³¹P-MMR spectra are reported in units of δ relative to 85% phosphoric acid as external standard, positive shifts are downfield. The following abbreviations are used in the assignment of NMR signals: s (singlet), d (doublet), t (triplet), q (quartet), m (multiplet), bs (broad signal), dd (doublet of doublet), dt (doublet of triplet). Starred signal signal are splitted due to stereoisomeric mixtures.

### Standard procedures

For practical purposes, standard procedures are given where applicable.

### Standard procedure 1: Synthesis of Amino ester hydrochloride salts.

To a stirring solution of anhydrous alcohol (10 mol eq.) was added thionyl chloride (2 mol eq.) at 0° C, and the resulting solution stirred for 1 hr. After warming to room temperature, the appropriate amino acid (1 mol eq) was added and the reaction heated at reflux for 6-16 hrs. Removal of solvent and recrystallisation from methanol/ether gave the amino ester hydrochloride salts.

### Standard procedure 2: Synthesis of Amino benzyl ester hydrochloride salts.

The appropriate amino acid (1.0 mol eq.), *p*-toluene sulfonic acid (1.0 mol eq.) and anhydrous benzyl alcohol (4.1 mol eq.) were heated at reflux in toluene (10 mol eq.) with Dean-Stark trap for 24 hrs. On cooling to room temperature, Et₂O was added and the mixture was left in ice bath for 1hr then filtrated and washed with Et₂O. The solid was dissolved in DCM and washed with 10% K₂CO₃ and water, The organic layer was dried over MgSO₄, filtered and the solvent removed under reduced pressure to give an oil. This was solubilized in acetone and neutralized with 1 M HCl. Et₂O was added and the solid was filtered and washed with Et₂O to give a white solid.

### Standard procedure 3: Synthesis of Phosphorodichloridate species.

Phosphorus oxychloride (1.0 mol eq.) and the appropriate substituted phenol (1.0 mol) were stirred with anhydrous diethylether (31 mol eq.). To this was added anhydrous triethylamine (1.0 mol eq) at -80 °C and left to rise to room temperature over 16 hrs. the triethylamine hydrochloride salt was filtered off, and the filtrate reduced to dryness to give the crude product as a clear liquid.

### Standard procedure 4: Synthesis of Phosphochloridate species.

Phosphodichloridate (1.0 mol eq.) and the appropriate amino ester hydrochloric salt (1.0 mol eq.) were suspended in anhydrous DCM. Anhydrous triethylamine was added dropwise at -80 °C and after 1hr the reaction was left to rise to room temperature. The formation of phosphochloridate was monitored by ³¹P-NMR. After 2-5 hrs the solvent was removed under reduced pressure and the solid obtained washed with anhydrous ether (2x20 ml), filtered, and the filtrate reduced to dryness to give the products as crude oil. These oils were usually used without further purification.

### Standard procedure 5: Synthesis of Phosphoroamidate derivatives.

To a stirring solution of (E)-5-(2-bromovinyl)-2'-deoxyuridine (1.0 mol eq.) and the appropriate phosphochloridate (2.0- 3.0 mol eq) in anhydrous THF at -80°C was added dropwise over 1 min NMI (5.0 mol eq.). After 15 mins the reaction was left to rise to room temperature and stirred at room temperature for 2-19 hrs. The solvent was removed under reduced pressure and the yellow oil obtained was dissolved in DCM, washed with 0.5 M HCl, and water. The organic layer is dried over MgSO₄, filtered, reduced to dryness and purified by flash chromatography (Chloroform/Methanol 97/3, Dichloromethane/Methanol 97/3).

### Synthesis of Methyl-1-amino-1-cyclopentanoate hydrochloride salt.

**C₆H₁₄ClNO₃, MW=179.68.**

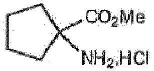

This was synthesised according to ***Standard Procedure 1***, using 1-amino-1-cyclopentanecarboxylic acid (3.876 g, 30 mmol) with thionyl chloride (4.44 mL, 45 mmol,) and anhydrous methanol (15.5 mL). The product was isolated as a white solid (4.81 g, yield 89%).
¹H-NMR (CDCl₃; 300 MHz): δ 9.1 (3H, bs, N*H₃*⁺Cl⁻), 3.85 (3H, s, OC*H₃*), 2.3-2.2 (4H, m, 4H cyclopentane), 2,15 (2H, 2H cyclopentane), 1.95 (2H, m, 2H cyclopentane),
¹³C-NMR (CDCl₃; 75 MHz): δ 26.6 (2CH₂ cyclopent), 38.1 (2CH₂ cyclopent), 54.8 (*C*H₃O), 66.6 (*Cq* cyclopentane), 174.1 (*C*OOMe).

### Synthesis of Ethyl-1-amino-1-cyclopentanoate hydrochloride salt.

**C₈H₁₆ClNO₂, MW=193.71.**

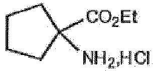

This was synthesised according to ***Standard Procedure 1***, using 1-amino-1-cyclopentanecarboxylic acid (5.0 g, 38.6 mmol) with thionyl chloride (5.72 mL, 58 mmol) and anhydrous ethanol (29 mL). The product was isolated as a white solid (6.98 g, yield 93%).
¹H-NMR (CDCl₃; 300 MHz): δ 9.0 (3H, bs, N*H₃*⁺Cl⁻), 4.3 (2H, q, ³*J*=8, OC*H₂*CH₃), 2.3-2.2 (4H, m, 4H cyclopentane), 2.15 (2H, 2H cyclopentane), 1.95 (2H, m, 2H cyclopentane), 1.4 (3H, t, ³*J*=8, OCH₂C*H₃*).
¹³C-NMR (CDCl₃; 75 MHz): δ 14.5 (*C*H₃CH₂), 25.8 (2CH₂ cyclopent), 37.4 (2CH₂ cyclopent), 63.0 (CH₃*C*H₂), 66.2 (*Cq* cyclopentane), 172.1 (*C*OOEt).

### Synthesis of Benzyl-1-amino-1-cyclopentanoate hydrochloride salt.

**C₁₄H₁₈ClNO₂, MW-255.78.**

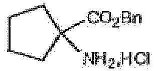

This was synthesised according to ***Standard Procedure 2***, using 1-amino-1-cyclopentanecarboxylic acid (3.682 g, 28.5 mmol) with *p*-toluene sulfonic acid monohydrate (5.625 g, 29.55 mmol) and anhydrous benzylic alcohol (12 mL, 116 mmol), in Toluene (20 mL). The product was isolated as a white solid (6.441 g, yield 88.5%) **Hydrochloride salt.** ¹H-NMR (CDCl₃; 300 MHz): δ 9.05 (3H, bs, N*H₃*⁺Cl⁻), 7.4-7.25 (5H, m, Ph), 5.15 (2H, s, CH₂*Ph*), 2.3 (4H, m, 4H cyclopentane), 2.15 (2H, 2H cyclopentane), 1.95 (2H, m, 2H cyclopentane).
¹³C-NMR (CDCl₃; 75 MHz): δ 25.9 (2CH₂ cyclopent), 37.3 (2CH₂ cyclopent), 66.3 (*Cq* cyclopentane), 68.3 (*C*H₂Ph), 129.2, 129.0, 128.8 ('*o*', '*m*', CH₂*Ph*), 135.5 ('*p*', CH₂*Ph*). 172.1 (*C*OOBn).

### Synthesis of methyl-2-amino-2-methylpropanoate hydrochloride salt

**C₅H₁₂ClNO₃, MW 153.61.**

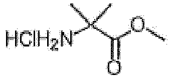

This was synthesised according to ***Standard Procedure 1***, using 2-amino-isobutyric acid (5.102 g, 48.49 mmol) with thionyl chloride (11.538 g, 96.98 mmol, 7.04 mL) and anhydrous methanol (19.6 mL). The product was isolated as a white solid (6.036 g, yield 89.2%).
¹H-NMR (CDCl₃; 300 MHz): δ 8,81 (3H, bs, N*H₃*Cl), 3.83 (3H, s, OC*H₃*), 1.74 (6H, s, [C*H₃*]₂C).
¹³C-NMR (CDCl₃; 75 MHz): δ 24.1, 24.3 ([*C*H₃]₂C), 57.9 (*C*[CH₃]₂), 172.4 (*C*OOCH₃).

### Synthesis of ethyl-2-amino-2-methylpropanoate hydrochloride salt.

**C₆H₁₄ClNO₂, MW 167.63.**

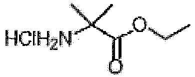

This was synthesized according to ***Standard Procedure 1***, using 2-amino-isobutyric acid (5.102 g, 48.49 mmol) with thionyl chloride (11.772 g, 98.95 mmol, 7.2 mL) and anhydrous ethanol (29 mL). The product was isolated as a white solid (7.159 g, yield 86.3%).
¹H-NMR (CDCl₃; 300 MHz): δ 8.93 (3H, bs, N*H₃*Cl), 4.3 (2H, q, ³*J*=7.1 Hz, OC*H₂*CH₃), 1.75 (6H, s, [C*H₃*]₂C), 1.33 (3H, t, ³*J*=7.1 Hz, OCH₂C*H₃*).
¹³C-NMR (CDCl₃; 75 MHz): δ 14.4 (*C*H₃CH₂O), 24.3 ([CH₃]₂C), 57.9 (*C*[CH₃]₂), 63.1 (O*C*H₂CH₃), 171.6 (*C*OOCH₂CH₃).

### Synthesis of benzyl-2-amino-2-methylpropanoate hydrochloride salt.

**C₁₁H₁₆ClNO₂, MW 229.70.**

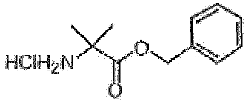

This was synthesised according to ***Standard Procedure 2***, using 2-amino-isobutyric acid (1.960 g, 19.00 mmol) with *p*-toluene sulfonic acid monohydrate (3.750g, 19.7 mmol) and benzylic alcohol (8.360 g, 77.30 mmol, 8 mL), in toluene (20 mL). The product was isolated as a white solid (2.556 g, yield 87.4%)
***p*-toluenesulfonate salt**: ¹H-NMR (CDCl₃, 300 MHz): δ 8.40 (3H, bs, N*H₃*Cl), 7.79 (2H, d, ³*J*=8.0 Hz, '*m*' *p*-TSA), 7.34 (5H, m, CH₂*Ph*), 7.14 (2H, d, ³*J*=8.0 Hz, '*o*' p-TSA), 5.16 (2H, s, C*H₂*Ph), 2.38 (3H, s, C*H₃ p*-TSA), 1.57 (6H, s, [C*H₃*]₂C)
   ¹³C-NMR (CDCl₃; 75 MHz): δ 21.8 (*C*H₃, *p*-TSA), 23.9 ([*C*H₃]₂C), 57.8 (*C*[CH₃]₂), 68.3 (*C*H₂Ph), 126.55, 128.5, 128.8, 129.0, 129.3 (CH₂*Ph*+*p*-TSA), 135.4 ('*ipso*', CH₂*Ph*), 140.8 ('*p*',*p*-TSA), 141.9 ('*ipso*',*p*-TSA), 171.9 (*C*OOCH₂Ph).
**Hydrochloride salt:** ¹H-HMR (CDCl₃; 300 MHz): δ 9.10 (3H, bs, N*H₃*Cl), 7.41-7.31 (5H, m, CH₂*Ph*), 5.27 (2H, s, CH₂*Ph*), 1.77 ([C*H*₃]₂C).
   ¹³C-NMR (CDCl₃; 75 MHz): δ 24.2 ([*C*H₃]₂C), 58.0 (*C*[CH₃]₂), 68.5 (*C*H₂Ph), 128.62, 129.0, 129.1 ('*o*', '*m*'*,* '*p*', CH₂*Ph*), 135.2 ('*ipso*', CH₂*Ph*), 171.8 (*C*OOCH₂Ph).

### Synthesis of illustrative example (E)-5-(2-bromovinyl)-2'-deoxyuridine

### (E)-5-(2-Carbomethoxyvinyl)-2'-deoxyuridine

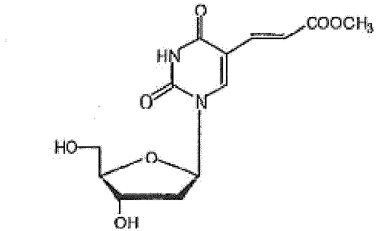

A mixture of Pd(OAc)₂ (0.316 g, 1.41 mmol), PPh₃ (0.741 g, 2.82 mmol), and triethylamine (4.9 mL) in 1,4-dioxane (50 mL) was stirred at 70°C until an intense red colour had developed, To this 5-iodo-2'-deoxyuridine (10 g, 28.24 mmol) and methylacrilate (4.862 g, 56.48 mmol, 5,1 mL) in 1,4-dioxane (20 mL) were added and the mixture stirred at refluxed for 30 mins. The reaction was filtered while still hot and the filtrate cooled over night at 4°C. The resulting pale yellow precipitate was filtered, washed with DCM and dried *in vacuo* to give the product as white solid (6.2 g, yield 70.7%).
¹H-NMR (DMSO-*d*₆; 300 MHz) δ 11.64 (1H, bs, N*H*-3), 8.42 (1H, s, H-6), 7.37 (1H, d, ³*J*=15.8 Hz, H vinylic), 6.86 (1H, d, ³*J*=15.8 Hz, H vinylic), 6.13 (1H, t, ³*J*=6.5 Hz, H-1'), 5.27-5.20 (2H, 2bs, OH-3', OH-5'), 4.27 (1H, m, H-3'), 3.81 (1H, m, H-4'), 3.68 (3H, s, C*H₃*), 3.60 (2H, m, H-5'), 2.18 (2H, m, H-2').
¹³C-NMR (DMSO-*d*₆; 75 MHz): δ 40.4 (C-2'), 51.6 (CH₃), 66.7 (C-5'), 70.0 (C-3'), 85.2 (C-4'), 88.0 (C-1'), 108.5 (C-5), 116.5 (C-5b), 138.5 (C-5a), 144.4 (C-6), 149.6, 162.1 (C-2, C-4), 167.6 (COO).

### Synthesis of illustrative example (E)-5-(2-Carboxyvinyl)-2'-deoxyuridine

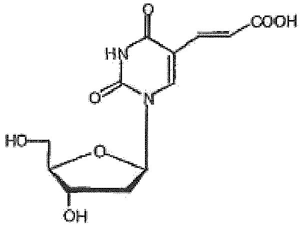

(E)-5-(2-carbomethoxyvinyl)-2'deoxyuridine (6.0 g, 19.33 mmol) was dissolved in 300 mL of 1 M NaOH and the mixture stirred at room temperature for 3 hrs, filtered and the filtrate adjusted to pH 2 with 1M HCl. On cooling at 4°C a white precipitate formed. This was filtered off and washed with cold water (2x 20 ml) and acetone (2x20 mL) and dred to give a white solid (4.441g, yield 77.1%).
¹H-NMR (DMSO-*d*₆; 300 MHz): δ 12.18 (1H, bs, CO₂H), 11.64 (1H,s, N*H*-3), 8.40 (1H, s, H-6),7.30(1*H*, d, ³*J*=15.6 Hz, Hvinylic), 6.78 (1H,d, ³*J*=15.8 Hz, H vinylic), 6.14 (1H, t, ³*J*=6.4 Hz, H-1'), 5.38 -5,08 (2H, bs, OH-3', OH-5'), 4.26 (1H, m, H-3'), 3.80 (1H, m, H-4'), 3.64 (2H, m, H-5'), 2.18 (2H, m, H-2').
¹³C-NMB (DMSO-*d*₆; 75 MHz): δ 40.1 (C-2'), 61.2 (C-5'), 70.1 (C-3'), 85.1 (C-4'), 88.0 (C-1'), 108.7 (C-5), 118.0 (C-5b), 137.9 (C-5a), 143.9 (C-6), 149.6, 162.1 (C-2, C-4), 168.4 (COOH).

### Synthesis of illustrative example (E)-5-(2-bromovinyl)-2'-deoxyuridine

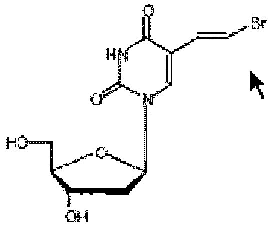

To a solution of (E)-5-(2-carboxyvinyl)-2'-deoxyuridine (5.777 g, 19.37 mmol) in dimethylforamide (29 mL) was added K₂CO₃ (5.890 g, 42,61 mmol) and the suspension stirred al room temperature for 15 mins. A solution of N-bromosuccinimide (3.655 g, 20.53 mmol) was added dropwise over 30 mins at 20°C. The resulting suspension was filtered and the solid washed with DMF. The combined filtrate and washings were evaporated to dryness *in vacuo* and the residue dissolved in MeOH. To this silica gel was added and the suspension evaporated to dryness and the solid applied to the top of chromatographic column. The column was eluted with chloroform/methanol 92/8 to give a white solid (5787g, 71.9%). Crystallisation from water gave a white powder.
¹H-NMR (DMSO-*d*₆ 300 MHz) δ 11.59 (1H, bs, NH-3), 8.08 (1H, s, H-6), 7.25 (1H, d, ³*J*=13.6 Hz, H-5b), 6.85 (1H, d, ³*J*=13.6 Hz, H-5a), 6.13 (1H, t, ³*J*=6.5 Hz, H-1'), 5.29 (1H, bs, OH-3'), 5.13 (1H, bs, OH-5'), 4.24 (1H, m, H-3'), 3.79 (1H, m, H-4'), 3.66 (2H, m, H-5'), 2.51 (1H, m, H-2'), 2.14 (1H, m, H-^{2'}).
¹³C-NMR (DMSO-*d*₆; 75 MHz): δ 40.2 (C-2'), 61.3 (C-5), 70.3 (C-4'), 84.8 (C-3'), 87.8 (C-1'), 108.9 (C-5b), 110,0 (C-5), 130.3 (C-5a), 149,6, 162.1 (C-2, C4).

### Synthesis of illustrative example (E)-5-(2-Bromovinyl)-2'-deoxyuridine-5'-[phenyl-(methoxy-L-alaninyl)]-phosphate (CPF 1).

**C₂₁H₂₅BrN₃O₉P, MW 574.32.**

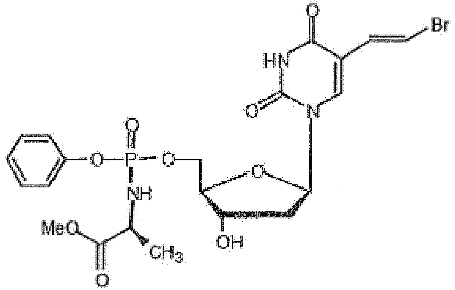

This was synthesised according to *Standard procedure* 5, using BVdU (300 mg, 0.90 mmol), Phenyl-(methoxy-L-alaninyl)-phosphorochloridate (472 mg, 1.7 mmol), NMI (4.5 mmol, 378 µL) in THF (9 mL) for 2 hrs. The crude product was purified by column chromatography, eluting with CH₂Cl₂/Methanol 97:3 to give the pure product as a white foamy solid (356 mg, yield 69%).
³¹P-NMR (CDCl₃, 121 MHz): δ 4.72, 4.40.
¹H-NMR (CDCl₃, 300 MHz): δ 9.9 (1H, bs, H-3), 7.64 (1H, 2xs, H-6),7,44-7.39 (1H, 2d, ³*J*=14 Hz, H-5b), 7.37-7.15 (5H, m, O*Ph*), 6.75-6.67 (1H, 2d, ³*J*=14 Hz, H-5a), 6.30-6.21 (1H, 2t, ³*J*=6 Hz, H1'), 4.57-4.29 (3H, m, H-5'+H-3'), 4.2-3.96 (3H, H-4', NH, CHala), 3.72 (3H, s, CH₃O), 2.49-2,40 (1H, m, one of H-2'), 2.12-2.01 (1H, m, one of H-2'), 1.38 (3H, d, ³*J*=7 Hz, CH_{3 ala}).
¹³C-NMR (DMSO; 75 MHz): δ 22.4 (CH_{3 ala}), 41,9, 41.8 (C-2'), 51.9 (*CH*[CH₃]), 54.3 (*C*H₃O), 67.5 (C-5'), 72.3, 71.9 (C-3'), 87.3, 87.2, 86.9, 86.8 (C-1',C-4'), 110.6 (C-5b), 113.1 (C-5), 121.7 ('*o*', *OPh),* 127.0 (*'p'*, *OPh*), 130.1 (C-5a), 131.5 ('*m*', *OPh*), 139.2 (C-6), 150.9 ('*ipso*', O*Ph*) 151.9 (C-4), 163.2(C-2), 175,7 (*C*OOCH₃),

### Synthesis of illustrative example (E)-5-(2-Bromovinyl)-2'-deoxyuridine-5'-[phenyl-(ethoxy-L-alaninyl)]-phosphate (CPF 3).

**C₂₂H₂₇BrN₃O₉P, MW=588.34.**

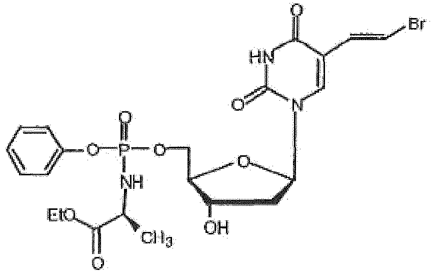

This was synthesised according to *Standard procedure* 5, using BVdU (150 mg, 0.45 mmol), Phenyl(ethoxy-L-alaninyl)-phosphorochloridate (249 mg, 0.9 mmol), NMI (2.8 mmol, 190 µL) in THF (4 mL) for 2 hrs. The crude product was purified by column chromatography, eluting with CH-₂Cl₂/Methanol 97:3 to give the pure product as a white foamy solid (145 mg, yield 55%).
³¹P-NMR (CDCl₃, 121 MHz): δ 4.48, 4.86.
¹H-NMR (CDCl₃, 300 MHz): δ 7.65 (1H, 2xs, H-6), 7.44-7.39 (1H, 2d, ³*J*=13 Hz, H-5b), 7.35-7.10 (5H, m, O*Ph*), 6.78-6.65 (1H, 2d, ³*J*=13 Hz, H-5a), 6.35-6.25 (1H, 2t, ³*J*=6 Hz, H1'), 4.62-3.95 (8H, m, H-5', H-3', H-4', CHala, NH, CH₃C*H₂*O), 2.49-2.40 (1H, m, one of H-2'), 2.10-2.00 (1H, m, one of H-2'), 1,40 (3H, d, ³*J*=7 Hz, CH_{3 ala}), 1.25 (3H, 2t, ³*J*=7 Hz, C*H*₃CH₂O).
¹³C-NMR (CDCl₃, 75 MHz): δ 14.5 (*C*H₃CH₂O) 21.2, 21.1 (CH₃ala), 40.9,40.7 (C-2'), 50.8, 50.7 (CHala), 62.2, 62.1 (CH₃*C*H₂O), 66.5, 66,3 (C-5'), 70,9,70.6 (C-3'), 86.0, 85.6 (C-1'. C-4'), 110.1 (C-5b), 111.8 (C-5), 120.6 ('o',*OPh*), 125,0) ('*p*', *OPh*), 129.0 (C-5a), 130.2 ('*m*', *OPh*), 138.2 (C-6), 149.9 (C-4), 150,7 ('*ipso*', O*Ph*), 162.3 (C-2), 174.2,174.1 (*C*OOCH₂CH₃).

### Synthesis of illustrative example (E)-5-(2-Bromovinyl)-2'-deoxyuridine-5'-[phenyl-(benzoxy-L-alaninyl)]-phosphate (CPF 2).

**C₂₇H₂₉BrN₃O₉P, MW=649.08.**

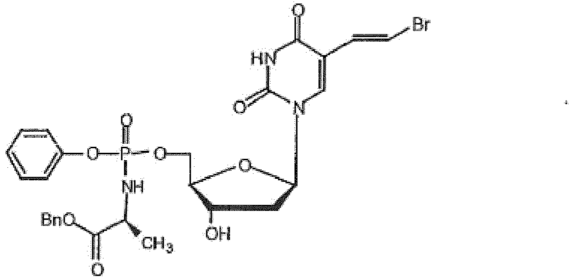

This was synthesized according to *Standard procedure* 5, using BVdU (150 mg, 0.45 mmol), Phenyl-(benzyloxy-L-alaninyl)-phosphorochloridate (249 mg, 0.9 mmol), NMI (2.8 mmol, 190 µL) in THF (5 mL) for 2 hrs. The crude product was purified by column chromatography, eluting with CH₂Cl₂/Methanol 97:3 to give the pure product as a white foamy solid (228 mg, yield 78%).
³¹P-NMR (CDCl₃, 121 MHz): δ 4,74, 4.44.
¹H-NMR (CDCl₃, 300 MHz): δ 10,31 (1H, bs, H-3), 7.63 (1H, 2xs, H-6), 7.45-7.14 (11H. m, O*Ph*+CH₂*Ph.* H-5b), 6.75-6.66 (1H, 2d, ³*J*=14 Hz, H-5a), 6.30-6.25 (1H, m, H-1'), 5.18-50.9 (1H, s, C*H*₂Ph), 4.70-4.04 (6H, m, H-3', H-5', H-4' N*H*, CHala), 2.42 (1H, m, one of H-2'), 2.02 (1H, m, one of H-2'), 1.40 (3H, d, ³*J*=7 Hz, C*H*₃ala).
¹³C-NMR (CDCl₃, 75 MHz): δ 20.7,20.8 (CH₃ala), 40.4 (C-2'), 50.4 (*C*Hala), 66.0 (C-5'), 67.4 (*C*H₂Ph), 70.6 (C-3'), 85.4, 85.5, 85.6, 85.8 (C-1', C-4'), 109.9(C-5b), 111.5 (C-5b), 120.2 ('o', O*Ph*), 125.4 ('*p*', *OPh*), 128.5, 128.6, 129.9 ('*m*' OPh, Bn, C-5a), 135.1 ('*ipso*', CH₂*Ph* 137.8 (C-6), 149.8 (C-4) 150.2 (*'ipso'*, *OPh*), 161.8 (C-2), 173.6 (*C*OOBn).

### Synthesis of illustrative example (E)-5-(2-Bromovinyl)-2'-deoxyuridine-5'-[para-fluorophenyl-(methoxy-L-alaninyl)]-phosphate (CPF 5).

**C₂₁H₂₄BrFN₃O₉P, MW=592.31.**

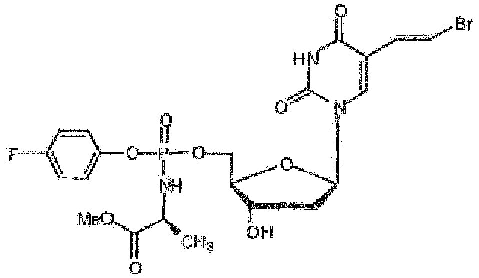

This was synthesised according to *Standard procedure* 5, using BVdU (200mg, 0.60 mmol), para-fluorophenyl-(methoxy-L-alaninyl)-phosphorochloridate (442 mg, 1.5 mmol), NMI (4.98 mmol, 332 µL) in THF (5 mL) for 2 hrs. The crude product was purified by column chromatography, eluting with CH₂Cl₂/Methanol 97:3 to give the pure product as a white foamy solid (177 mg,yield 50%),
³¹P-NMR (CDCl₃, 121 MHz): δ 5.10, 4.81.
¹H-NMR (CDCl₃; 300 MHz): δ 10.1 (1H, bs, H-3), 7.60 (1H, 2xs, H-6), 7.39-7.32 (1H, 2d, ³*J*=14 Hz, H-5b), 7.20-6,95 (4H, m, O*Ph*), 6.70-6.60 (1H,2d, ³*J*=14 Hz, H-5a), 6.30-6.15 (1H, 2t, ³*J*=6 Hz, H1'), 4.55-4.29 (3H, m, H-5'+H-3'), 4.15 (1H, NH), 4.05-3.85 (2H, H-4', CHala), 3.72 (3H, 2s, CH₃O), 2.49-232 (1H, m, one of H-2'), 2.15-2.05 (1H, m, one of H-2'), 1.35 (3H, 2d, ³*J*=6 Hz, CH_{3 ala}).
¹³C-NMR (DMSO; 75 MHz); δ 21.2 (CH_{3 ala}, 40.8 (C-2'), 50.8, 50,6 (*CH*[CH₃]), 53.2 (*C*H₃O), 66.7, 66.3 (C-5'), 71.9, 71.8 (C-3'), 86',1, 85.7, 85.8 (C-1', C-4'), 110.3 (C-5b), 111.9 (C-5), 117.0, 116.7 ('*o',* O*Ph*), 122.0 ('*m*', O*Ph*), 128.2 (C-5a), 138.2 (C-6), 149.0 ('*ipso*', O*Ph*) 149.9 (C-4), 158.5 ('p',*OPh*), 163.2(C-2), 175.1 (*C*OOCH₃).

### Synthesis of illustrative example (E)-5-(2-Bromovinyl)-2'-deoxyuridine-5'-[para-fluorophenyl-(ethoxy-L-alaninyl)]-phosphate (CPF 6).

**C₂₂H₂₆BrFN₃O₉P, MW-606.33.**

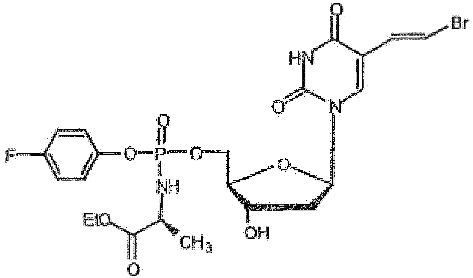

This was synthesised according to *Standard procedure* 5, using BVdU (200 mg, 0.60 mmol), para-fluorophenyl-(ethoxy-L-alaninyl)-phosphorochloridate (464 mg, 1.5 mmol), NMI (4.98 mmol, 332 µL) in THF (5 mL) for 2 hrs. The crude product was purified by column chromatography, eluting with CH₂Cl₂/Methanol 97:3 to give the pure product as a white foamy solid (240 mg, yield 66%).
³¹P-NMR (CDCl₃ 121 MHz): δ 5.14, 4.88,
¹H-NMR (CDCl₃, 300 MHz): δ 10.25 (1H, bs, H-3), 7.85 (1H, 2xs, H-6), 7.44-7.39 (1H, 2d, ³*J*=14 Hz, H-5b),7.3-7.0 (4H, m, *OPh*), 6.8-6.65 (1H, 2d, ³*J*=14 Hz, H-5a), 6.35-6.25 (1H, 2t, ³*J*=6 Hz, H1'), 4.6-4.1 (6H, m, H-5', H-3', CHala, NH, CH₃C*H*₂O), 4.02 (1H, m, H-4'), 2.55-2.45 (1H, m, one of H-2'), 2.20-2.10 (1H, m, one of H-2'), 1.40 (3H, d, ³*J*=8 Hz, CH₃ₐₗₐ), 1.25 (3H, 2t, ³*J*=7 Hz, C*H*₃CH₂O).
¹³C-NMR (CDCl₃, 75 MHz): δ 14.5 (*C*H₂CH₂O) 21.3 (CH₃ala), 40.8,40.7 (C-2'), 50.8, 50.7 (CHala), 62.3 (CH₃CH₂O), 66,7. 66.3 (C-5'), 71.1, 70.7 (C-3'), 86.1, 85.8, 85.6, 85.4 (C-1', C-4'), 1104 (C-5b), 111.9 (C-5), 117.0 ('o', O*Ph*). 122.2 '*m*', *OPh*), 128.9 (C-5a), 138.2 (C-6), 146,4 (*'ipso',* O*Ph*), 149.9 (C-4), 158.5 ('*p*', *OPh*), 162.2, 161.8 (C-2), 174.2 (*C*OOCH₂CH₃).

### Synthesis of illustrative example (E)-5-(2-Bromovinyl)-2'-deoxyuridine-5'-[para-fluorophenyl-(benzoxy-L-alaninyl)]-phosphate (CPF 7).

**C₂₇H₂₈BrFN₃O₉P, MW=668.40.**

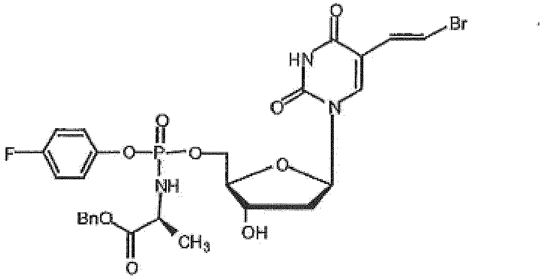

This was synthesised according to *Standard procedure* 5, using BVdU (200 mg, 0.60 mmol), para-fluorophenyl-(benzyloxy-L-alaninyl)-phosphorochloridate (556 mg, 1.5 mmol), NMI (4.98 mmol, 332 µL) in THF (5 mL) for 2 hrs. The crude product was purified by column chromatography, eluting with CH₂Cl₂/Methanol 97:3 to give the pure product as a white foamy solid (256 mg, yield 64%).
³¹P-NMR (CDCl₃, 121 MHz): δ 4.74,4.44.
¹H-NMR (CDCl₃, 300 MHz): δ 7.69 (1H, 2xs, H-6), 7.45-7.39 (1H, 2d, ³*J*=14 Hz, H-5b), 7.37-7.00 (9H. m, *OPh*+*C*H₂*Ph*), 6,75-6.65 (1H, 2d, ³*J*=14 Hz, H-5a), 6.30-6.2 (1H, 2t, ³*J*=6Hz, H-1'), 5.2 (1H, 2s, C*H*₂Ph), 4.85-4.00 (6H, m, H-3',H-5',H-4', N*H*, CHala), 2.47 (1H, m, one of H-2'), 2.0-2.15 (1H, m, one of H-2'), 1.38 (3H, d, ³*J*=7 Hz, CH₃ala).
¹³C-NMR (CDCl₃, 75 MHz): δ 21.2, 21.1 (CH₃ala), 40.7 (C-2'), 50.4 (*C*Hala), 66.7, 66.4 (C-5'), 67.8 (*C*H₂Ph), 71.1, 70.7 (C-3'), 86.0, 85.7, 85.4, 85.3 (C-1', C-4'), 110.4 (C-5b), 111,9 (C-5), 117,0 ('ο', O*Ph*), 122,0 (*'m'*, *O-Ph*), 128.1, 128.6 (Bn, C-5a), 135.4('*ipso*', CH₂*Ph*) 138.2 (C-6), 146.5 ('*ipso*', O*Ph*), 149.9 (C-4), 158.5 '*p*' OPh), 162.2 (C-2), 173.9 (*C*OOBn).

### Synthesis of illustrative example (E)-5-(2-Bromovinyl)-2'-deoxyuridine-5'-[para-nitrophenyl-(methoxy-L-alaninyl)]-phosphate (CPF 10).

**C₂₁H₂₄BrN₄O₁₁P, MW=619.31.**

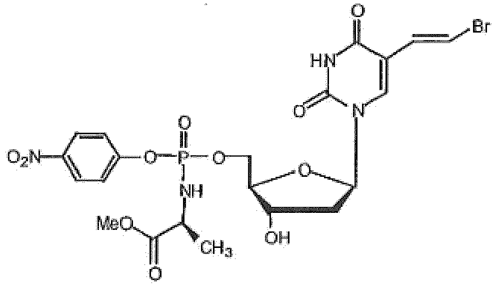

This was synthesised according to ***Standard procedure 5***, using BVdU (200 mg, 0.60 mmol), para-nitrophenyl-(methoxy-L-alaninyl)-phosphorochloridate (483 mg, 1.5 mmol), NMI (4.98 mmol, 332 µL) in THF (5 mL) for 2 hrs. The crude product was purified by column chromatography, eluting with CH₂Cl₂/Methanol 97:3 to give the pure product as a white foamy solid (211 mg, yield 57%).
³¹P-NMR (CDCl₃, 121 MHz): δ 4.95.
¹H-NMR (MeOD; 300 MHz): δ 8.3-8.2 (2H, m, O*Ph*) 7.8-7.75 (1H, 2xs, H-6), 7.35-7.30, 7.55-7.4 (2H, m, O*Ph*), 7.35-7.30 (1H, 2d, ³*J*=14 Hz, H-5b), 6.80-6.70 (1H, 2d, ³*J*=14 Hz, H-5a), 6.30-6.2 (1H, 2t, ³*J*=6 Hz, H1'), 4.5-4.3 (3H, m, H-5',H-3'), 4.2-4.0 (2H, m, H-4', CHala), 3.72 (3H, 2s, CH₃O), 2.35-2.15 (2H, m, 2 H-2'), 1.35 (3H, 2d, ³*J*=7Hz, CH₃ₐₗₐ).
¹³C-NMR (DMSO; 75 MHz): δ 20.9 (CH₃ₐₗₐ), 41.6, 41.5 (C-2'), 52.0, 51.9 (*CH*[CH₃]), 53.4 (*C*H₃O), 68.5 (C-5'), 72,4, 72.3 (C-3'), 87.7, 87.4, 87.0, 86.9 (C-1', C-4'), 109.8 (C-5b), 112.8 (C-5), 122.6 ('*o*', O*Ph*), 127.1 ('*m*', O*Ph*), 130.8 (C-5a), 140.3 (C-6), 146.5 ('*ipso*', O*Ph*), 151.4 (C-4), 157.2 ('*p*', O*Ph*), 163,9 (C-2), 175.8,175.5 (*C*OOCH₃).

### Synthesis of illustrative example (E)-5-(2-Bromovinyl)-2'-deoxyuridine-5'-[para-nitrophenyl-(ethoxy-L-alaninyl)]-phosphate (CPF 9).

**C₂₂H₂₆BrN₄O₁₁P, MW=633.34.**

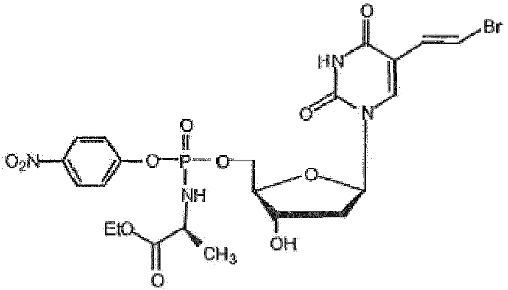

This was synthesised according to. ***Standard procedure 5***, using BVdU (200 mg, 0,60 mmol), para-nitrophenyl-(ethoxy-L-alaninyl)-phosphorochloridate (504 mg, 1.5 mmol), NMI (4.98 mmol, 332 µL) in THF (5 mL) for 1 hr. The crude product was purified by column chromatography, eluting with CH₂Cl₂/Methanol 97:3 to give the pure product as a white foamy solid (232 mg, yield: 61%).
³¹P-NMR (CDCl₃, 121 MHz): 6 4.28.
¹H-NMR (CDCl₃, 300 MHz): δ 10.25 (1H, bs, H-3), 8,25-8.2 (2H, 2d, ³*J*=9Hz O*Ph*), 7.7 (1H, 2xs, H-6), 7.5-7.45 (2H, 2d , ³*J*=9Hz, O*Ph*), 7.4-7.35 (1H, 2d, ³*J*=14 Hz, H-5b), 6.7-6.65 (1H, 2d, ³*J*=14 Hz, H-5a), 6.3-6.2 (1H, 2t, ³*J*=6 Hz, H1'), 4.8-4.1 (7H, m, H-5', H-4' H-3', CHala, NH, CH₃C*H*₂O), 2.45-2.4 (1H, m, one of H-2'), 2.20-2.10 (1H, m, one of H-2'), 1.40 (3H, d, ³*J*=8 Hz, CH₃ₐₗₐ), 1.3 (3H, 2t, ³*J*=7 Hz, C*H*₃CH₂O).
¹³C-NMR (CDCl₃, 75 MHz): δ 14.5 (*C*H₃CH₂O) 21.1 (CH₃ala), 40.6 (C-2'), 50.8, 50.7 (CHala), 62.5 (CH₃*C*H₂O), 66.9, 66.8 (C-5'), 71.2, 70.9 (C-3'), 86.3, 85.9, 85.4, 85.3 (C-1', C-4'), 110.3 (C-5b), 111.8 (C-5), 121.3 ('o', O*Ph*), 126.1 ('*m*', O*Ph*), 128.8 (C-5a), 138.4 (C-6), 145.1 ('*ipso*)', O*Ph*), 149.9 (C-4), 155.5 ('*p*', O*Ph*), 162.3 (C-2), 174.0, 173,9 (*C*OOCH₂CH₃).

### Synthesis of illustrative example (E)-5-(2-Bromovinyl)-2'-deoxyuridine-5'-[para-nitrophenyl-(benzoxy-L-alaninyl)]-phosphate (CPF 8).

**C₂₇H₂₈BrN₄O₁₁P, MW=695.41.**

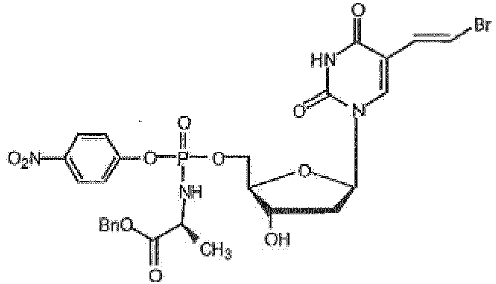

This was synthesized according to ***Standard procedure 5***, using BVdU (200 mg, 0.60 mmol), para-nitrophenyl-(benzyloxy-L-alaninyl)-phosphorochloridate (597 mg, 1.5 mmol), NMI (4.98 mmol, 332 µL) in THF (5 mL) for 2 hrs. The crude product was purified by column chromatography, cluting with CH₂Cl₂/Methanol 97:3 to give the pure product as a white foamy solid (228 mg, yield 55%).
³¹P-NMR (CDCl₃, 121 MHz): δ 4,74, 4.44.
¹H-NMR (CDCl₃, 300 MHz): δ 10.4-10.3 (1H, bs, H-3), 8.2-8.1 (2H, m, O*Ph*), 7.69 (1H, 2xs, H-6), 7.4-7.2 (1H, 2d, ³*J*=14 Hz, H-5b), 7.37-7.00 (7H, m, O*Ph*+CH₂*Ph*), 6.75-6.65 (1H, 2d, ³*J*=14 Hz,H-5a), 6.25-6.15 (1H, 2t, ³*J*=6Hz, H-1'), 5.2 (1H, d, C*H₂*Ph), 4.87 (1H, m, H-3'), 4.6-4.2 (3H, m, H-5', CHala) 4.2-4.00 (2H, m, H-4', N*H*,), 2.55-2.45 (1H, m, one of H-2'), 2.2-2.05 (1H, m, one of H-2'), 1.38 (3H, d, ³*J*=7 Hz, CH₃ala).
¹³C-NMR (CDCl₃, 75 MHz): δ 21.2, 21.1 (CH₃ala), 40.6 (C-2'), 50.9 (*C*Hala), 67.1, 67.0 (C-5'), 68.0 (*C*H₂Ph), 71.3, 70.9 (C-3'), 86.3, 86.0, 85.3, 85.2 (C-1', C-4'), 110.4 (C-5b), 111.9, 111.8 (C-5), 121.3 ('ο', O*Ph*), 126.2-126.1 ('*m*', O*Ph*), 129.1, 128.7, 128.6 (Bn, C-5a), 135.4 ('*ipso*', CH₂*Ph*), 138.3 (C-6), 145.1 ('*ipso*', O*Ph*), 149.9 (C-4), 155.6 ('*p*' OPh), 162.2 (C-2), 173.8,173.7 (*C*OOBn).

### Synthesis of illustrative example (E)-5-(2-bromovinyl)-2'-deoxyuridine-5'-[para-(trifluoromethyl)-phenyl-(methoxy-L-alaninyl)]-phosphate (CPF 15).

**C₂₂H₂₄BrF₃N₃O₉, MW=642.31.**

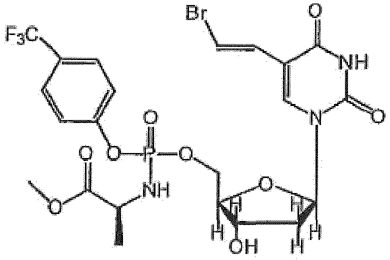

This was synthesized according to ***Standard procedure 5***, using BVdU (200 mg, 0.60 mmol), phenyl-(methoxy-L-alaninyl)-phosphorochloridale (518.8 mg, 1.5 mmol), NMI (246.3 mg, 3.0 mmol, 239 µL) in THF (5 mL) for 4 hrs. The crude product was purified by column chromatography, cluting with chloroform/methanol 97:3 to give the pure product as a white foamy solid (211.1 mg, yield 34,7%).
³¹P-NMR (MeOD, 121 MHz): δ 5.23, 5.07.
¹H-NMR (MeOD, 300 MHz): δ 7.80 (1H, s, H-6), 7.70 (2H, d, ³*J*=8.7 Hz, O*Ph*), 7.47-7.42 (2H, m, O*Ph*), 7.37 (1H, d, ³*J*=13.6 Hz, H-5b), 6.82-6.78 (1H, d, ³*J*=13.6 Hz, H-5a), 6.30-6.23 (1H, m, H-1'), 4.52-4.29 (3H, m, H-3'+H-5'), 4.17-4.13 (1H, m, H-4'), 4.05-3.91 (1H, m, C*H*CH₃), 3.67 (3H, s, O*CH₃*), 2.35-2.32 (1H, m, one of H-2'), 2.23-2.16 (1H, m, one of H-2'), 1.37-1.34 (3H, d, ³*J*=7.1 Hz, CH*CH₃*).
¹³C-NMR (MeOD, 75 MHz): δ 20.6, 20.7, 20.8, 20,9 (CH*C*H₃), 41.5, 41.7 (C-2'), 51.9, 52.0 (*C*HCH₃), 68.2, 68.3 (C-5'), 72.4, 72.5 (C-3'), 87.1, 87.2, 87.4, 87.6 (C-1', C-4'), 109.7 (C-5b), 112.6 (C-5), 122.5, 122.7 ('*o*', O*Ph*), 125.8 (*C*F₃, *J*=269 Hz), 128.7 ('*m*', O*Ph*), 128.8 ('*p*', *J*=33 Hz, O*Ph*), 130.9 (C-5a), 140.3 (C-6), 151.4, 151.5 ('*ipso*', O*Ph*), 155.1, 155.2 (C-4), 164.0 (C-2), 175.6, 175.9, (*C*OOCH₃).

### Synthesis of illustrative example (E)-5-(2-bromovinyl)-2'-deoxyuridine-5'-[para-(trifluoromethyl)-phenyl-(ethoxy-L-alaninyl)]-phosphate (CPF 25).

**C₂₃H₂₆BrF₃N₃O₉P, MW=656.34**

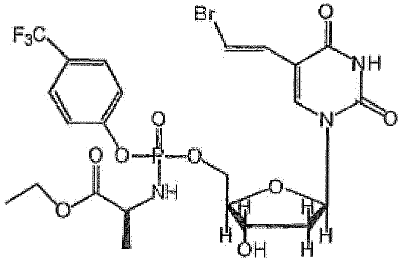

This was synthesised according to ***Standard procedure 5***, using BVdU (200 mg, 0.60 mmol), phenyl-(ethoxy-L-alaninyl)-phosphorochloridate (539.5 mg, 1,5 mmol), NMI (246.3 mg, 3.0 mmol, 239 µL) in THF (5 mL) for 20 hrs. The crude product was purified by column chromatography, eluting with dichloromethane/methanol 95:5 to give the pure product as a white foamy solid (172.6 mg, yield 43.8%).
³¹P-NMR (CDCl₃, 121 MHz): δ 4.65, 4.35.
¹H-NMR (CDCl₃, 300 MHz); δ 10.05 (1H, s, H-3), 7.69-7.64 (3H, m, H-6+O*Ph*), 7.46-7.39 (3H, m, O*Ph*+ H-5b), 6.70-6.68 (1H, 2d, ³*J*=13.6 Hz, H-5a), 6.34-6.25 (1H, m, H-1'), 4.57-4.35 (4H, m, H-3'+H-5'+N*H*), 4.27-4.13 (4H, m, H-4'+OC*H₂*CH₃+OH-3'), 4.12-3,98 (1H, m, C*H*CH₃), 2.53-2.47 (1H, m, one of H-2'), 2.21-2.12 (1H, m, one of H-2'), 1.43-1.40 (3H, d, ³*J*=7.0 Hz, CH*CH₃*), 1.28, 1.27 (3H, 2t, ³*J*=7.0 Hz, OCH₂C*H₃*)
¹³C-NMR (CDCl₃, 75 MHz): δ 14.5 (*C*H₃CH₂O), 21.2, 21.3 (CH*C*H₃), 40.7 (C-2'), 50.8, 50.9 (*C*HCH₃), 62.4 (CH₃*C*H₂O), 66.3, 66.7 (C-5'), 70.7, 71.1 (C-3'), 85.3, 85.4, 85.8, 86.1 (C-1', C-4'), 110.5 (C-5b), 112.0 (C-5), 122.0 ('*o*', O*Ph*), 124.2 (*C*F₃, *J*=271 Hz), 127.7, 127.8, 128.7 ('*m'*, '*p*', O*Ph*), 128.8 (C-5a), 138.0 (C6), 149.7 ('*ipso*', O*Ph*), 153.2 (C-4), 161.9 (C-2), 174.0, 174.1 (*C*OOCH₂CH₃).

### Synthesis of illustrative example (E)-5-(2-Bromovinyl)-2'-deoxyuridine-5'-[para-trifluoromethyl-(benzoxy-L-alaninyl)]-phosphate (CPF 4).

**C₂₈H₂₈BrF₃N₃O₉P, MW=718.41.**

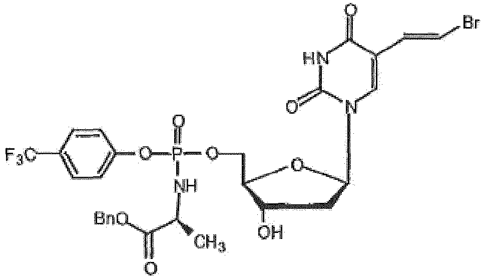

This was synthesised according to ***Standard procedure 5**,* using BVdU (200 mg, 0.60 mmol), para-trifluorophenyl-(benzyloxy-L-alaninyl)-phosphorochloridate (632 mg, 1.5 mmοl), NMI (4.98 mmol, 332 µL) in THF (6 mL) for 2 hrs. The crude product was purified by column chromatography, cluting with CH₂Cl₂/Methanol 97:3 to give the pure product as a white foamy solid (308 mg, yield 71%).
³¹P-NMR (CDCl₃, 121 MHz): δ 5.31, 4.87.
¹H-NMR (CDCl₃, 300 MHz): δ 10.05 (1H, bs, H-3), 7.7, 7.25 (11H. m, H-5b, H-6 O*Ph*+CH₂*Ph*), 6.75-6.65 (1H, 2d, ³*J*=14 Hz, H-5a), 6.35-6.2 (1H, 2t, ³*J*=6Hz, H-1'), 5.15 (1H, 2s, *CH₂*Ph), 4.6-4.25 (4H, m, H-5', H-3', CHala) 4.2-4.00 (2H, m, H-4', N*H*,), 2.55-2.4 (1H, m, one of H-2'), 2.2-2.05 (1H, m, one of H-2'), 1.38 (3H, d, ³*J*=7 Hz, CH₃ala).
¹³C-NMR (CDCl₃, 75 MHz): δ 21.2, 21.1 (CH₃ala), 40.7 (C-2'), 50.9, 50.8 (*C*Hala), 67.1, 67.0 (C-5'), 68.0(*C*H₂Ph), 71.2, 10.9 (C-3'), 86.1, 85.8, 85.5, 85.4 (C-1', C-4'), 110.2 (C-5b), 111.9, 111.8 (C-5), 121.1 ('o', O*Ph*), 125.1 (d, J=270Hz, CF₃), 127.6 ('*m*', O*Ph*), 129.1, 128.7, 128,6 (Bn, C-5a), 130.1 ('*p*',q, J=32Hz, O*Ph*) 135.4 ('*ipso*', CH₂*Ph*) 138.2 (C-6), 150.2, 150.1 (C-4), 153.6 ('*ipso*' OPh), 162.7 (C-2), 173.9, 173.6 (*C*OOBn).

### Synthesis of illustrative example (E)-5-(2-bromovinyl)-2'-deoxyuridine-5'-[4-chlorophenyl-(methoxy-L-alaninyl)]-phosphate (CPF 13).

**C₂₁H₂₄BrCIN₃O₉P, MW=608.76.**

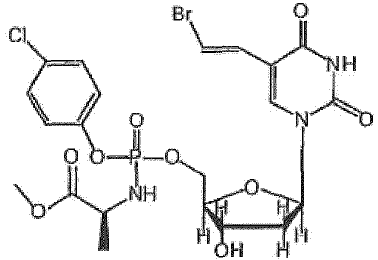

This was synthesised according to ***Standard procedure 5***, using BVdU (200 mg, 0.60 mmol), 4-chlorophenyl-(methoxy-L-alaninyl)-phosphorochloridate (374.5 mg, 1.2 mmol), NMI (246.3 mg, 3.0 mmol, 239 µL) in THF (8 mL) for 5 hrs. The crude product was purified by column chromatography, eluting with Chloroform/Methanol 97:3 to give the pure product as a white foamy solid (139.0 mg, yield 38.0%).
³¹P-NMR (CDCl₃, 121 MHz): δ 4.81, 4.54.
¹H-NMR (CDCl₃, 300 MHz): δ 10.11 (1H, bs, H-3), 7.68 (1H, s, H-6), 7.46-7.40 (1H, d, ³*J*=13.6 Hz, H-5b), 7.35-7.20 (4H, m, O*Ph*), 6.76-6.67 (1H, 2d, ³*J*=13.6 Hz, H-5a), 6.34-6,24 (1H, m, H-1'), 4.58-4.40 (5H, m, H-3'+H-5'+N*H*), 4.36-4.19 (1H, m, H-4'), 4.07-3.99 (1H, m, C*H*CH₃), 3.75 (3H, s, OC*H₃*), 2.49-2.48 (1H, m, one of H-2'), 2.17-2.15 (1H, m, one of H-2'), 1.42-1.39 (3H, d, ³*J*=7.0 Hz, CH*CH₃*).
¹³C-NMR (CDCl₃, 75 MHz): δ 21.2 (CH*C*H₃), 40.7, 40.8 (C-2'), 50.6, 50.8 (*C*HCH₃), 53.2, 53.3 (OCH₃), 66.4, 66.7 (C-5'), 70.8, 71.2 (C-3'), 85.4, 85.5, 85.8, 86.2 (C-1', C-4'), 110.5 (C-5b), 111.9, 112.0 (C-5), 122.0 ('*o*', O*Ph*), 128.9 (C-5a), 130.3 ('*m*', O*Ph*), 131.1 ('*p*', O*Ph*), 138.2 (C-6), 149.1, 149.2 ('*ipso*', O*Ph*), 149.8 (C-4), 162.1, 162.2 (C-2), 174.5, 174.6 (*C*OOCH₃).

### Synthesis of illustrative example (E)-5-(2-bromovinyl)-2'-deoxyuridine-5'-[4-chlorophenyl-(ethoxy-L-alaninyl)]-phosphate (CPF 11).

C₂₂H₂₆BN₃O₉P, MW=622.79.

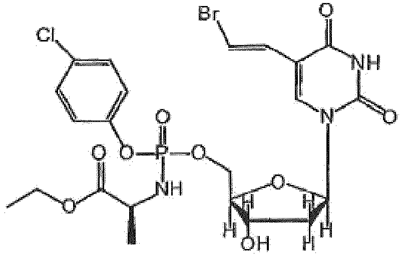

This was synthesised according to ***Standard procedure 5*,** using BVdU (300 mg, 0.90 mmol), 4-chlorophenyl-(ethoxy-L-alaninyl)-phosphorochloridate (557.7 mg, 1.71 mmol), NMI (221.7 mg, 2.7 mmol, 215 µL) in THF (10 mL) for 16 hrs. The crude product was purified by column chromatography, eluting with dichloramethane/methanol 97:3 to give the pure product as a white foamy solid (168.4 mg, yield 30.0%).
³¹P-NMR (CDCl₃, 121 MHz): δ 4.88, 4.65.
¹H-NMR (CDCl₃, 300 MHz): δ 9.51 (1H, bs, H-3), 7.69-7,68 (1H, 2s, H-6), 7.49-7.43 (1H, 2d, ³*J*=13.6 Hz, H-5h), 7.37-7.22 (4H, m, O*Ph*), 6.79-6.71 (1H, 2d, ³*J*=13.6 Hz, H-5a), 6.33-6.24 (1H, m, H-1'), 4,62-4.34 (3H, m, H-3'+H-5'), 4.28-3.89 (5,H, m, H-4'+*OCH₂*CH₃+C*H*CH*₃*+NH), 2.59-2.45 (1H, m, one of H-2'), 2.22-2.14 (1H, m, one of H-2'), 1.43-1.41 (3H, d, ³*J*=7,0 Hz., CHC*H*₃), 1.33-1.28 (3H, 2t, ³*J*=7.2 Hz, OCH₂C*H*₃)
¹³C-NMR (CDCl₃, 75 MHz): δ 14.5 (*C*H₃CH₂O), 21.2, 21.3 (CH*C*H₃), 40.7 (C-2'), 50.7, 50.8 (*C*HCH₃), 62.4(CH₃*C*H₂O), 66.7 (C-5'), 70.8, 71.2 (C-3'), 85.4, 85.8, 86.1 (C-1', C-4'), 110.4 (C-5b), 112.0 (C-5), 122.0, 122.1 ('*o*', O*Ph*), 128.9 (C-5a), 130.3 ('*m*', O*Ph*), 131.1 ('*p*', O*Ph*), 138.2 (C-6), 149.2 ('*ipso*', O*Ph*), 150.0 (C-4), 162.2 (C-2), 174.1, 174.2 (*C*OOCH₂CH₃).

### Synthesis of illustrative example (E)-5-(2-bromovinyl)-2'-deoxyuridine-5'-[4-chlorophenyl-(benzoxy-L-alaninyl)] -phosphate (CPF 12).

C₂₂H₂₆BrN₃O₉P, MW=622.79.

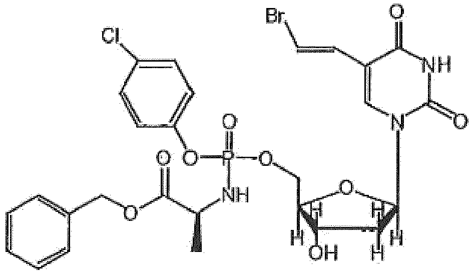

This was synthesised according to ***Standard procedure 5*,** using BVdU (300 mg, 0.90 mmol), 4-chlorophenyl-(berizoxy-L-alaninyl)-phosphorochloridate (698.7 mg, 1.80 mmol), NMI (369,5 mg, 4.5 mmol, 358.7 µL) in THF (10 mL) for 2 hrs. The crude product was purified by column chromatography, eluting with dichloromethane/methanol 95:5 to give the pure product as a white foamy solid (310.0 mg, yield 50.3%).
³¹P-NMR (CDCl₃, 121 MHz): δ 4.81, 4.53.
¹H-NMR (CDCl₃, 300 MHz): δ 10.10 (1H, bs, H-3), 7.65-7.63 (1H, 2s, H-6), 7.69-7.68 (1H, 2s, H-6), 7.46, 7.41 (1H, 2d, ³*J*=13.6 Hz, H-5b), 7.40-7.17 (9H, m, O*Ph*), 6.75-6.66 (1H, 2d, ³*J*=13.6 Hz, H-5a), 6.33-6,23 (1H, 2t, ³*J*=6.0 Hz, H-1'), 5.17 (2H, s, C*H*₂Ph), 4.60-4.23 (4H, m, H-3'+H-5'+N*H*), 4,20-3,97 (2H, m, H-4'+ C*H*CH₃), 2.48-2.44 (1H, m, one of H-2'), 2,15-2.05 (1H, m, one of H-2'), 1.43-1.40 (3H, d, ³*J*=7.0 Hz, CH*CH₃*)
¹³C-NMR (CDCl₃, 75 MHz): δ 21.2 (CH*C*H₃), 40.7 (C-2'), 50.8, 50.9 (*C*HCH₃), 66.6 (C-5'), 67.9 (*C*H₂Ph), 70.7, 71.1 (C-3'), 85.4, 85.5, 85.8, 86.1 (C-1', C-4'), 110.5 (C-5b), 111.9, 112.0 (C-5), 122.0, ('*o*', O*Ph*), 128,7, 129.0, 129.1, 130.3 ('*m*', O*Ph*+C-5a), 131.1 (*'ipso',* CH₂*Ph*), 135.4 ('*p*', O*Ph*), 138.2 (C-6), 149.1 ('*ipso*', O*Ph*), 150.0 (C-4), 162.1 (C-2), 173.9, 174.0 (*C*OOCH₂Ph).

### Synthesis of illustrative example (E)-5-(2-bromovinyl)-2'-deoxyuridine-5'-[phenyl-(methoxy-a,a-dimethylglycinyl)]-phosphate (CPF 26).

**C₂₂H₂₇BrN₃O₉P, MW 588.34**

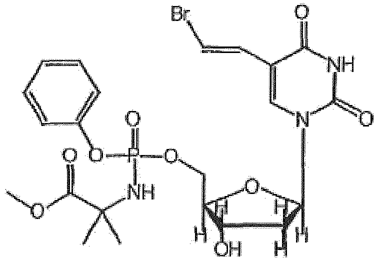

This was synthesised according to ***Standard procedure 5*,** using BVdU (200 mg, 0.60 mmol), phenyl-(methyl-2-amino-2-methylpropanoate)-phrosphorochloridate (437.5 mg, 1.5 mmol), NMI (246.3 mg, 3.0 mmol, 239.1 µL) in THF (5 mL) for 4 hrs. The crude product was purified by column chromatography, eluting with chloroform/methanol 97:3 to give the pure product as a white foamy solid (117 mg, yield 33.1%).
³¹P-NMR (CDCl₃, 121 MHz): δ 3.36, 3.14
¹H-NMR (CDCl₃; 300 MHz): δ 9.91 (1H,bs, H-3), 7.73,7.65 (1H, 2s, H-6), 7.50-7.43 (1H, 2d, ³*J*=13.6 Hz, H-5b), 7.41-7.02 (5H, m, O*Ph*), 6.81-6.71 (1H, 2d, ³*J*=13.6 Hz, H-5a), 6.34-6,28 (1H, m, H1'), 4.55-4.17 (6H, m, H-5'+H-4'+H-3', NH, OH-3'), 3.78 (3H, s, C*H₃*O), 2.53-2.39 (1H, m, one of H-2'), 2.25-1.99 (1H, m, one of H-2'), 1.60 (6H, s, [C*H₃*]₂C).
¹³C-NMR (CDCl₃; 75 MHz): δ 27.5,27.4, 27.2 ([*C*H₃] ₂C), 40.7, 40.6 (C-2'), 53.5 (*C*H₃O), 57.6 (*C*[CH₃]₂), 66.5, 66.2 (C-5'), 70.7, 71.1 (C-3'), 85.4, 85.6, 85.5, 85.9 (C-1', C-4'), 110.4 (C-5b), 111.9 (C-5), 120.5, 120.6 ('*o*', O*Ph*), 125.7 ('*p*', O*Ph*), 128.9 (C-5a), 130.3 ('*m*', O*Ph*)*,* 138.0, 138.3 (C-6), 149.8 ('*ipso*', O*Ph*) 150.9, 150.8 (C-4), 162.0, 162.1 (C-2), 176.4, 176.2 (*C*OOCH₃).

### Synthesis of illustrative example (E)-5-(2-bromovinyl)-2'-deoxyuridine-5'-[phenyl-(ethoxy-a,a-dimethylglycinyl)]-phosphate (CPF 27).

**C₂₃H₂₉BrN₃O₉P, MW=602.37**

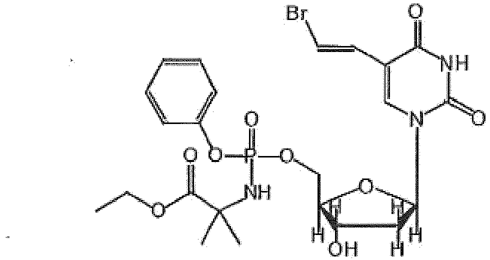

This was synthesised according to ***Standard procedure 5*,** using BVdU (200 mg, 0.60 mmol), phenyl-(ethyl-2-amino-2-methylpropanoate)phosphorocloridate (458.0 mg, 1.5 mmol), NMI (246.3 mg, 3.0 mmol, 219.1 µL) in THF (5 mL) for 5 hrs. The crude product was purified by column chromatography, eluting with cholorform/methanol 97:3 to give the pure product as a white foamy solid (106 mg, yield 29.3%).
³¹P-NMR (MeOD, 121 MHz): δ 3.91, 3.85
¹H-NMR (MeOD, 300 MHz): δ 7.84, 7.81 (1H, 2s, H-6), 7.44-7.20 (6H, m, O*Ph*+H-5b), 6.88-6.81 (1H, 2d, ³*J*=13.6Hz, H-5a), 6.34-6,28 (1H, m, H1'), 4.50-4.34 (3H, m, H-5'+H-3'), 4.23-4.15 (3H, m, H-4'+CH₃C*H₂*O), 2,38-2,28 (1H, m, one of H-2'), 2.22-2.09 (1H, m, one of H-2'), 1.51 (6H, s, [C*H₃*]₂C), 1.29 (3H, t, ³*J*=7 Hz, C*H₃*CH₂O)
¹³C-NMR (MeOD, 75 MHz): δ 14.9 (*C*H₃CH₂O) 27.9, 28.3 ([*C*H₃]₂C), 41.5 (C-2'), 58.51 (*C*[CH₃]₂), 63.1 (CH₃*C*H₂O), 68.2 (C-5'), 72.6 (C-3'), 87.1, 87.4 (C-1', C-4'), 109.6 (C-5b), 112.7 (C-5b), 122.0, 122.1, 122.2, ('*o*', O*Ph*), 126.7 ('*p*', O*Ph*), 131.0, 131.2 (C-5a, *'m'* O*Ph*)*,* 140.4 (C-6), 151.4 ('*ipso*', O*Ph*) 152.5 (C-4), 164.0 (C-2), 177.2 (*C*OOCH₂CH₃).

### Synthesis of illustrative example (E)-5-(2-bromovinyl)-2'-deoxyuridine-5'-[phenyl-(benzoxy-a,a-dimethylglycinyl)]-phosphate (CPF 14).

**C₂₈H₃₁BrN₃O₉P, MW=664.44.**

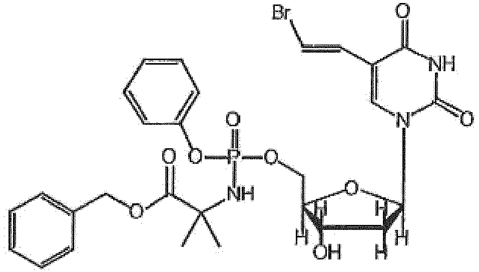

This was synthesized according to ***Standard procedure 5*,** using BVdU (242 mg, 0.73 mmol), phenyl-(benzyl-2-amino-2-methyphropanoate)-phrosphorochloridate (533.0 mg, 2.0 mmol), NMI (298.0 mg, 3.63 mmol, 289 µL) in THF (5 mL) for 4 hrs. The crude product was purified by column chromatography, eluting with chloroform/methanol 97:3 to give the pure product as a white foamy solid (129.0 mg, yield 26.7%).
³¹P-NMR (CDCl₃, 121 MHz): δ 3.30,3.12.
¹H-NMR (CDCl₃, 300 MHz): δ 9.92 (1H, bs, H-3), 7.67-7.60 (1H, 2s, H-6), 7.48-7.41 (1H, 2d, ³*J*=13.6 Hz, H-5b), 7.40-7.16 (10H. m, O*Ph*+CH₂*Ph*), 6.78-6.67 (1H, 2d, ³*J*=13.6 Hz, H-5a), 6.31-625 (1H, m, H-1'), 5.18 (1H, s, C*H*₂Ph), 4.50-4.09 (6H, m, H-3'+H-5'+H-4', N*H*, OH-3'), 2.48-2.25 (1H, m, one of H-2'), 2,16-1.82 (1H, m, one of H-2'), 1.60 (6H, s, [C*H3*] ₂C).
¹³C-N,MR (CDCl₃, 75 MHz): δ 27.3, 27.4, 28.5 ([*C*H₃]₂C), 40.6, 40.7 (C-2'), 57.6, 57.6 (*C*[CH₃]₂), 66.2, 66.5 (C-5'), 68.1 (*C*H₂Ph), 70.6, 71.1 (C-3'), 85.4, 85.5, 85.6, 85.8 (C-1', C-4'), 110.4 (C-5b), 112.0 (C-5), 120.4, 120.5, 120.6, 125,7, 128.4, 128.5, 128.8, 128.9, 130.3 (O*Ph*, C-5a), 135.7(*'ipso'*, CH₂*Ph*) 138.1, 138.3 (C-6), 149.8, 150.8, 150.9 ('*ipso*' O*Ph*, C-4), 162.1 (C-2), 177.5,175.7 (*C*OOCH₂Ph).

### Synthesis of illustrative example (E)-5-(2-bromovinyl)-2'-deoxyuridine-5'-[4-nitrophenyl-(methoxy-α,α-dimethylglycinyl)]-phosphate (CPF 45).

**C₂₂H₂₆BrN₄O₁₁P, MW=633.34.**

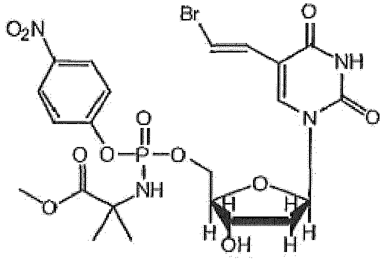

This was synthesized according to ***Standard procedure 5*,** using BVdU (150 mg, 0.45 mmol), 4-nitrophenyl-(methyl-2-amino-2-methylpropanoate)-phosphorochloridate (378.8 mg, 1.13 mmol), NMI (184.7 mg, 2.25 mmol, 179.4 µL) in THF (5 mL) for 3 hrs. The crude product was purified by column chromatography, eluting with dichloromethane/methanol 97:3 to give the pure product as a white foamy solid (145.7 mg, yield 50.9 %).
³¹P-NMR (MeOD, 121 MHz): δ 3.61, 3.56.
¹H-NMR (MeOD, 300 MHz): δ 8.30-8.25 (2H, 2d, ³*J*=9.0 Hz, O*Ph*), 7.79-7.78 (1H, 2s, H-6), 7.49-7.46 (2H, d, *³J*=9.0 Hz, O*Ph*), 7.37-7,32 (1H, 2d, ³*J*=13.6 Hz, H-5b), 6.79-6.72 (1H, 2d, ³*J*=13.6 Hz, H-5a), 6.32-6.25 (1H, m, H-1'), 4.48-4.35 (3H, m, H.3'+H-5'), 4,15-4.14 (1H, m, H-4'), 3.71 (3H, s, C*H*₃O), 2.41-2.17 (2H, m, H-2'), 1.51 (6H, s, [C*H*₃]₂C).
³C-NMR (CDCl₃, 75 MHz): δ 28.0, 28.1, 28.2, 28.3 ([*C*H₃]₂C), 41.4, 41.5 (C-2'), 53.6 (*C*H₃O), 58.7 (*C*[CH₂]₂), 68,5 (C-5'), 72.3, 72.4 (C-3'), 86.9, 87.0, 87.4, 87.5 (C-1', C-4'), 109.7 (C-5b), 112,6 (C-5), 122.8, 122.9 ('*o*', O*Ph*), 127.0 ('*m*' O*Ph*), 130.9 (C-5a), 140.5 (C-6), 146.5 ('*p*', O*Ph*), 151.5 ('*ipso*', O*Ph*)*,* 157.3 (C-4), 164.0 (C-2), 177.5 (*C*OOCH₃).

### Synthesis of illustrative example (E)-5-(2-bromovinyl)-2'-deoxyuridine-5'-[4-nitrophenyl-(ethoxy-a,a-dimethylglycinyl)]-phosphate (CPF 46).

**C₂₃H₂₈BrN₄O₁₁P, MW=647.3.**

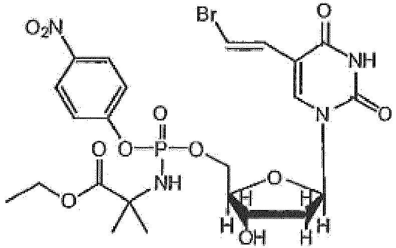

This was synthesised according to ***Standard procedure 5*,** using BVdU (150 mg, 0.45 mmol), 4-nitrophenyl-(ethy)-2-amino-2-methylpropanoate)-phosphorochloridate (442.1 mg, 1.26 mmol), NMI (184.7 mg, 2.25 mmol, 179.4 µL) in THF (5 mL) for 4 hrs. The crude product was purified by column chromatography, eluting with dichloromethane/methanol 97:3 to give the pure product as a white foamy solid (152.9 mg, yield 52,5 %).
³¹P-NMR (CDCl₃, 121 MHz): δ 3.00, 2.96.
¹H-NMR (CDCl₃, 300 MHz): δ 10.28 (1H, bs, H-3), 8.25.-8.12 (2H, 2d, ³*J*=9.0 Hz, O*Ph*), 7.68-7.67 (1H, 2s, H-6), 7.46-7.32 (3H, m, O*Ph*+H-5b), 6.69-6.67 (1H, 2d, ³*J*=13.5 Hz, H-5a), 6.32-6.26 (1H, m, H-1'), 4.75-4,36 (5H, m, H-3'+H-5'+OH-3'+N*H*), 4.25-4.17 (3H, m, OC*H₂*CH₃,H-4'), 2.60-2.98 (1H, m, one of H-2'), 2.31-2.10 (1H, m, one of H-2'), 1.58 (6H, s, [C*H₃*]₂C), 1.30-1.28 (3H, 2t, ³*J*=7.1 Hz, OCH₂C*H₃*).
¹³C-NMR (CDCl₃, 75 MHz): δ 14.5 (*C*H₃CH₂O), 27.1, 27.2,27.3, 27.4 ([*C*H₃]₂C), 40.6 (C-2'), 57.7 (*C*[CH₃]₂), 62.7 (CH₃*C*H₂O), 67.0 (C-5'), 71.0, 71.2 (C-3'), 85.4, 85.9, 86.1 (C-1', C-4'), 110.3 (C-5b), 111.9 (C-5), 121.2,121.3 ('*o*', O*Ph*), 126.2 (*'m'* O*Ph*), 128.8 (C-5a), 138.4 (C-6), 145.0 ('*p*', O*Ph*), 150.0 (C-4), 155.7-155.9 ('*ipso*', O*Ph*), 162.2 (C-2), 175.0-175.1 (*C*OOCH₂CH₃).

### Synthesis of illustrative example (E)-5-(2-bromovinyl)-2'-deoxyuridine-5'-[4-nitrophenyl-(benzoxy-α,α-dimethylglycinyl)]-phosphate (CPF 47).

**C₂₈H₃₀BRN₄O₁₁P, MW=709.44**

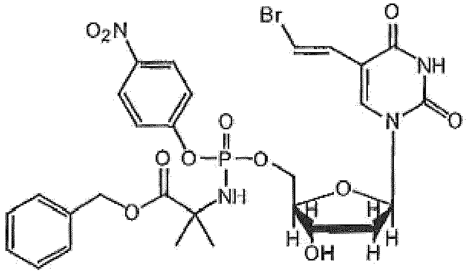

This was synthesised according to ***Standard procedure 5***, using BVdU (100 mg, 0,30 mmol), 4-nitrophenyl-(benzyl-2-amino-2-methylpropanoate)-phosphorochloridate (309.6 mg, 1.07 mmol), NMI (123.7 mg, 1,5 mmol, 120.1 µL) in THF (5 mL) for 5 hrs. The crude product was purified by column chromatography, eluting with dichloromethane/methanol 97:3 to give the pure product as a white foamy solid (160.2 mg, yield 50.2 %).
³¹P-NMR (CDCl₃, 121 MHz): δ 2.95, 2.89.
¹H-NMR (CDCl₃ 300 MHz): δ 10.16 (1H, bs, H-3), 8.26-8.24 (2H, 2d, ³*J*=9.1 Hz, O*Ph*), 7.71-7.69 (1H, 2s, H-6), 7.48-7.37 (8H, m, O*Ph*+CH₂*Ph*, H-5b), 6.75-6.12 (1H, 2d, ³*J*=13.5 Hz, H-5a), 6.36-6.29 (1H, m, H-1'), 5.24 (2H, s, C*H*₂Ph), 4.81-4.40 (5H, m, H-3'+H-5'+OH-3', N*H*), 4.22-4.21 (1H, m, H-4'), 2.57-2.36 (1H, m, one of H-2'), 2.27-2.22 (1H, m, one of H-2'), 1.64 (6H, s, [C*H*₃]₂C).
¹³C-NMR (CDCl₃, 75 MHz): δ 27.4 ([*C*H₃]₂C), 40.6 (C-2'), 57.8 (*C*[CH₃]₂), 67.0 (C-5'), 68.2 (*CH*₂Ph), 71.1, 71.2 (C.3'), 85.3, 86.2 (C-1', C-4'), 110.5 (C-5b), 111.9 (C-5), 121.2, 126.2, 128.5, 128.8, 129.0, 129.1 ('*o'*, '*m*'*,* '*p*', CH₂*Ph*+O*Ph*+C-5a), 135.5 ('*ipso'*, CH₂*Ph*), (C-5a), 138.4 (C-6), 145.0 ('*p*', O*Ph*), 150.0 (C-4), 155.7 ('*ipso*', O*Ph*), 162.2 (C-2), 175.4-175.5 (*C*OOCH₂Ph).

### Synthesis of illustrative example (E)-5-(2-bromovinyl)-2'-deoxyuridine-5'-[4-nitrophenyl-(methoxy-α,α-dimethylglycinyl)]-phosphate (CPF 42).

**C₂₂H₂₆BrCIN₃O₉P, MW=622,79.**

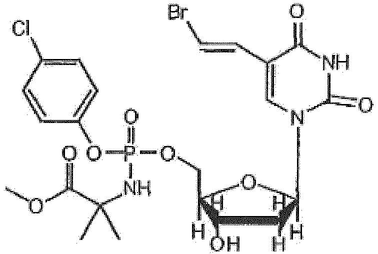

This was synthesised according to ***Standard procedure 5***, using BVdU (150 mg, 0.45 mmol), 4-chlorophenyl-(methyl-2-amino-2-methylpropanoate)-phosphorochloridate (440.2 mg, 1.35 mmol), NMI (184.7 mg, 2.25 mmol, 179.4 µL) in THF (5 mL) for 6 hrs. The crude product was purified by column chromatography, eluting with dichloromethane/methanol 97:3 to give the pure product as a white foamy solid (146.7 mg, yield 56.5 %).
³P-NMR (MeOD, 121 MHz): δ 3.98 (s).
¹H-NMR (MeOD, 300 MHz): δ), 7.71-7.69 (1H, 2s, H-6), 7.31-7.13 (5H, m, O*Ph*+H-5b), 6.73-6.66 (1H, 2d, ³*J*=13.6 Hz, H-5a), 6.23-6.16 (1H, m, H-1'), 4.39-4.22 (3H, m, H-3'+H-5'), 4.05-4.03 (1H, m, H-4'), 3.61 (3H, s, C*H*₃O), 2.29-2.19 (1H, m, one of H-2'), 2.15-2.05 (1H, m, one of H-2'), 1.38 (6H, s, [C*H*₃]₂C).
¹³C-NMR (CDCl₃; 75 MHz): δ 28.0, 28.2, 28.3, 28.4 ([*C*H₃]₂C), 41.5, 41.6 (C-2'), 53.5, 53.6 (*C*H₃O), 58.6 (*C*[CH₃]₂), 68.2 (C-5'), 72.4, 72.5 (C-3'), 87.1, 87.2, 87.3, 87.4 (C-1', C-4'), 109.7 (C-5b), 112.7 (C-5), 123.7, 123.8 ('*o'*, O*Ph*), 130.9, 131.1 ('*m'*, O*Ph*+C-5a), 131.9 ('*p*', O*Ph*), 140.4 (C-6), 151.1, 151.2, 151.4 (*'ipso'*, O*Ph*+C-4), 164.0 (C-2), 177.6, 177.7 (*C*OOCH₃).

### Synthesis of illustrative example (E)-5-(2-bromovinyl)-2'-deoxyuridine-5'-[4-chlorophenyl-(ethoxy-α,α-dimethylglycinyl)]-phosphate (CPF 43).

**C₂₃H₂₈BrCIN₃O₉,P, MW=636.91.**

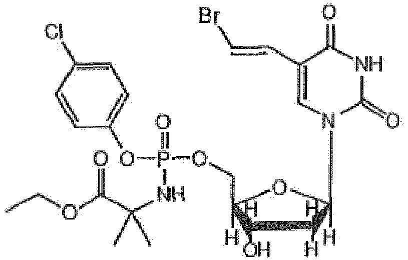

This was synthesised according to ***Standard procedure 5***, using BVdU (150 mg, 0,45 mmol), 4-chlorophenyl-(ethyl-2-amino-2-methylpropanoate)-phosphorochloridate (413.3 mg, 1.22 mmol), NMI (184.7 mg, 2.25 mmol, 179.3 µL) in THF (5 mL) for 16 hrs. The crude product was purified by column chromatography, eluting with dichloromethane/methanol 97:3 to give the pure product as a white foamy solid (74 mg, yield 25.8 %).
³¹P-NMR (CDCl₃, 121 MHz): δ 3.47, 3.33.
¹H-NMR (CDCl₃, 300 MHz): δ 10.03-9.99 (1H, 2bs, H-3), 7.70-7.67 (1H, 2s, H-6), 7.47-7.43 (1H, 2d, ³*J*=13.6 H₂, H-5b), 7.35-7.20 (4H, m, O*Ph*), 6.77-6.68 (1H, 2d, ³*J*=13.6 Hz, H-5a), 6.33-6.27 (1H, m, H-1'), 4.55-4.29 (5H, m, H-3'+H-5'+ OH-3'-N*H*), 4.22-4.17 (2H, q, ³*J*=7.1 Hz, OC*H*₂CH₃+H-4'), 2.53-2.42 (1H, m, one of H-2'), 2.22-2.08 (1H, m; one of H-2'), 1.57-1.54 (6H, 2s, [C*H₃*]₂C), 1.31-1.30 (3H, 2t, ³*J*=7.1 Hz, OCH₂C*H₃*).
¹³C-NMR (CDCl₃, 75 MHz): δ 14.5 [*C*H₃CH₂O), 27.2, 27.3, 27.4 ([*C*H₃]₂C), 40.7 (C-2'), 57.6 (*C*[CH₃]₂), 62.6 (CH₃*C*H₂O), 66.5, 66.6 (C-5'), 70.8, 71.1 (C-3'), 85.5, 85.74, 86.0 (C-1', C-4'), 110.4 (C-5b), 112.0 (C-5), 121.9, 122.0, 122.1 ('*o*', O*Ph*), 128.9, 130.2 ('*m*', O*Ph*+C-5a), 130.9 ('*p'*, O*Ph*), 138.3 (C-6), 149.4 (*'ipso'*, O*Ph*), 149.9 (C-4), 162.1, 162.2 (C-2), 175.7-175.9 (*C*OOCH₂CH₃).

### Synthesis of illustrative example (E)-5-(2-bromovinyl)-2'-deoxyuridine-5'-[4-chlorophenyl-(benzoxy-α,α-dimethylglycinyl)]-phosphate (CPF 44).

**C₂₈H₃₀BrCIN₃O₉P, MW=698.88.**

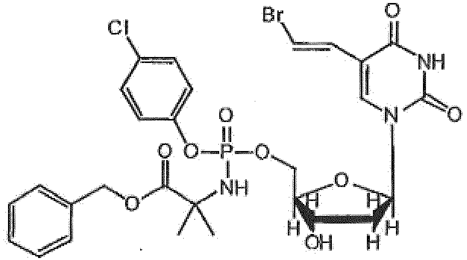

This was synthesised according to ***Standard procedure 5***, using BVdU (150 mg, 0.45 mmol), 4-chlorophenyl-(benzyl-2-amino-2-methylpropanoate)-phosphorochloridate (505,0 mg, 1.25 mmol), NMI (184.7 mg, 2,25 mmol, 179.3 µL) in THF (5 mL) for 16 hrs. The crude product was purified by column chromatography, eluting with dichloromethane/methanol 97:3 to give the pure product as a white foamy solid (134.8 mg, yield 42.9%).
³¹P-NMR (CDCl₃, 121 MHz): δ 3.44, 3.26.
¹H-NMR (CDCl₃, 300 MHz): δ 9.96-93 (1H, 2bs, H-3), 7.66-7.65 (1H, 2s, H-6), 7.47-7.41 (1H, 2d, ³*J*=13.5, H-5b), 7.39-7.18 (9H, m, O*Ph*+CH₂*Ph*) 6.74-6.69 (1H, 2d, ³*J*=13.5 Hz, H-5a), 6.31-6.25 (1H, m, H-1'), 5.19 (2H, C*H*₂Ph), 4.51-4.29 (4H, m, H-3'+H-5'+N*H*), 4.15-4.12 (2H, m, H-4'+OH-3'), 2.48-2.40 (1H, m, one of H-2'), 2.18-2.05 (1H, m, one of H-2'), 1.60-1.59 (6H, 2s, [C*H₃*]₂C).
¹³C-NMR (CDCl₃, 75 MHz): δ 27.1, 27.5 ([*C*H₃]₂C), 40,7 (C-2'), 57.7 (*C*[CH₃]₂), 66.4, 66.6 (C-5'), 68.2 (*CH*₂Ph), 70.7, 71.1 (C-3'), 85.4, 85.5, 85.7, 86.0 (C-1', C-4'), 110.5 (C-5b), 112.0 (C-5), 121.9, 122.0, 128.4, 128.5, 128.9, 129.1 ('*o'*, '*m'*, '*p*', CH₂*Ph*+O*Ph*+C-5a), 131.0 (*'ipso'*, CH₂*Ph*), 135.6 ('*p'*, O*Ph*), 138.1 (C-6), 149.3 (*'ipso*', *OPh*), 149.8 (C-4), 162.1 (C-2), 175.6 (COOCH₂Ph).

### Synthesis of illustrative example (E)-5-(2-bromovinyl)-2'-deoxyuridine-5'-[para-(trifluoromethyl)-phenyl-(benzoxy-α,α-dimethylglycinyl)]-phosphate (CPF 48).

**C₂₉H₃₀BrF₃N₃O₉P, MW=732.44.**

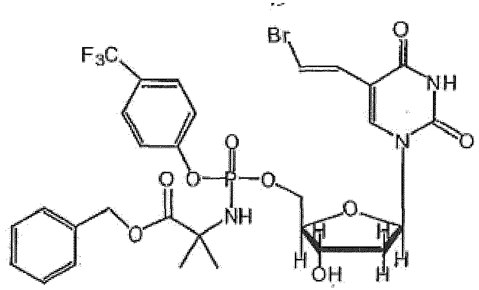

This was synthesised according to ***Standard procedure 5***, using BVdU (150 mg, 0.45 mmol), 4-(trifluoromethyl)-phenyl-(benzyl-2-amino-2-methylpropanoate)-pbosphorochloridate (529.4.5 mg, 1.22 mmol), NMI (184.7 mg, 2.25 mmol, 179.4 µL) in THF (5 mL) for 4 hrs. The crude product was purified by column chromatography, eluting with dichloromethane/methanol 97:3 to give the pure product as a white foamy solid (142.1 mg, yield 43.1%).
³¹P-NMR (CDCl₃, 121 MHz): δ 3.16,3.01.
¹H-NMR (CDCl₃, 300 MHz): δ 10.06-10.02 (1H, 2bs, H-3), 7.67-7.66 (1H, s, H-6), 7.64-7.60 (2H, 2d, ³*J*=8.8 Hz, O*Ph*), 7.46-7.32 (8H, m, O*Ph*+CH₂*Ph*+H-5b), 6.77-6.68 (1H, 2d, ³*J*=13.6 Hz, H-5a), 6.31-6.26 (1H, m, H-1'), 5.18 (2H, s, C*H*₂Ph), 4.61-4.32 (4H, m, H-3'+H-5'+N*H*), 4.16-4.15 (2H, m, H-4'+OH-3'), 2.48-2.41 (1H, m, one of H-2'), 2.23-2.09 (1H, m, one of H-2'), 1.60-1.58 (6H, 2s, C[C*H*₃]₂)
¹³C-NMR (CDCl₃, 75 MHz): δ 27.0, 27.4, 27.5 (C[*C*H₃]₂), 40.6 (C-2'), 57.7, 57.8 (*C*[CH₃]₂), 66.8, 66.5 (C-5'), 68.2(*C*H₂Ph), 70.8, 71.1 (C-3'), 85.4, 85.7, 86.0 (C-1', C-4'), 110.4 (C-5b), 111.9 (C-5), 120,8, 120.9, 121.0, 127.6, 127.7, 128.0, 128.5, 128.8, 129.0 ('*o'*, '*m*', '*p*', -O*Ph*+CH₂*Ph*+C-5a), 124.2 (*C*F₃, *J*=267 Hz), 135.6 ('*ipso'*, CH₂*Ph*), 138.2 (C-6), 149.9 (C-4), 153.3 ('*ipso'*, O*Ph*), 162.1 (C-2), 175.4 (*C*OOCH₂Ph).

### Synthesis of illustrative example (E)-5-(2-Bromovinyl)-2'-deoxyuridine-5'-[phenyl-(methoxy-α,α-cycloleucinyl)]-phosphate (CPF 16).

**C₂₄H₂₉BrN₃O₉P, MW-614.38.**

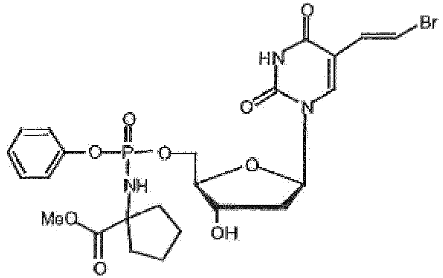

This was synthesised according to ***Standard procedure 5***, using BVdU (250 mg, 0.75 mmol), Phenyl-(methoxy-α,α-cycloleucinyl)-phosphorochloridate (589 mg, 1,87 mmol), NMI (6.2 mmol, 415 µL) in THF (7 mL) for 3 hrs. The crude product was purified by column chromatography, eluting with CH₂Cl₂/Methanol 97:3 to give the pure product as a white foamy solid (234 mg, yield 51%).
³¹P-NMR (CDCl₃, 121 MHz): δ 3.87, 3.82.
¹H-NMR (CDCl₃; 300 MHz): δ 10.35-10.2 (1H, bs, H-3), 7.65 (1H, 2xs, H-6), 7.44-7,39 (1M, 2d, ³*J*=13 Hz, H-5b), 7.37-7.15 (5H, m, O*Ph*), 6.8 (1H, 2d, ³*J*=13 Hz, H-5a), 6.30 (1H, 2t, ³*J*=6 Hz, H1'), 4.4-4.2 (4H, m, H-5', H-3', NH), 4,1 (1H, H-4'), 3.72 (3H, 2s, CH₃O), 2.49-2.40 (1H, m, one of H-2'), 2.35-2.01 (5H, m, one of H-2'+4H cyclopentane), 1.8-1.6 (4H, m, 4H cyclopentane).
¹³C-NMR (DMSO; 75 MHz); δ 24.4, 24.3, 24.2 (2CH₂ cyclopent), 39.2, 38.6, 38.5 (2CH₂ cyclopent), 40.0 (C-2'), 53.2 (*C*H₃O), 66.4 (*Cq* cyclopentane), 66.6 (C-5'), 70.9 (C-3'), 85.8, 85.6, 85.4, 85.3 (C-1', C-4'), 110.2 (C-5b), 111.9 (C-5), 120.7-120.6 ('*o'*, O*Ph*), 125.7 ('*p'*, O*Ph*), 129.0 (C-5a), 130.2 ('*m*', O*Ph*), 138.5 (C-6), 149.9 (C-4), 150.9, 150.8 (*'ipso*', O*Ph*), 162.3(C-2), 176.3, 176.2 (*C*OOCH₃).

### Synthesis of illustrative example (E)-5-(2-Bromovinyl)-2'-deoxyuridine-5'-[phenyl-(ethoxy-α,α-cycloleucinyl)]-phosphate (CPF 17).

**C₂₅H₃₁BrN₃O₉P, MW=628.41.**

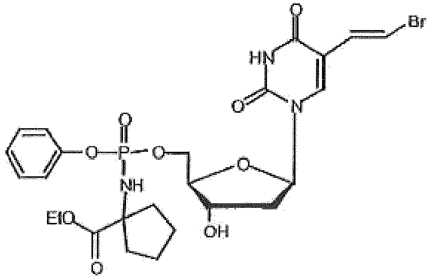

This was synthesised according to ***Standard procedure 5***, using BVdU (250 mg, 0.75 mmol), Phenyl-(ethoxy-α,α-cycloleucinyl)-phosphorochloridate (642 mg, 1.87 mmol), NMI (6.2 mmol; 415 µL) in THF (7 mL) for 2 hrs. The crude product was purified by column chromatography, eluting with CH₂Cl₂/Methanol 97:3 to give the pure product as a white foamy solid (258 mg, yield 55%).
³¹P-NMR (CDCl₃, 121 MHz): δ 4.23, 4.1.
¹H-NMR (CDCl₃, 300 MHz): δ 10.3-10.1 (1H, bs, H-3), 7.8-7.75 (1H, 2xs, H-6), 7.51 (1H, 2d, ³*J*=14 Hz, H-5b), 7.45-7.10 (5H, m, O*Ph*),6.8 (1H, 2d, ³*J*=14 Hz, H-5a), 6.22 (1H, 2t, ³*J*=4 Hz, H1'), 4.55-4.05 (7H, m, H-5', H-3', H-4', NH, CH₃C*H*₂O), 2.50-2.40 (1H, m, one of H-2'), 2.35-1.95 (5H, m, one of H-2'+4H cyclopentane), 1.95-1.75 (4H, m, 4H cyclopentane), 1.25 (3H, 2t, ³*J*=7 Hz, C*H*₃CH₂O).
¹³C-NMR (CDCl₃, 75 MHz): δ 14.5 (*C*H₃CH₂O), 24.5, 24,4 (2CH₂ cyclopent), 39.2, 38.9 38.8, 38.4 (2CH₂ cyclopent), 40.6 (C-2'), 62.2, 62.1 (CH₃*C*H₂O), 66.2 (*Cq* cyclopentane), 66.6 (C-5'), 70.8 (C-3'), 85.7, 85.5 (C-1', C-4'), 110.2 (C-5b), 111.5 (C-5), 120.7, 120.6 (*'*o', O*Ph*), 125.6 ('*p'*, O*Ph*), 129.7 (C-5a), 130.2 (*'m'*, O*Ph*), 138.5, 138.3 (C-6), 149.7 (C-4), 150.9, 150.8 (*'ipso'*, O*Ph*), 162.3 (C-2), 176.3 (*C*OOCH₂CH₃).

### Synthesis of illustrative example (E)-5-(2-Bromovinyl)-2'-deoxyuridine-5'-[phenyl-(benzoxy-α,α-cycloleucinyl)]-phosphate (CPF 18).

**C₃₀H₃₃BrN₃O₉P, MW=690.48.**

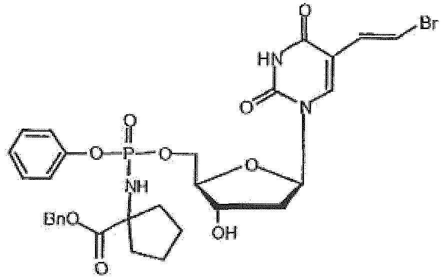

This was synthesised according to ***Standard procedure 5***, using BVdU (200 mg, 0.6 mmol), Phenyl-(benzyloxy-α,α-cycloleucinyl)-phosphorochloridate (589 mg, 1.5 mmol). NMI (4.98 mmol, 332 µL) in THF (5 mL) for 10 hrs, The crude, product was purified by column chromatography, during with CH₂Cl₂/Methanol 97:3 to give the pure product as a white foamy solid (127 mg, yield 31%).
³¹P-NMR (CDCl₃, 121 MHz): δ 4,11, 4.01.
¹H-NMR (CDCl₃, 300 MHz): δ 10.2 (1H, bs, H-3), 7.8-7.6 (1H, 2xs, H-6), 7.45-7.4 (1H, 2d, ³*J*=14 Hz, H-5b), 7.40-7.10 (10H. m, O*Ph*+CH₂*Ph*), 6.85 (1H, 2d, *³J*=14 Hz, H-5a), 6.20 (1H, m, H-1'), 5.15 (1H, s, C*H*₂Ph), 4,4-4.2 (3H, m, H-3',H-4', N*H*), 4.1 (2H, m, H-5'), 2.45-2.35 (1H, m, one of H-2'), 2.35-1.95 (5H, m, one of H-2'+4H cyclopentane), 1.95-1.75 (4H, m, 4H cyclopeutane).
¹³C-NMR (CDCl₃, 75 MHz): δ 24.4, 24,3, 24.2 (2CH₂ cyclopent), 39.9, 39.7 38.6, 38.5 (2CH₂ cyclopent), 40.5 (C-2'), 66.2 (*Cq* cyclopentane), 66.5 (C-5'), 67.8 (*C*H₂Ph), 70.8, 70.7 (C-3'), 85.7, 85.6, 85.5, 85.4 (C-1', C-4'), 110.2 (C-5b), 111.8, 118,7 (C-5b), 120.7, 120.5 ('o', O*Ph*), 125,7 ("*p*" O*Ph*), 130.2, 129.0, 128.8, 128.7, 128.5 ('*m*' OPh, Bn, C-5a), 135.8('*ipso*', CH₂*Ph*) 138.4, 138:2 (C-6), 149.8 (C-4), 150.9, 150.8 (*'ipso'*, O*Ph*), 162.2 (C-2), 175.7, 175.5 (*C*OOBn).

### Synthesis of illustrative example (E)-5-(2-Bromovinyl)-2'-deoxyuridine-5'-[para-nitrophenyl-(methoxy-α,α-cycloleucinyl)]-phosphate (CPF 19).

**C₂₄H₂₈BrN₄O₁₁P, MW=659.38.**

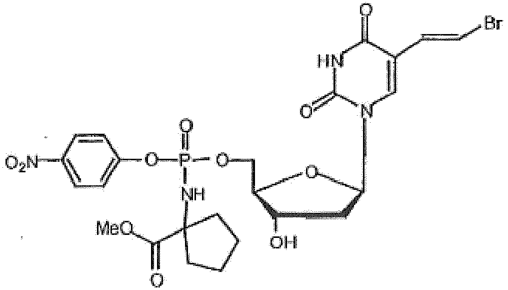

This was synthesized according to ***Standard procedure 5***, using BVdU (200 mg, 0.60 mmol), para-nitropheny]-(methoxy-α,α,-cycloleucinyl)-phosphorochloridate (543 mg, 1.5 mmol), NM1 (4.98 mmol, 332 µL) in THF (5 mL) for 2 hrs. The crude product was purified by column chromatography, eluting with CH₂Cl₂/Methanol 97:3 to give the pure product as a white foamy solid (239 mg, yield 60%),
³¹P-NMR (CDCl₃, 121 MHz): δ 3.73.
¹H-NMR (CDCl₃; 300 MHz): δ 10.5.10.2 (1H, bs, H-3), 8.35-8.25 (2H, 2d, ³*J*=6 Hz O*Ph*) 7.8-7.75 (1H, 2xs, H-6), 7.47 (2H, 2d, ³*J*=6 Hz, O*Ph*), 7.45-7.35 (1H, 2d, ³*J*=14 Hz, H-5b), 6.75-6.67 (1H, 2d, ³*J*=14 Hz, H-5a), 6.30 (1H, 21, ³*J*=6 Hz, H1'), 4.65-4.4 (3H, m, H-5',H. 3'), 4,25-4,20 (1H, m, H-4'), 3.79 (3H, s, CH₃O), 2.6-2.4 (1H, m, one of H-2'), 2.3-1.98 (5H, m, one of H-2'+4H cyclopentane), 1.9-1.76 (4H, m, 4H cyclopentane).
¹³C-NMR (CDCl₃; 75 MHz): δ 24.4, 24,3, 24.2 (2CH₂ cyclopent), 39.2, 39.1 (2CH₂ cyclopent), 40.5 (C-2'), 53.4, 53.3 (*C*H₃O), 66.8 (*Cq* cyclopentane), 67.1 (C-5'), 70.9 (C-3'), 86.1, 86.0, 85.5, 85.4 (C-1', C-4'), 110.2 (C-5b), 111.8 (C-5), 121.3, 121.2 ('*o*', *OPh*)*,* 126.2 ('*m*' O*Ph*), 128.9 (C-5a), 138.6 (C-6), 144.9 ('*ipso*', O*Ph*) 149.9 (C-4), 155.9, 155.8 ('*p*' O*Ph*), 162.3 (C-2), 176.3 (*C*OOCH₃).

### Synthesis of illustrative example (E)-S-(2-Bromovinyl)-2'-deoxyuridine-5'-[para-nitrophenyl-(ethoxy-α,α-cycloleucinyl)]-phosphate (CPF 20).

**C₂₅H₃₀BrN₃O₁₁P, MW=673.4.**

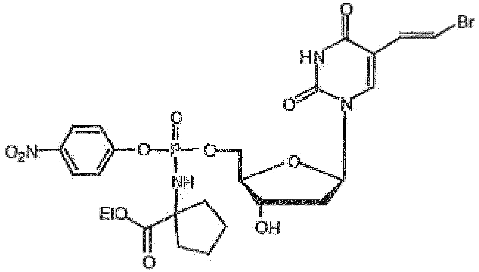

This was synthesised according to ***Standard procedure 5***, using BVdU (200 mg, 0.60 mmol), para-nitrophenyl-(ethoxy-α,α-cycloleucinyl)-phosphorochloridate (563 mg, 1.5 mmol), NMI (4.98 mmol, 332 µL) in THF (5 mL) for 1 hr. The crude product was purified by column chromatography, eluting with CH₂Cl₂/Methanol 97:3 to give the pure product as a white foamy solid (240 mg, yield: 59%),
³¹P-NMR (CDCl₃, 121 MHz): δ 3.83, 3.79,
¹H-NMR (CDCl₃, 300 MHz): δ 8.25-8.2 (2H, 2d, ³*J*=9Hz *OPh*), 7.66 (1H, s, H-6). 7.4 (2H, 2d, ³*J*=9Hz, O*Ph*), 7.3 (1H, 2d, ³*J*=14 Hz, H-5b), 6.85 (1H, 2d, ³*J*=14 Hz, H-5a), 6.3-6.2 (1H, m, II1'), 4.7-4.45 (4H, m, H-5', H-3', NH), 4.2-4.05 (3H, m, H-4', CH₃C*H*₂O), 2.55-2.4 (1H, m, one of H-2'), 2.2-1.95 (5H, m, one of H-2'+4H cyclopentane), 1.95-1.8 (4H, m, 4H cyclopentane), 1.2 (3H, 2t, ³*J*=8 Hz, C*H*₃CH₂O).
¹³C-NMR (CDCl₃, 75 MHz): δ 14.9 (*C*H₃CH₂O), 24.5, 24,4 (2CH₂ cyclopent), 39.1, 39.0, 38.8 (2CH₂ cyclopent), 40.7 (C-2'), 62.4 (CH₃*C*H₂O), 66.5 (*Cq* cyclopentane), 67.0 (C-5'), 70.9 (C-3'), 85.9, 85.4 (C-1', C-4'), 110.2 (C-5b), 111.8 (C-5), :121.3 ('o', O*Ph*), 126.2 (*'m',* O*Ph*), 128.8 (C-5a), 138.5 (C-6), 144.9 (*'ipso*', O*Ph*), 149.9 (C-4), 155.5 ('*p*', O*Ph*), 162.3 (C-2), 175.8, 175.7 (*C*OOCH₂CH₃).

### Synthesis of illustrative example (E)-5-(2-Bromovinyl)-2'-deoxyuridine-5'-[para-nitrophenyl-(benzoxy-α,α-cycloleucinyl)]-phosphate (CPF 21).

**C₃₀H₃₂BrN₄O₁₁P, MW=735.47.**

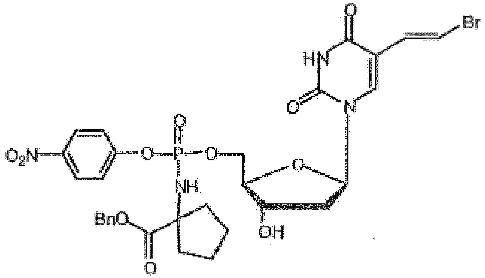

This was synthesised according to ***Standard procedure 5***, using BVdU (200 mg, 0.60 mmol), para-nitrophenyl-(benzyloxy-α,α-cycloleucinyl)-phosphorochloridate (656 mg, 1.5 mmol), NMI (4.98 mmol, 332 µL) in THF (5 mL) for 3 hrs. The crude product was purified by column chromatography, eluting with CH₂Cl₂/Methanol 97:3 to give the pure product as a white foamy solid (269 mg, yield 61%).
³¹P-NMR (CDCl₃, 121 MHz): δ 3.72.
¹H-MMR (CDCl₃, 300 MHz): δ 10.3 (1H, bs, H-3), 8.22-8.12 (2H, 2d, ³*J*=7 Hz, O*Ph*), 7.65 (1H, 2xs, H-6), 7.45-730 (8H, m, H-5b+O*Ph*+CH₂*Ph*), 6.72-6.65 (1H, 2d, ³*J*=14 Hz, H-5a), 6.28 (1H, 2t, ³*J*=6Hz, H-1'), 5.15 (1H, d, C*H*₂Ph), 4.6-4.35 (4H, m, H-3', H-5', H-4', N*H*,), 2,55-2.4 (1H, m, one of H-2'), 2.3-1.92 (5H, m, one of H-2'+4H cyclopentane), 1.85-1.6 (4H, m, 4H cyclopentane).
¹³C-NMR (CDCl₃, 75 MHz): δ 24.4, 24,3, 24.2 (2CH₂ cyclopent), 39.1, 38.9, 38.7 (2CH₂ cyclopent), 40.5 (C-2), 66.9 (*Cq* cyclopentane), 67.1 (C-5'), 68.0 (*C*H₂Ph), 70.9 (C-3'), 85.3, 85.0 (C-1', C-4'), 110.3 (C-5b), 111.8 (C-5), 121.2 ('o', O*Ph*), 126.1 ('*m*', *OPh*)*,* 129.0, 128.8 (Bn, C-5a), 135.7 (*'ipso'*, CH₂*Ph*), 138.5 (C-6), 144.9 (*'ipso'*, O*Ph*), 149.9 (C-4), 155.8 ('*p*' OPh), 162.3 (C-2), 175.6 (*C*OOBn).

### Synthesis of illustrative example (E)-S-(2-Bromovinyl)-2'-deoxyuridine-5'-[para-fluorophenyl-(methoxy-α,α-cycloleucinyl)]-phosphate (CPF 22).

**C₂₄H₂₈BrFN₃O₉P, MW=632.37.**

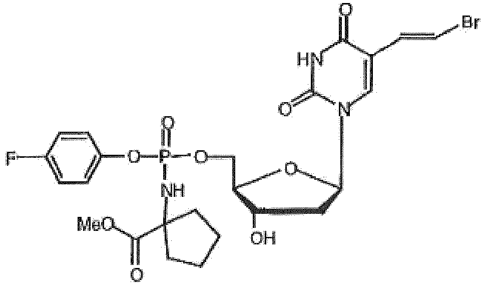

This was synthesised according to ***Standard procedure 5***, using BVdU (200 mg, 0.60 mmol), para-fluorophenyl-(methoxy-α,α-cycloleucinyl)-phosphorochloridate (503 mg, 1.5 mmol), NMI (4.98 mmol, 332 µL) in THF (5 mL) for 2 hrs. The crude product was purified by column chromatography, eluting with CH₂Cl₂/Methanol 97:3 to give the pure product as a white foamy solid (251 mg, yield 66%).
³¹P-NMR (CDCl₃, 121 MHz): δ 4.22.
¹H-NMR (CDCl₃; 300 MHz): δ 10.3 (1H, bs, H-3), 7.70 (1H, 2xs, H-6), 7.4 (1H, 2d, ³*J*=14 Hz, H-5b), 7.25-7.15 (2H, m, O*Ph*), 7.1-6.95 (2H, m, O*Ph*), 6.70(1H. 2d, ³*J*=14 Hz, H-5a), 6.30-6.15 (1H, 2t, ³*J*=5 Hz, H1'), 4.55-4.05 (5H, m, H-5'+H-3', NH, H-4'), 3.72 (3H, 2s, CH₃O), 2.55-2.35 (1H, m, one of H-2'), 2.25-1.92 (5H, m, one of H-2'+4H cyclopentane), 1.85-1.6 (4H, m, 4H cyclopentane),
¹³C-NMR (DMSO; 75 MHz): δ 24.4, 24,3, 24.2 (2CH₂ cyclopent), 39.3, 39.2, 38.9, 38.5 (2CH₂ cyclopent), 40.6 (C-2'), 53.3, 53.2 (*C*H₃O), 66.5 (*Cq*_cyclopentane), 66.7 (C-5'), 70.9 (C-3'), 85.8, 85.7, 85.4 (C-1', C-4'), 110.2 (C-5b), 111.9 (C-5), 116.9, 116.6 ('*o*', *OPh*), 122,2, 122.0 ('*m*', O*Ph*), 128.5 (C-5a), 138.5 (C-6), 146.7 (*'ipso'*, O*Ph*) 149.9 (C-4), 158.5 ('*p*', O*Ph*), 162.3(C-2), 176.4, 176.3 (*C*OOCH₃).

### Synthesis of illustrative example (E)-S-(2-Bromovinyl)-2'-deoxyuridine-5'-[para-fluorophenyl-(ethoxy-α,α-cycloleucinyl)]-phosphate (CPF 23).

**C₂₅H₃₀BrFN₃O₉P, MW=646.4.**

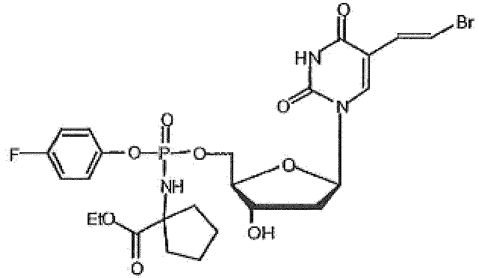

This was synthesised according to ***Standard procedure 5***, using BVdU (200 mg, 0.60 mmol), para-fluorophenyl-(ethoxy-α,α-cycloleucinyl)-phosphorochloridate (524 mg, 1.5 mmol), NMI (4.98 mmol, 332 µL) in THF (5 mL) for 2 hrs. The crude product was purified by column chromatography, eluting with CH₂Cl₂/Methanol 97:3 to give the pure product as a white foamy solid (274 mg, yield 71%).
³¹P-NMR (CDCl₃, 121 MHz): δ 5.30.
¹H-NMR (CDCl₃, 300 MHz): δ 10.35 (1H, bs, H-3), 7.7 (1H, 2xs, H-6), 7.44 (1H, 2d, ³*J*=14 Hz, H-5b), 7.25-7.15 (2H, m, O*Ph*), 7,1-6.95 (2H, m, O*Ph*), 6.7 (1H, 2d, ³*J*=14 Hz, H-5a), 6.30 (1H, 2t, ³*J*=6 Hz, H1'), 4.55,4.3 (3H, m, H-5', H-3'), 4.24.1 (4H, m, NH, H-4', CH₃C*H*₂O), 2.55-2.4 (1H, m, one of H-2'), 2.22-1.90 (5H, m, one of H-2'+4H cyclopentane), 1.8-1.6 (4H, m, 4H cyclopentane), 1.3-1.2 (3H, 2t, ³*J*=7 Hz, C*H*₃CH₂O),
¹³C-NMR (CDCl₃, 75 MHz): δ 14,5 (*C*H₃CH₂O), 24.6, 24,4, 24.3 (2CH₂ cyclopent), 39.3, 39.2, 38.9, 38.6 (2CH₂ cyclopent), 40.6 (C-2'), 62.2 (CH₃*C*H₂O), 66.5 (*Cq* cyclopentane), 66.7 (C-5'), 71.0 (C-3'), 85.8, 85.7, 85.5, 85.4 (C-1', C-4'), 110.2 (C-5b), 111.9 (C-5), 116.9, 116.5 ('o', O*Ph*), 122.2, 122.1 (*'m',* O*Ph*), 129.0 (C-5a), 138.5 (C-6), 146.8,146.7 ('*ipso*', O*Ph*), 149.9 (C-4), 158.5 ('*p*', O*Ph*), 162.3 (C-2), 175.9, 175.8 (*C*OOCH₂CH₃).

### Synthesis of illustrative example (E)-S-(2-Bromovinyl)-2'-deoxyuridine-5'-[para-fluorophenyl-(benzoxy-α,α-cycloleucinyl)]-phosphate (CPF 24).

**C₃₀H₃₂BrN₃O₉P, MW=708,47.**

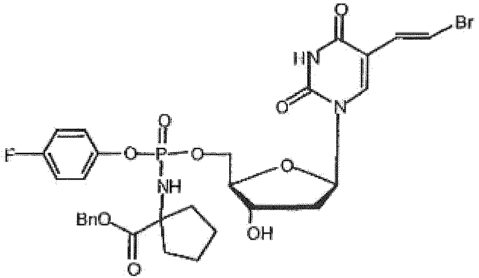

This was synthesized according to ***Standard procedure 5***, using BVdU (200 mg, 0.60 mmol), para-fluorophenyl-(benzyloxy-α,α-cycloleucinyl)-phosphorochloridate (616 mg, 1.5 mmol), NMI (4.98 mmol, 332 µL) in THF (5 mL) for 2 hrs. The crude product was purified by column chromatography, during with CH₂Cl₂/Melhanol 97:3 to give the pure product as a white foamy solid (283 mg, yield 67%).
³¹P-NMR (CDCl₃, 121 MHz): δ 4.27.
¹H-NMR (CDCl₃, 300 MHz): δ 10.3-9.85 (1H, bs, H-3), 7.65 (1H, 2xs, H-6), 7.45-7.35 (1H, 2d, ³*J*=14 Hz, H-5b), 7.40-7.30 (5H. m, CH₂*Ph*), 7.25-7.15 (2H, m, O*Ph*), 7.05-6.95 (2H, m, O*Ph*), 6.71 (1H, 2d, ³*J*=14 Hz, H-5a), 6.27 (1H, 2t, ³*J*=6Hz, H-1'), 5.15 (1H, s, C*H₂*Ph), 4.45 (1H, m, H-3'), 4.40-4.30 (2H, m, H-5') 4.20-4.05 (2H, m, H-4', N*H*), 2.5-2.4 (1H, m, one of H-2'), 2.25-1.9 (5H, m, one of H-2'+4H cyclopeutane), 1.8-1.6 (4H, m, 4H cyclopentane).
¹³C-NMR (CDCl₃, 75 MHz): δ 24.5, 24,3, 24.2 (2CH₂ cyclopent), 39.7, 39.6, 39.3, 39.2 (2CH₂ cyclopent), 40.5, 40.0 (C-2'), 66.6 (*Cq c*yclopentane), 67.2, 66.7 (C-5'), 67.9 (*C*H₂Ph), 70.8, 70.7 (C-3'), 85.3, 85.7, 85.4, 85.3 (C-1', C-4'), 110.3 (C-5b), 111.8 (C-5), 116.9, 116.6 ('o', O*Ph*), 122.2, 122.1 ('*m*', *OPh*)*,* 129.0, 128.9, 128.6, 128.5 (Bn, C-5a), 135.8('*ipso*', CH₂*Ph*) 138.5 (C-6), 146.8, 146.7 ('*ipso*', *OPh*)*,* 149.9 (C-4), 158,5 ('*p*' OPh), 162.2 (C-2), 175.7, 175.0 (*C*OOBn),

### Synthesis of illustrative example (E)-5-(2-Bromovinyl)-2'-deoxyuridine-5'-[para-chlorophenyl-(methoxy-α,α-cycloleucinyl)]-phosphate (CPF 32).

**C₂₄H₂₈BrClN₃O₉P, MW=648,82.**

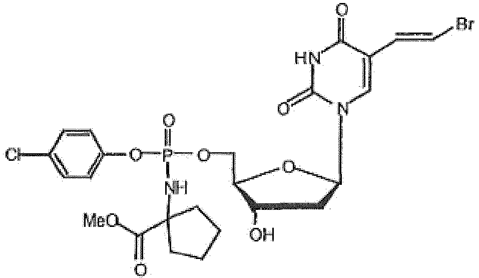

This was synthesised according to ***Standard procedure 5***, using BVdU (150 mg, 0.45 mmol), para-chlorophenyl-(methoxy-α,α-cycloleucinyl)-phosphorochloridate (475 mg, 1.35 mmol), NMI (4.5 mmol, 300 µL) in THF (5 mL) for 2 hrs. The crude product was purified by column chromatography, eluting with CH₂Cl₂/Methanol 97:3 to give the pure product as a white foamy solid (187 mg, yield 64%).
³¹P-NMR (MeOD, 121 MHz): δ 4.64.
¹H-NMR (MeOD; 300 MHz): δ 7.75 (1H, 2xs, H-6), 7.32 (1H, 2d, ³*J*=14 Hz, H-5b), 7.32-7.27(2H, m, O*Ph*), 7-20-7.11 (2H,m, O*Ph*), 6.72 (1H,2d, ³*J*=14 Hz, H-5a), 6.27-6.20 (1H, 2t, ³*J*=6 Hz, H1'), 4.35 (1H, m, H-3'), 4.30 (2H, m, H-5') 4.1 (2H, m, H-4'), 3.72 (3H, 2s, CH₃O), 2.32-2.20 (1H, m, one of H-2'), 2.20-1.92 (5H, m, one of H-2'+4H cyclopentane), 1.8-1.6 (4H, m, 4H cyclopentane).
¹³C-NMR (MeOD; 75 MHz): δ 25.7, 25.6 (2CH₂ cyclopent), 41,7, 41.6, 41.4, 41.3 (2CH₂ cyclopent), 42.7 (C-2'), 54.1, 53.9 (*C*H₃O), 67.8 (*Cq* cyclopentane), 69.1, 69.0 (C-5'), 73.8 (C-3'), 884, 8.3, 88.2 (C-1', C-4'), 110.2 (C-5b), 111.8 (C-5), 122.1, 121.9 ('*o*', O*Ph*), 128.9 (C-5a), 130.6 ('*m*', O*Ph*), 130.8 ('*p*', O*Ph*), 138.5 (C-6), 149.5, 149.4 ('*ipso*', *OPh*), 149.9 (C-4), 162.2(C-2), 175.6 (*C*OOCH₃).

### Synthesis of illustrative example (E)-5-(2-Bromovinyl)-2'-deoxyuridine-5'-[para-chlorophenyl-(ethoxy-α,α-cycloleucinyl)]-phosphate (CPF 33).

**C₂₅H₃₀BrClN₃₁O₉P, MW=662.85.**

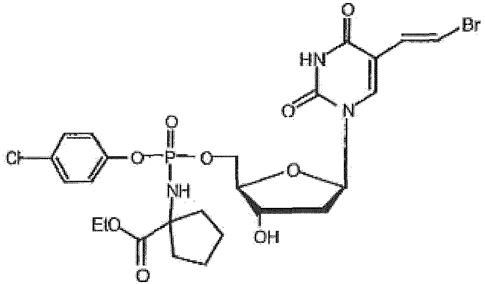

This was synthesized according to ***Standard procedure 5***, using BVdU (150 mg, 0.45 mmol), para-chlorophenyl-(ethoxy-α,α-cycloleucinyl)-phosphorochloridate (495 mg, 1.35 mmol), NMI (4.5 mmol, 300 µL) in THF (5 mL) for 2 hrs. The crude product was purified by column chromatography, eluting with CH₂Cl₂/Methanol 97:3 to give the pure product as a white foamy solid (240 mg, yield 66%).
³¹P-NMR (CDCl₃, 121 MHz): δ 4.15.
¹H-NMR (CDCl₃, 300 MHz): δ 10.25-10,1 (1H, bs, H-3), 7.65 (1H, 2xs, H-6), 7.4-7.3 (1H, 2d, ³*J*=14 Hz, H-5b), 7.25-7,20 (2H, m, O*Ph*). 7.20-7.10 (2H, m, O*Ph*)*,* 6.75 (1H, 2d, ³*J*=14 Hz, H-5a), 6.20 (1H, m, H1'), 4,3 5 (3H, m, H-3', H-5'), 4.2-4.0 (4H, m, H-4', NH, CH₃C*H₂*O), 2.45-2.25 (1H, m, one of H-2% 2.25-1.85 (5H, m, one of H-2'+4H cyclopentane), 1.75-1.55 (4H, m, 4H cyclopentane), 1.2 (3H, 2t, ³*J*=7 Hz, C*H₃*CH₂O).
¹³C-NMR (CDCl₃, 75 MHz): δ 14.5 (*C*H₃CH₂O), 24.5, 24,4 (2CH₂ cyclopent), 39.3, 39.2, 38.8, 38.6 (2CH₂ cyclopent), 40.5 (C-2'), 62.3 (CH₃*C*H₂O), 66.1 (*Cq* cyclopentane), 66.7 (C-5'), 70.8 (C-3'), 85.8, 85.4 (C-1', C-4'), 110.3 (C-5b), 111.9 (C-5), 122.1, 121.9 ('o', O*Ph*), 129.0 (C-5a), 130.2 ('*m*', *OPh),* 130.8 ('*p*', O*Ph*)*,* 138.5 (C-6), 149.5, 149.4 ('*ipso*', O*Ph*), 149.9 (C-4), 162.3 (C-2), 175.9 (*C*OOCH₂CH₃).

### Synthesis of illustrative example (E)-5-(2-Bromovinyl)-2'-deoxyuridine-5'-[para-chlorophenyl-(benzoxy-a,a-cycloleucinyl)]-phosphate (CPF 34).

**C₃₀H₃₂BrClN₃O₉P, MW=724.92.**

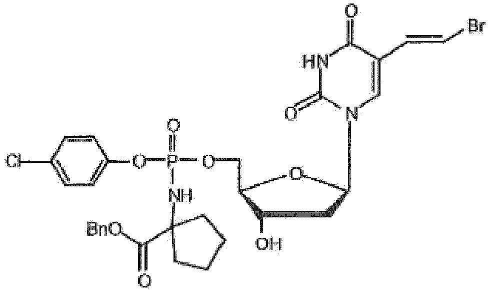

This was synthesised according to ***Standard procedure 5***, using BVdU (150 mg, 0.45 mmol), para-chlorophenyl-(benzyloxy-α,α-cycloleucinyl)-phosphorochloridate (578 mg, 1.35 mmol), NMI (4.5 mmol, 300 µL) in THF (5 mL) for 2 hrs. The crude product was purified by column chromatography, eluting with CH₂Cl₂/Methanol 97:3 to give the pure product as a white foamy solid (222 mg, yield 68%),
³¹P-NMR (CDCl₃, 121 MHz): δ 4.11, 4.05.
¹H-NMR (CDCl₃, 300 MHz): δ 7.65 (1H, 2xs, H-6), 7.45-7.29 (10H, m, H-5b, 2H O*Ph*+CH₂*Ph*), 7.20-7.15 (2H, m, O*Ph*), 6.75-6.67 (1H, 2d, ³*J*=14 Hz, H-5a), 6.28 (1H, 2t, ³*J*=6Hz, H-1'), 5.15 (1H, 2s, C*H₂*Ph), 4.5 (1H, m, H-3'), 4.35 (2H, m, H-5') 4.1 (H, m, H-4'), 4.00 (1H, m, N*H*), 2.48-2.35 (1H, m, one ofH-2'), 2.3-1.92 (5H, m, one of H-2'+4H cyclopentane), 1.8-1.6 (4H, m, 4H cyclopentane).
¹³C-NMR (CDCl₃, 75 MHz): δ 24.5, 24.4, 24,3, 24.2 (2CH₂ cyclopent), 39.3, 38.8, 38.6 (2CH₂ cyclopent), 40.5 (C-2'), 66.7 (*Cq* cyclopentane), 67.9 (*C*H₂Ph), 68.4 (C-5'), 70.7 (C-3'), 85.7 , 85.7, 85.4, 85,3 (C-1', C-4'), 110.3 (C-5b), 111.8 (C-5), 122.0, 121.9 ('o', *OPh*)*,* 129.1, 128.3, 128.2 (Bn*,* '*m*', O*Ph*), 130.2 (C-5a), 135.8 ('*ipso*', CH₂*Ph*), 136.3 ('*p*' OPh), 138.2 (C-6), 149.5, 149.3 ('*ipso*', O*Ph*), 149.9 (C-4), 162.2 (C-2), 175.7, 175.5 (*C*OOBn).

### Synthesis of illustrative example (E)-5-(2-Bromovinyl)-2'-deoxyuridine-5'-[para-trifluorophenyl-(methoxy-α,α-cycloleucinyl)]-phosphate (CPF 28).

**C₂₅H₂₈BrF₃N₃O₉P, MW=682.38.**

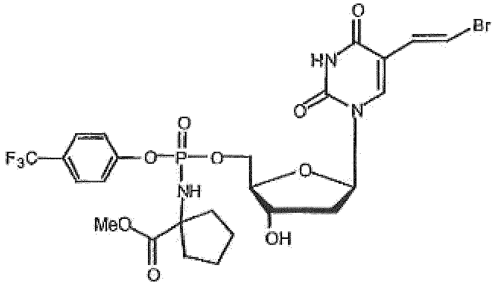

This was synthesized according to ***Standard procedure 5***, using BVdU (150 mg, 0.45 mmol), para-trifluorophenyl-(methoxy-α,α-cycloleucinyl)-phosphorochloridate (521 mg, 1.35 mmol), NMI (4.5 mmol, 300 µL) in THF (5 mL) for 2 hrs. The crude product was purified by column chromatography, eluting with CH₂Cl₂/Methanol 97:3 to give the pure product as a white foamy solid (199 mg, yield 65%).
³¹P-NMR (CDCl₃, 121 MHz): δ 3.80.
¹H-NMR (CDCl₃; 300 MHz): δ 7.70 (1H, 2s, H-6), 7.55 (1H, 2d, ³*J*=14 Hz, H-5b), 7.45-7.32 (4H, m, O*Ph*), 6.72 (1H, 2d, ³*J*=14 Hz, H-5a), 6.28 (1H, 2t, ³*J*=6Hz, H1'), 4.55 (1H, m, H-3'), 4.45 (2H, m, H-5'), 4.25 (1H, H-4'), 4.15 (1H, NH), 3.71 (3H, 2s, CH₃O), 2.6-2.4 (1H, m, one of H-2'), 2.3-1.9 (5H, m, one of H-2'+4H cyclopentane), 1.85-1.6 (4H, m, 4H cyclopentane).
¹³C-NMR (CDCl₃; 75 MHz): δ 24.4, 24,3, 24.2 (2CH₂ cyclopent), 39.2, 39.1, 38.8, 38.6 (2CH₂ cyclopent), 40.5 (C-2'), 53.9 (*C*H₃O), 66.3 (*Cq* cyclopentane), 66.8 (C-5'), 70.9 (C-3'), 85.8, 85.4 (C-1', C-4'), 110.3 (C-5b), 111.9 (C-5), 125.1 (d, J=270Hz, CF₃), 127.1, 127.0 ('*o*', O*Ph*), 127.8 ('*m*', O*Ph*), 128.9 (C-5a), 129,0 ('*p*', q, J=32Hz, O*Ph*), 138.5 (C-6), 149.9 (C-4), 153.5 ('*ipso*', O*Ph*), 162.2 (C-2), 176.3, 176.2 (*C*OOCH₃).

### Synthesis of illustrative example (E)-5-(2-Bromovinyl)-2'-deoxyuridine-5'-[para-trifluorophenyl-(ethoxy-α,α-cycloleucinyl)]-phosphate (CPF 29).

**C₂₆H₃₀BrF₃N₃O₉P, MW=696.40.**

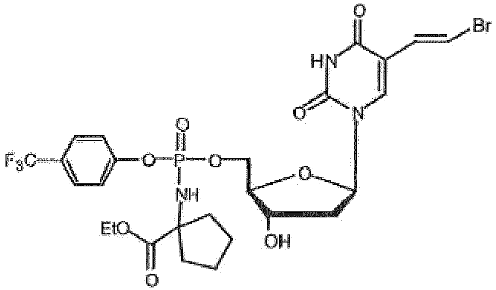

This was synthesised according to ***Standard procedure 5***, using BVdU (150 mg, 0.45 mmol), para-trifluorophenyl-(ethoxy-α,α-cycloleucinyl)-phosphorochloridate (540 mg, 1.35 mmol), NMI (4.50 mmol, 300 µL) in THF (5 mL) for 2 hrs. The crude product was purified by column chromatography, eluting with CH₂Cl₂/Methanol 97:3 to give the pure product as a white foamy solid (185 mg, yield 59%).
³¹P-NMR (CDCl₃, 121 MHz): δ 4.30.
¹H-NMR (CDCl₃, 300 MHz): δ 10.35 (1H, bs, H-3), 7,70 (1H, 2xs, H-6), 7.40 (1H, 2d, ³*J*=14 Hz, H-5b), 7.28-7.14 (2H, m, O*Ph*), 7.05-6.95 (2H, m, O*Ph*), 6.70 (1H, 2d, ³*J*=14 Hz, H-5a), 6.3 (1H, m, H1'), 4.55-4.3 (3H, m, H-5', H-3'), 4.2-4.1 (3H, m, H-4', CH₃C*H₂*O), 2.5-2.35 (1H, m, one of H-2'), 2.20-1.9 (5H, m, one of H-2'+4H cyclopentane), 1.85-1.6 (4H, m, 4H cyclopentane), 1.25 (3H, 2t, ³*J*=7 Hz, C*H₃*CH₂O).
¹³C-NMR (CDCl₃, 75 MHz): δ 14.5 (*C*H₃CH₂O), 24.5, 24,4 (2CH₂ cyclopent), 39.3, 39.2, 38.9, 38.5 (2CH₂ cyclopent), 40.6 (C-2'), 62.2 (CH₃*C*H₂O), 66.7 (*Cq* cyclopentane), 67.4, 67.3 (C-5'), 70.9 (C-3'), 85.8, 85.7 (C-1', C-4'), 110.2 (C-5b), 111,9 (C-5), 116.8, 116.5 ('o', O*Ph*), 122.2, 122.1 ('*m*', O*Ph*), 125.1 (d, J=270Hz, CF₃), 129.0 (C-5a), 131.1 ('*p*', q, J=32Hz, O*Ph*), 138.5 (C-6), 146.8, 146.7 ('*ipso*', O*Ph*), 149.9 (C-4), 162.3 (C-2), 175.9,175.8 (*C*OOCH₂CH₃).

### Synthesis of illustrative example (E)-5-(2-Bromovinyl)-2'-deoxyuridine-5'-[para-trifluorophenyl-(benzoxy-α,α-cycloleucinyl)]-phosphate (CPF 30).

**C₃₁H₃₂BrF₃N₃O₉P, MW=758.47.**

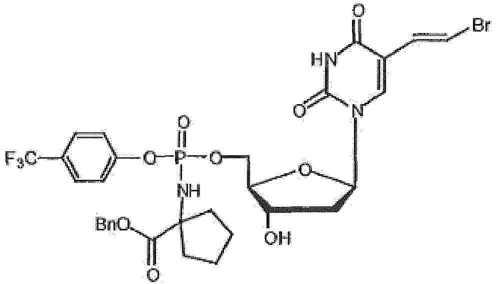

This was synthesized according to ***Standard procedure 5***, using BVdU (150 mg, 0.45 mmol), para-trifluorophenyl-(benzyloxy-α,α-cycloleucinyl)-phosphorochloridate (623 mg, 1.35 mmol), NMI (4.5 mmol, 300 µL) in THF (5 mL) for 2 hrs. The crude product was purified by column chromatography, eluting with CH₂Cl₂/Methanol 97:3 to give the pure product as a white foamy solid (218 mg, yield 64%).
³¹P-NMR (CDCl₃, 121 MHz): δ 4.30.
¹H-NMR (CDCl₃, 300 MHz): δ 10.35 (1H, bs, H-3), 7.65 (1H, 2xs, H-6), 7.55 (2H, m, 2H O*Ph*), 7.45-7.25 (8H. m, 2H O*Ph*+CH₂*Ph*+H-5b), 6.7 (1H, 2d, ³*J*=14 Hz, H-5a), 6.30 (1H, 2t, ³*J*=6Hz, H-1'), 5.15 (1H, 2.s, C*H₂*Ph), 4.55-4.35 (3H, m, H-3'+ H-5'), 4.25 (1H, H-4'), 4.10 (1H, NH), 2.55-2.35 (1H, m, one of H-2'), 2.30-1.92 (5H, m, one of H-2'+4H cyclopentane), 1.8-1.6 (4H, m, 4H cyclopentane),
¹³C-NMR (CDCl₃, 75 MHz): δ 25.5, 24.4, 24,3, 24.2 (2CH₂ cyclopent), 39.2,39.1, 38.7, 38.6 (2CH₂ cyclopent), 40.5, 40.0 (C-2'), 66.4 (*Cq* cyclopentane), 66.8 (C-5'), 68.0 (*C*H₂Ph), 70.9 (C-3'), 86.0, 85.8, 85.4, 85.3 (C-1', C-4'), 110.3 (C-5b), 111.9 (C-5), 121.8, 120.8 ('o, *m*', O*Ph*), 125.2 (d, J=270Hz, CF₃), 128.5, 127.7, 127.5 (Bn, C-5a), 129,2 ('*p*',q , J=32Hz, *OPh*), 135.4 ('*ipso*', CH₂*Ph*), 138.5 (C-6), 149.9 (C-4), 153.5 ('*ipso*' OPh), 162.2 (C-2), 175.6,175.5 (*C*OOBn).

### Synthesis of illustrative example (E)-5-(2-Bromovinyl)-2'-deoxyuridine-5'-[phenyl-(methoxy-L-phenylalaninyl)]-phosphate (CPF 36).

**C₂₇H₂₉BᵣN₃O₉P, MW=650.41.**

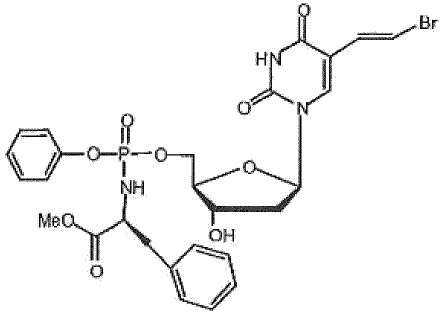

This was synthesised according to ***Standard procedure 5***, using BVdU (150 mg, 0.45 mmol), Phenyl-(methoxy-L-phenylalaninyl)-phosphorochloridate (477 mg, 1.35 mmol), NMI (4.42 mmol, 190 µL) in THF (5 mL) for 2 hrs. The crude product was purified by column chromatography, eluting with CH₂Cl₂/Methanol 97:3 to give the pure product as a white foamy solid (169 mg, yield 58%),
³¹P-NMR (CDCl₃, 121 MHz): δ 4.79, 4.71.
¹H-NMR (CDCl₃; 300 MHz): δ 9.95 (1H, bs, H-3), 7.60-7.55 (1H, 2xs, H-6), 7.48-7.4 (1H, 2d, ³*J*=14 Hz, H-5b), 7.3-7.1 (10H, m, CH₂Ph+ O*Ph*), 6,75-6,65 (1H, 2d, ³*J*=14 Hz, H-5a), 6.27-6.18 (1H, m, H1'), 4.57-4,29 (6H, m, H-5',H-3',H-4', NH, CHphenylala), 3.70 (3H, 2s, CH₃O), 3.01 (2H, m, CH₂Ph), 2.35-2.20 (1H, m, one of H-2'), 2.07-1.95 (1H, m, one of II-2').
¹³C-NMR (CDCl₃; 75 MHz): δ 36.3 (CH₂phenylalanine), 41.9, 41.8 (C-2'), 53.0 (*C*H₃O), 56.6, 56.1 (CHphenylala), 67.1 (C-5'), 713, 70.7 (C-3'), 85.7, 85.6, 85.5, 85.4 (C-1', C-4'), 110.4 (C-5b), 111.9 (C-5), 120.6,120.5 ('*o*', O*Ph*), 127.8 ('*p*', *OPh*), 130.1, 129.9, 129.8, 129.1 (CH₂Ph, C-5a, '*m*' O*Ph*), 138.0, 137.9 (C-6), 149.8 (C-4), 150.7,150.6 ('*ipso*', O*Ph*), 162.1, 162.0 (C-2), 173.5 (*C*OOCH₃).

### Synthesis of illustrative example (E)-5-(2-Bromovinyl)-2'-deoxyuridine-5'-[phenyl-(methoxy-L-leucinyl)]-phosphate (CPF 35).

**C₂₄H₃₁BrN₃O₉P, MW=616,40.**

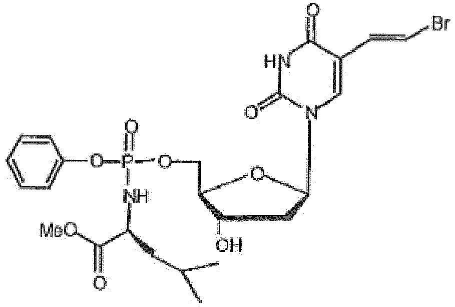

This was synthesised according to ***Standard procedure 5***, using BVdU (150 mg, 0.45 mmol), Phenyl-(methoxy-L-leucinyl)-phosphorochloridate (432 mg, 1.35 mmol), NMI (4.42 mmol, 190 µL) in THF (5 mL) for 2 hrs. The crude product was purified by column chromatography, eluting with CH₂Cl₂/Methanol 97:3 to give the pure product as a white foamy solid (167 mg, yield 60%).
³¹P-NMR (CDCl₃, 121 MHz): δ 5.14, 4,60.
¹H-MMR (CDCl₃; 300 MHz): δ 10.1 (1H, bs, II-3), 7.75 (1H, 2xs, H-6), 7.45 (1H, 2d, ³*J*=14 Hz, H-5b), 7.4-7.2 (5H, m, O*Ph*), 6.85 (1H, 2d, ³*J*=14 Hz, H-5a), 6.27-6.18 (1H, 2t, ³*J*=6 Hz, H1'), 4.5-4.2 (4H, m, H-5',H-3', NH), 4.1 (1H, m,H-4'), 3.95 (1H, m, C*H*CH₂CH(CH₃)₂), 3.70 (3H, 2s, CH₃O), 2.40-2.20 (1H, m, one of H-2'), 2-05-1.95 (1H, m, one of H-2'), 1.8 (1H, m, CHCH₂C*H*(CH₃)₂), 1.8-1.5 (2H, m, CHC*H₂*(CH₃)₂), 1.0-0.9 (6H, m, CHCH₂CH(C*H₃*)*₂*.
¹³C-NMR (CDCl₃; 75 MHz): δ 23.2, 23.1, 22.0, 21.9 (2C, CHCH₂CH(*CH₃*)*₂*), 24.9, 24.7 (CHCH₂C*H*(*CH₃*)*₂*), 40.6 (C-2'), 43.7, 43.6 (CHC*H₂*(CH₃)₂), 53.0 (*C*H₃O), 53.7, 53.6 (C*H*CH₂*CH*(C*H*₃)₂) 66.6, 66.3 (C-5'), 71,1, 70.8 (C-3'), 86.0, 85.7, 85.6, 85.5 (C-1', C-4'), 110.4 (C-5b), 111.9 (C-5), 120.6, 120,5, 120.4 ('*o',* O*Ph*), 125.8, 125.7 ('*p*', O*Ph*), 128.9 (C-5a), 130.2 ('*m' OPh*), 138.1 (C-6), 149.9 (C-4), 150.8. 50.7 (*'ipso',* O*Ph*), 162 2 (C-2), 175.1, 174.9 (*C*OOH₃).

### Synthesis of illustrative example (E)-5-(2-Bromovinyl)-2'-deoxyuridine-5'-[phenyl-(benzoxy-L-leucinyl)]-phosphate (CPF 37).

**C₃₀H₃₅BrN₃O₉P, MW=692.49.**

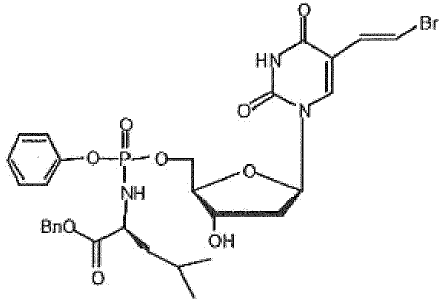

This was synthesised according to ***Standard procedure 5***, using BVdU (150 mg, 0.45 mmol), Phenyl-(benzoxy-L-leucinyl)-phosphorochloridate (534 mg, 1.35 mmol), NMI (4.42 mmol, 190 µL) in THF (5 mL) for 2 hrs. The erode product was purified by column chromatography, eluting with CH₂Cl₂/Methanl 97:3 to give the pure product as a white foamy solid (199 mg, yield 64%).
³¹P-NMR (CDCl₃, 121 MHz): δ 5.18, 4.54.
¹H-NMR (CDCl₃; 300 MHz): δ 9.95-9.85 (1H, bs, H-3), 7.55 (1H, 2xs, H-6), 7.38 (1H, 2d, ³*J*=14 Hz, H-5b), 7.3-7.1 (5H,m, CH₂Ph+ O*Ph*)*,* 6.65 (1H, 2d, ³*J*=14 Hz, H-5a), 6.26-6.14 (1H, 2t, ³*J*=6 Hz, H1'), 5.1 (2H, 2s, CH₂Ph) 4.,4-3.8 (6H, m, H-5',H-3', NH, H-4', C*H*CH₂CH(CH₃)₂), 2.35-2.25 (1H, m, one of H-2'), 1.95-1.85 (1H, m, one of H-2'), 1.6-1.4 (3H, m, CHC*H₂*C*H*(CH₃)₂), 0.8 (6H, m, CHCH₄CH(*CH₃*)*₂*).
¹³C-NMR (CDCl₃; 75 MHz): δ 23.2, 23.1, 22,0, 21.9 (2C, CHCH₂CH(*CH₃*)*₂*) 24.9, 24.7 (CHCH₂*CH*(*CH₃*)*₂*), 40.7 (C-2'), 43.9, 43.8 (CH*CH₂*CH(CH₃)₂), 53.9, 53.7 (*CH*CH₂CH(CH₃)₂), 66.4, 66.2 (C-5'), 67.8 ,67.7 (*CH₂*Ph), 71.1, 70.7 (C-3'), 85.9, 85.6, 85.4, 85.3 (C-1', C-4'), 110.4 (C-5b), 111.9 (C-5), 120.6, 120.5 ('*o*', O*Ph*), 125.8, 125.7 ('*p*', O*Ph),* 130.2, 129.1, 128.9 (C-5a, *CH₂*Ph, '*m*' O*Ph),* 135,4 ('*ipso*', CH₂*Ph*), 138.1 (C-6), 149.8 (C-4), 150.2 ('*ipso*', O*Ph*), 162.1 (C-2), 175.7, 174.6 (*C*OOBn).

### Synthesis of illustrative example (E)-5-(2-Bromovinyl)-2'-deoxyuridine-5'-[para-nitrophenyl-(benzoxy-L-leucinyl)]-phosphate (CPF 38).

**C₃₀H₃₄BrN₄O₁₁P, MW-737.49.**

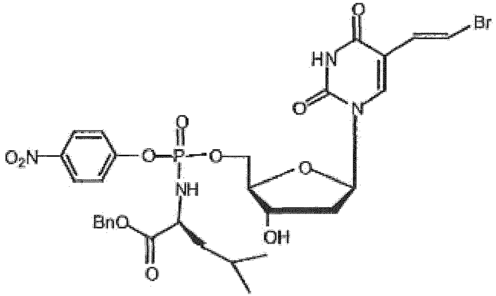

This was synthesised according to *Standard procedure* 5, using BVdU (150 mg, 0.45 mmol), para-nitrophenyl-(benzoxy-L-leucinyl)-phosphorochloridate (595 mg, 1.35 mmol), NM1 (4.42 mmol, 190 µL) in THF (5 mL) for 2 hrs. The crude product was purified by column chromatography, eluting with CH₂Cl₂/Methanol 97:3 to give the pure product as a white foamy solid (176 mg, yield 53%).
³¹P-NMR (CDCl₃, 121 MHz): δ 5.72, 4.35.
¹H-NMR (CDCl₃; 300 MHz): δ 10.2 (1H, bs, H-3), 8.1(2H, m, 2H O*Ph*), 7.65 (1H, 2xs, H-6), 7.45-7.2 (8H, m, H-5b, CH₂Ph+ 2H O*Ph*), 6.65 (1H, 2d, ³*J*=14 Hz, H-5a), 6.35-6.2 (1H, 2t, ³*J*=6 Hz, H1'), 5.15 (2H, 2s, CH₂Ph) 4,7-3.9 (6H, m, H-5',H-3', NH, H-4', C*H*CH₂CH(CH₃)₂), 2.55-2.4 (1H, m, one of H-2'), 2,15-2.05 (1H, m, one of H-2'), 1.7-1.5 (3H, m, CHCH₂C*H*(CH₃)₂), 0.95-0.8 (6H, m, CHCH₂CH(*CH₃*)*₂*).
¹³C-NMR (CDCl₃; 75 MHz): δ 23.2, 23.1, 22.0, 21.9 (2C, CHCH₂CH(*CH₃*)*₂*),24.9, 24.8 (CHCH₂*CH*(*CH₃*)*₂*), 40.6 (C-2'), 43.7, 43.6 (CH*CH₂*CH(CH₃)₂), 53.9, 53.7 (*CH*CH₂CH(CH₃)₂), 66.9 (C-5'), 67.9 (*CH₂*Ph), 71.2, 70.8 (C-3'), 85.8, 85.3, 85.2 (C-1', C-4'), 110.6 (C-5b), 111.9 (C-5), 121.3 ('*o*', O*Ph*), 129,2, 129.1, 128.8, 126.2 (C-5a, *CH₂*Ph*,* '*m*' O*Ph*), 135.4, 135.3 (*'*i*pso*', CH₂*Ph*), 138.2 (C-6), 145,2, 145,1 ('*ipso*', O*Ph*)*,* 149.9 (C-4), 155.5 ('*p*', O*Ph*), 162.1 (C-2), 274.2 (*C*OOBn).

### Synthesis of illustrative example (E)-5-(2-Bromovinyl)-2'-deoxyuridine-5'-[para-chlorophenyl-(benzoxy-L-leucinyl)]-phosphate (CPF 39).

**C₃₀H₃₄BrCIN₃O₉P, MW-726.94.**

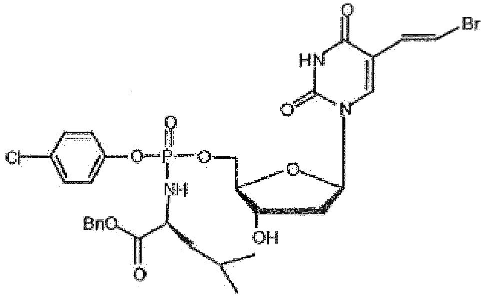

This was synthesized, according to ***Standard procedure 5***, using BVdU (150 mg, 0.45 mmol), para-chlorophenyl-(benzoxy-L-leucinyl)-phosphorochloridate (581 mg, 1.35 mmol.), NM1 (4.42 mmol, 190 µL) in THF (5 mL) for 2 hrs. The crude product was purified by column chromatography, eluting with CH₂Cl₂/Methanol 97:3 to give the pure product as a white foamy solid (221 mg, yield 68%).
³¹P-NMR(CDCl₃, 121 MHz): δ 5.27, 4.76.
¹H-NMR (CDCl₃; 300 MHz): δ 10.25-10.15 (1H, bs, H-3), 7.65 (1H, 2xs, H-6), 7,45 (1H, 2d, ³*J*=14 Hz, II-5b), 7.4-7.15 (9H, m, CH₂Ph+ O*Ph*), 6.7 (1H, 2d, ³*J*=14 Hz, H-5a), 6.35-6.2 (1H, 21, ³*J*=6 Hz, H1'), 5.15 (2H, 2s, CH₂Ph) 4.55-3.9 (6H, m, H-5', H-3', NH, H-4', C*H*CH₂CH(CH₃)₂), 2.5-2.4 (1H, m, one of H-2'), 2.15-.2.0 (1H, m, one of H-2'), 1.7-1,45 (3H, m, CHCH₂C*H*(CH₃)₂), 0.94-0.82 (6H, m, CHCH₂CH(*CH₃*)*₂*).
¹³C-NMR (CDCl₃; 75 MHz): δ 23.1, 23.0, 22.2, 22.0 (2C, CHCH₂CH(*CH₃*)*₂*), 24.9, 24.7 (CHCH₂*CH*(*CH₃*)*₂*), 40.7 (C-2'), 43.9, 43.8 (CH*CH*₂CH(CH₃)₂), 53.9, 53.7 (*CH*CH₂CH(CH₃)₂), 66.7, 66.3 (C-5'), 67.8 (*CH*₂Ph), 71.1, 70.7 (C-3'), 85.8, 85.7, 85.4 (C-1', C-4'), 110.5 (C-5b), 111.9 (C-5), 122.1, 122.0 ('*o*', O*Ph*), 130,2, 129.1, 129.0 (C-5a, CH*₂*Ph, '*m*' O*Ph*)*,* 131,1, 130.9 ('*p*', O*Ph*), 135.5, 135.4 ('*ipso*', CH₂*Ph*), 138.2 (C-6), 149.2, 149,1 ('*ipso*', *OPh*), 149.2, 149.1 (C-4), 162.2 (C-2), 174.2, 174.2 (*C*OOBn).

### Synthesis of Gemcitabine-[phenyl-(benzoxy-L-alaninyl)]-phosphate.

**C₂₅H₂₇F₂N₄O₈P, MW=580.47 (CPF 31).**

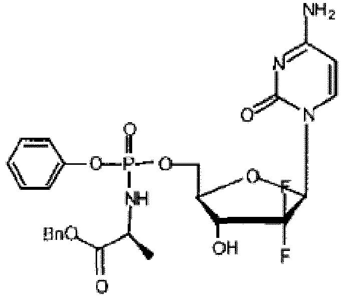

This was synthesized according to ***Standard procedure 5***, using gemcitabine (131 mg, 0,5 mmol), Phenyl-(benzoxy-L-alaninyl)-phosphorochloridate (529 mg, 1.5 mmol), NMI (4.42 mmol, 300 µL) in THF/pyridine (4/2 mL) for 2 hrs. The crude product was purified by column chromatography, eluting with CH₂Cl₂/Methanol 95:5 to give the pure product as a white foamy solid (46 mg, yield 16%).
³¹P-NMR (MeOD, 121 MHz): δ 5.05, 4.94.
¹H-NMR (MeOD, 300 MHz): δ 7.6-7.5 (1H, 2d, ³*J*=7Hz H-6), 7.4-7.2 (10H, m, O*Ph*+CH₂*Ph*), 6.25 (1H, m, H-1'), 5.95 (1H, 2d, ³*J*=7Hz, H-5), 5.19 (1H, 2s, C*H₂*Ph), 4-55-4.1(3H, m, H-3', H-4', CHala), 4.05 (2H, m, H-5'), 1.20 (3H, 2t, ³*J*=6 Hz, CH₃ala).
¹³C-NMR (MeOD, 75 MHz): δ 20.8, 20.7 (CH₃ala), 52.2, 52.0 (*C*Hala), 66.1 (C-5'), 68.4 (*C*H₂Ph), 71.9, 71.1 (C-3'), 80.6 (C-4'), 85.9 (C-1'), 97.1 (C-5), 121-8, 121.6 ('o', O*Ph*)*,* 123 (C-2'), 126.2 ('*p*', O*Ph*)*,* 131.8, 130.0, 129.7 ('*m*' OPh, Bn), 137.9('*ipso*', CH₂*Ph*), 142.7, 142.6 (C-6), 152.5, 152.4 ('*ipso*', O*Ph*), 158,2 (C-2), 168.0 (C-4), 175.3, 174.9 (*C*OOBn).

### Synthesis of Gemcitabine-[para-chlorophenyl-(benzoxy-L-alaninyl)-phosphate,

**C₂₅H₂₆ClF₂N₄O₈P, MW=614.92 (CPF 40).**

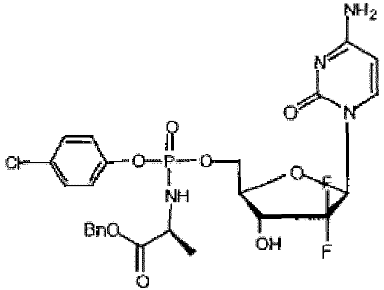

This was synthesised according to ***Standard procedure 5***, using gemcitabine (131 mg, 0.5 mmol), para-chlorophenyl-benzoxy-L-alaninyl)-phosphorochloridate (582 mg, 1.5 mmol), NMI (4.42 mmol, 300 µL) in THF/pyridine (4/2 mL) for 2 hrs. The crude product was purified by column chromatography, eluting with CH₂Cl₂/Methanol 95:5 to give the pure product as a white foamy solid (76 mg, yield 25%),
³¹P-NMR (MeOD, 121 MHz): δ 5.08.
¹H-NMR (MeOD, 300 MHz); δ 7.65 (1H, 2d, ³*J*=7Hz H-6), 7.5-7.2 (9H. m, O*Ph*+CH₂*Ph*), 6.2 (1H, m, H-1'), 5.9 (1H, 2d, ³*J*=7Hz, H-5), 5.12 (1H, 2s, C*H₂*Ph), 4.6-4.1 (3H, m, H-3', H-4', CHala), 4.05 (2H, m, H-5'), 1.45-1.35 (3H, 2t, ³*J*=6 Hz, CH₃ala).
¹³C-NMR (McOD, 75 MHz): δ 20.9, 20.7 (CH₃ala), 52.2, 52.0 (*C*Hala), 66.4, 66.2 (C-5'), 68.5 (*C*H₂Ph), 71.5 (C-3'), 80.7 (C-4'), 86,4 (C-1'), 97.2 (C-5), 123.5 ('o', O*Ph*), 126.9 (C-2'), 131.2, 130.6, 130.3 ('*m*' OPh, Bn), 131.9 ('*p*', O*Ph*) 137.5 ('*ipso*'*,* CH₂*Ph*), 142.8, 142.7 (C-6), 151.4, 151.0 ('*ipso*', O*Ph*), 158.2 (C-2), 166.9 (C-4), 175.1, 174.9 (*C*OOBn).

### Synthesis of Gemcitabine-[para-chlorophenyl-(benzoxy-α,α-dimethylglycinyl)]-phosphate (CPF 41).

**C₂₆H₂₈ClF₂N₄O₈P, MW=628.95.**

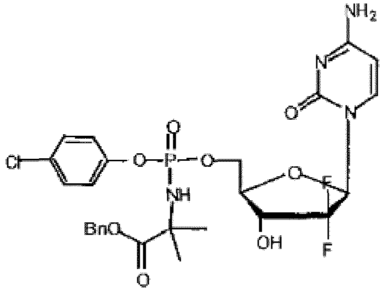

This was synthesised according to ***Standard procedure 5***, using gemcitabine (131 mg, 0,5 mmol), para-chlorophenyl-(benzoxy-α,α-dimethylglycinyl)-phosphorochloridate (603 mg, 1.5 mmol), NM1 (4.42 mmol, 300 µL) in THF/pyridine (4/3 mL) for 2 hrs. The crude product was purified by column chromatography, eluting with CH₂Cl₂/Methanol 95:5 to give the pure product as a white foamy solid (163 mg, yield 52%).
³¹P-NMR (MeOD, 121 MHz): δ 3.56, 3.52.
¹H-NMR (MeOD, 300 MHz): δ 7.55 (1H, 2d, ³*J*=7Hz, H-6), 7.4-7.15 (9H. m, O*Ph*+CH₂*Ph*), 6.25 (1H, m, H-1'), 5.85 (1H, 2d, ³*J*=7H₂, H-5), 5.15 (1R, 2s, C*H₂*Ph), 4.55-4.1 (3H, m, H-3', H-4'), 4.05 (2H, m, H-5'), 1.50 (6H, m, ³*J*=6 Hz, 2CH₃dimethygly).
¹³C-NMR (MeOD, 75 MHz): δ 28.2, 28.0 (CH₃ dimethygly), 58.6 (*C*q dimethygly), 66.2, 66.1 (C-5'), 66.7 (*C*H₂Ph), 71.5 (C-3'), 80.6 (C-4'), 86.4 (C-1'), 97.0 (C-5), 123.9, 123.6 ('o', O*Ph*), 127.3 (C-2'), 130.0, 129.7 ('*m*' OPh, Bn), 131.8 ('*p*', O*Ph*), 137.6 ('*ipso*', CH₂*Ph*), 142.8, 142.7 (C-6), 151.2, 151.1 ('*ipso*', O*Ph*), 158.1 (C-2), 167,9 (C-4), 176.8, 176.7 (*C*OOBn),

### Synthesis of Phenyl-(methoxy-L-alaninyl)-phosphorochloridate.

**C₁₀H₁₃ClNO₄P, MW-277.64.**

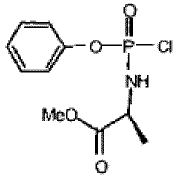

This is synthesised according to ***Standard procedure 4***, using L-alanine methyl ester hydrochloride (2 g, 14.3 mmol), phenyldichlorophosphate (3.02 g, 2.14 ml, 14.3 mmol), and TEA (2,9 g, 4.0 ml, 28.7 mmol) in DCM (60 mL), to yield 3.91 g (98%) of crude product used without further purification.
³¹P-NMR (CDCl₃, 121 MHz): δ 9.28, 8.97.
¹H-NMR (CDCl₃; 300 MHz): δ 7.39-7.34 (2H, m, 'o' O*Ph*), 7.29-7.20 (2H, m, 'm+p' O*Ph*), 4.98 (1H, bs, N*H*), 4.27-4.09 (1H, m, CHala), 3.78 (3H, s, OC*H₃*), 1.52-1.49 (3H, 2xd, ³*J*=7Hz, C*H₃*ala).
¹³C-NMR (CDCl₃; 75 MHz): δ 20.9 (*C*H₃ala), 51.0 (*C*Hala), 53.6 (O*C*H₃), 120.9 ('*o*' OPh), 126.4 ('*p*', OPh), 130.2 ('*m*', OPh), 150.1 ('*ipso*', OPh), 173.6(*C*OOCH₃).

### Synthesis of Phenyl-(ethoxy-L-alaninyl)-phosphorochloridate

**C₁₁H₁₅ClNO₄P, MW-291.67.**

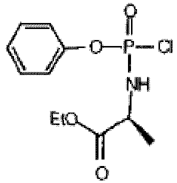

This is synthesised according to ***Standard procedure 4**,* using L-alanine ethyl ester hydrochloride (770 mg, 5.01 mmol), phenyldichlorophosphate (1.12g, 5.01 mmol, 749 (µL), and TEA (1.4 mL, 10.02 mmol) in DCM (40 mL). The crude was purified by flash chromatography (ethyl acetate/petroleum ether 7:3) affording 1.02 (69%) of oil.
³¹P-NMR (CDCl₃, 121 MHz): δ 9.49, 9.07.
¹H-NMR (CDCl₃; 300 MHz): δ 7.39-7.34 (2H, m, 'o' O*Ph*), 7.29-7.20 (2H, m, 'm+p' O*Ph*), 4.95 (1H, bs, N*H*), 4.3-4.1 (3H, m, OC*H₂*CH₃, CHala), 1.50 (3H, 2xd, ³*J*=7Hz, C*H₃*ala), 1.30 (3H, t, ³*J*=7.1 Hz, OCH₂-CH₃.
¹¹C-NMR (CDCl₃; 75 MHz): δ 14.5 (*C*H₃CH₂), 20.9 (*C*H₃ala), 51.0 (*C*Hala), 62.6 CH₃*C*H₂), 120.9 ('*o*' OPh), 126.5 ('*p*', OPh), 130.1 ('*m*'*,* OPh), 150.1 ('*ipso*'*,* OPh), 175.1 (*C*OOCH₂CH₃).

### Synthesis of Phenyl-(benzoxy-L-alaninyl)-phosphorochloridate.

**C₁₆H₁₇ClNO₄P, MW= 353.74.**

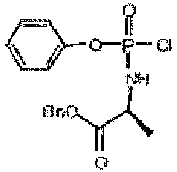

This is synthesised according to ***Standard procedure 4**,* using L-alanine benzyl ester hydrochloride (1.0 g, 4.64 mmol), phenyl-dichlorophosphate (980 mg, 0.69 ml, 4,64 mmol), and TEA (0.94 g, 1290 µL, 9.27 mmol) in DCM (40 mL). The crude was purified by flash chromatography (ethyl acetate/petroleum ether 6:4) affording 1.61 (98%) of oil.
³¹P-NMR (CDCl₃, 121 MHz): δ 9.41, 9.23.
¹H-NMR (CDCl₃; 300 MHz): δ 7.41-7.21 (10H, m, O*Ph*+*CH₂Ph*), 5.24 (2H, s, *CH₂*Ph), 4.95-4.88 (1H, bs, N*H*), 4.36-4.15 (1H, m, CHala), 1.52-1.49 (3H, 2xd, ³*J*=7Hz, C*H₃*ala).
¹³C-NMR (CDCl₃; 75 MHz): δ 20.8 (*C*H₃ala), 51.1 (*C*Hala), 68.0 (*C*H₂Ph), 121.0 ('*o*' OPh), 126.4 ('*p*', OPh), 130.3, 129,0, 128.7 ('*m*'OPh, CH₂*Ph*), 135.5 ('*ipso*', CH₂Ph), 150.2 ('*ipso*' OPh), 172.9 (*C*OOCH₂Ph).

### Synthesis of p-nitrophenyl-(methoxy-L-alaninyl)-phosphorochloridate.

**C₁₀H₁₂ClN₂O₆P, MW=322.64.**

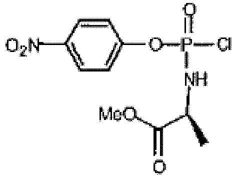

This is synthesised according to ***Standard procedure 4**,* using L-alanine methyl ester hydrochloride (0.70 g, 5.01 mmol), p-nitrophenyldichlorophosphate (1.362 g, 5.01 mmol), and TEA (1.4 ml, 10 mmol) in DCM (40 mL), to yield 1.60 g (99%) of crude product used without further purification.
³¹P-NMR (CDCl₃, 121 MHz): δ 9.13, 9.03.
¹H-NMR (CDCl₃; 300 MHz): δ 8.1 (2H, 2d, ³*J*=8Hz, O*Ph*), 7.3 (2H, 2d, ³*J*=8Hz, O*Ph*), 5.0 (1H,bs, N*H*), 4.1 (1H, m, CHala), 3.75 (3H, s, OC*H*₃), 1.5-1.45 (3H, m, C*H₃*ala).
¹³C-NMR (CDCl₃; 75 MHz): δ 20.8, 20.7 (*C*H₃ala), 51.1, 50.9 (*C*Hala), 53.2, 53.2 (O*C*H₃), 121.8, 121.6 ('*o*' OPh), 126.5 ('*m*', OPh), 145.7 ('*ipso*', OPh), 154.7, 154.6 ('*p*', OPh), 173.4, 173.2 (*C*OOCH₃).

### Synthesis of p-nitrophenyl-(ethoxy-L-alaninyl)phosphorochloridate.

**C₁₁H₁₄ClN₂O₆P, MW=336.67.**

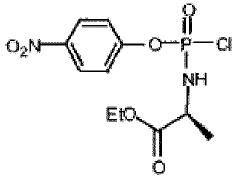

This is synthesised according to ***Standard procedure 4***, using L-alanine ethyl ester hydrochloride (770 mg, 5.01 mmol), p-nitrophenyldichlorophosphate (1.362g, 5.01 mmol), and TEA (1.4 mL, 10.02 mmol) in DCM (40 mL), to yield 1.64 g (98%) of crude product used without further purification.
³¹P-NMR (CDCl₃, 121 MHz): δ 9.06, 8.81.
¹H-NMR (CDCl₃; 300 MHz): δ 8.1 (2H, m, O*Ph*)*,* 7.4 (2H, m, O*Ph*), 4.9-4.7 (1H, bs, N*H*), 4.3-4.1 (3H, m, OC*H₂*CH₃, CHala), 1.55-1.45 (3H, 2xd, ³*J*=7Hz, C*H₃*ala), 1.40 (3H, t, ³*J*=7Hz, OCH₂C*H*₃).
¹³C-NMR (CDCl₃; 75 MHz): δ 14.5 (*C*H₃CH₂), 21.1, 20.9 (*C*H₃ala), 51.2, 51.0 (*C*Hala), 62.6CH₃*C*H₂), 121.7, 121.3 ('*o*' OPh), 126.2, 126.0 ('*m*', OPh), 145.7 ('*ipso*', OPh), 154.5 ('*p*', OPh), 173.4, 173.3 (*C*OOCH₂CH₃).

### Synthesis of p-nitrophenyl-(benzoxy-L-alaninyl)-phosphorochloridate.

**C₁₆H₁₆ClN₂O₆P, MW= 398.04.**

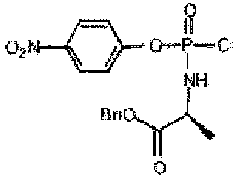

This is synthesised according to ***Standard procedure 4***, using L-alanine benzyl ester hydrochloride (1.08 g, 5.01 mmol), para-nitrophenyl-dichloro phosphate (1.362 g, 5.01 mmol), and TEA (1.4 mL, 1.4 mmol) in DCM (40 mL), to yield 1.85 g (93%) of crude product used without further purification.
³¹P-NMR (CDCl₃, 121 MHz): δ 9.15, 9.06.
¹H-NMR (CDCl₃; 300 MHz): δ 8.15 (2H, m, O*Ph*), 7.45 (2H, m,O*Ph*), 7.35-7.25 (5H, m, *CH₂Ph*), 5.2 (2H, 2s, *CH₂*Ph), 5.00 (1H, bs, N*H*), 4.2 (1H, m, CHala), 1.64 (3H, 2xd, ³*J*=7Hz, C*H₃*ala).
¹³C-NMR (CDCl₃; 75 MHz): δ 20.8 (*C*H₃ala), 51.1 (*C*Hala), 68.0 (*C*H₂Ph), 121.4 ('*o*' OPh), 126.1 ('*m*'OPh), 130.3, 129.0 (CH₂*Ph*), 145.7 ('*ipso*', CH₂Ph), 150.2 ('*ipso*', OPh), 154.6 ('*p*', OPh), 172.9 (*C*OOCH₂Ph).

### Synthesis of p-fluorophenyl-(methoxy-L-alaninyl)-phosphorochloridate.

**C₁₀H₁₂ClFNO₄P, MW=295.63.**

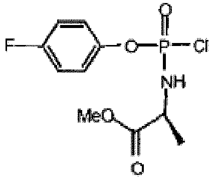

This is synthesised according to ***Standard procedure 4**,* using L-alanine methyl ester hydrochloride (0.70 g, 5.01 mmol), p-fluorophenyldichlorophosphate (1.210 g, 5.01 mmol), and TEA (1.4 ml, 10 mmol) in DCM (40 mL). The crude was purified by flash chromatography (ethyl acetate/petroleum ether 7:3) affording 1.11 g (75%) of oil.
³¹P-NMR (CDCl₃, 121 MHz): δ 9.98, 9.96.
¹H-NMR (CDCl₃; 300 MHz): δ 7.1 (2H, m, O*Ph*), 6.95 (2H, m, O*Ph*), 5.0 (1H, bs, N*H*), 4.25-4.1 (1H, m, CHala), 3,78 (3H, 2s, OC*H₃*), 1.55 (3H, m, C*H₃*ala).
¹³C-NMR (CDCl₃; 75 MHz): δ 20.8 (*C*H₃ala), 51.1, 50.9 (*C*Hala), 53.3 (O*C*H₃), 117.1, 117.0 ('*o*' OPh), 122.6, 122.5 ('*m*', OPh), 146.0 ('*ipso*', OPh), 159.1, 159.0 ('*p*', OPh), 173.4, 173.2 (*C*OOCH₃).

### Synthesis of p-fluorophenyl-(ethoxy-L-alaninyl)-phosphorochloridate.

**C₁₁H₁₄ClFNO₄P, MW=309.66.**

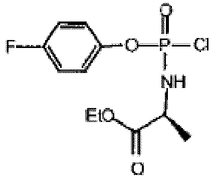

This is synthesised according to ***Standard procedure 4**,* using L-alanine ethyl ester hydrochloride (770 mg, 5.01 mmol), p-fluorophenyldichlorophosphate (1.210g, 5.01 mmol), and TEA (1.4 mL, 10.02 mmol) in DCM (40 ml). The crude was purified by flash chromatography (ethyl acetate/petroleum ether 7:3) affording 1.07 (69%) of oil.
³¹P-NMR (CDCl₃, 121 MHz): δ 10.01, 9.95.
¹H-NMR (CDCl₃; 300 MHz); δ 7.1 (2H, m, O*Ph*), 6.95 (2H, m, O*Ph*)*,* 5.0 (1H, bs, N*H*), 4.25-4.1 (3H, m, OC*H₂*CH₃, CHala), 1.55 (3H, m, C*H₃*ala), 1.40 (3H, t, ³*J*=7Hz, OCH₂C*H₃*.
¹³C-NMR (CDCl₃; 75 MHz): δ 14.5 (*C*H₃CH₂), 21.1, 21.0 (*C*H₃ala), 51.2, 51.1 (*C*Hala), 62.6_CH₃*C*H₂), 117.3 ('*o*' OPh), 122.2, 122.0 ('*m*', OPh), 145.9, 145.8 ('*ipso*', OPh), 159.0 ('*p*', OPh), 173.6, 173.5 (*C*OOCH₂CH₃).

### Synthesis of p-fluorophenyl-(benzoxy-L-alaninyl)-phosphorochloridate.

**C₁₆H₁₆ClFNO₄P, MW=371.73.**

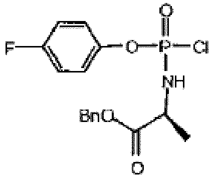

This is synthesized according to ***Standard procedure 4***, using L-alanine benzyl ester hydrochloride (1.08 g, 5.01 mmol), para-fluorophenyl-dichloro phosphate (1,210 mg, 5.01 mmol), and TEA (1.4mL, 1.4 mmol) in DCM (40 mL). The crude was purified by flash chromatography (ethyl acetate/petroleum ether 7:3) affording 1.599 (86%) of oil.
³¹P-NMR (CDCl₃, 121 MHz): δ 9.15, 9.06.
¹H-NMR (CDCl₃; 300 MHz): δ 7.35-7.25 (5H, m, *CH₂Ph*), 7.1 (2H, m, O*Ph*), 6.95 (2H ,m , O*Ph*), 5.2 (2H, 2s, *CH₂*Ph), 5.00 (1H, bs, N*H*), 4.25-4.1 (1H, m, CHala), 1.55 (3H, m, C*H₃*ala).
¹³C-NMR (CDCl₃; 75 MHz): δ 20.8 (*C*H₃ala), 51.1, 51.0 (*C*Hala), 68.1 (*C*H₂Ph), 117.0, 116.9 ('*o*' OPh), 122.6 ('*m*'OPh), 130.3, 129.0 (CH₂*Ph*), 135.7 ('*ipso*', CH₂Ph), 146.1, 146.0('*ipso*', OPh), 158.9 ('*p*', OPh), 173.1 (*C*OOCH₂Ph).

### Synthesis of 4-(trifluoromethyl)-phenyl-(methoxy-L-alaninyl)-phosphorochloridate.

**C₁₁H₁₂ClF₃NO₄P, MW=345.64.**

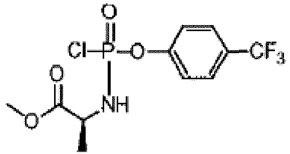

This is synthesised according to ***Standard procedure 4**,* using L-alanine methyl ester hydrochloride (1.0 g, 7.16 mmol), 4-(trifluoromethyl)-phenyl-phosphodichloridate (1.998 g, 7.16 mmol), and TEA (1.449 g, 14.32 mmol, 1916 µL) in DCM (30 mL), to yield 2.202 g (89.0%) of crude product used without further purification.
³¹P-NMR (CDCl₃, 121 MHz): δ 9.36, 9.22.
¹H-NMR (CDCl₃; 300 MHz): δ 7.66 (2H, d, ³*J*=8.1 Hz, O*Ph*), 7.44-7.33 (2H, m, O*Ph*), 5.10 (1H, bs, N*H*), 3.81-3.78 (3H, 2s, C*H₃*O), 3.77-3.68 (1H, m, CH₃C*H*), 1.56-1.52 (3H, m, CHC*H₃*).
¹³C-NMR (CDCl₃; 75 MHz): δ 20.6, 20.7 (*C*H₃CH), 50.9, 51.1 (*C*HCH₃), 53.2 (*C*H₃O), 121.4 ('*o*', O*Ph*), 124.1 (*CF₃*, *J*=270 Hz), 128.0 ('*m*', *OPh*), 128.6 ('*p*', *J*=34 Hz), 152.4, 152.6 ('*ipso*', O*Ph*), 173.4, 173.5 (*C*OOCH₃).

### Synthesis of 4-(trifluoromethyl)-phenyl-(ethoxy-L-alaninyl)phosphorochloridate.

**C₁₂H₁₄ClF₃NO₄, MW-359.67.**

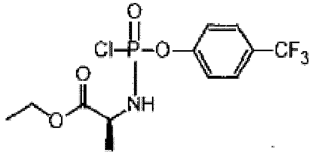

This is synthesised according to ***Standard procedure 4**,* using L-alanine ethyl ester hydrochloride (1.0 g, 6.50 mmol), 4-(trifluoromethyl)-phenyl-phosphodichloridate (1.813 g, 6.50 mmol), and TEA (1.316 g, 13.00 mmol, 1740 µL) in DCM (30 mL), to yield 2.150 g (92.2%) of crude product used without further purification.
³¹P-NMR (CDCl₃, 121 MHz): δ 9.33, 9.28.
¹H-NMR (CDCl₃; 300 MHz): δ 7.70 (2H, d, *³J*=8.2 Hz, O*Ph*), 7.46-739 (2H, m, O*Ph*), 4.78 (1H, bs, N*H*), 4.33-4.17 (3H, m, CH₃C*H₂*O+ C*H*CH₃), 1.59-1.55 (1H, m, CHC*H₃*), 1.56-1.52 (3H, m, CH₂C*H₃*).
¹³C-NMR (CDCl₃; 75 MHz): δ 14.5 (*C*H₃CH₂O), 20.8, 20.9 (*C*H₃CH), 50.3, 50.9 (*CH*CH₃), 62.3, 62.5 (CH₃*C*H₂O), 121.4 ('*o*', O*Ph*), 124.1 (*CF₃*, *J*=270 Hz), 127.7 ('*m*', O*Ph*), 128.7 ('*p*', *J*=33 Hz), 152.4 (*'ipso*', O*Ph*), 172.9 (*C*OOCH₂CH₃).

### Synthesis of p-trifluorophenyl-(benzoy-L-alaninyl)-phosphorochloridate.

**C₁₇H₁₆ClF₃NO₄P, MW= 421.73.**

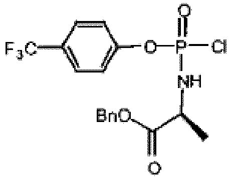

This is synthesized according to ***Standard procedure 4*,** using L-alanine benzyl ester hydrochloride (1.08 g, 5.01 mmol), para-trifluorophenyl-dichloro phosphate (1.490 mg, 5.01 mmol), and TEA (1.4 mL, 1.4 mmol) in DCM (40 mL). The crude was purified by flash chromatography (ethyl acetate/petroleum ether 6:4) affording 1.80 (85%) of oil.
³¹P-NMR (CDCl₃, 121 MHz): δ 9.11, 8.84.
¹H-NMR (CDCl₃; 300 MHz): δ 7.65 (2H, m, O*Ph*), 7.4-7.2 (7H, m, *CH₂Ph* + 2H O*Ph*), 5.25 (2H, 2s, *CH₂*Ph), 4.75-4.55 (1H, bs, N*H*), 4.25-4.1 (1H, m, CHala), 1.60-1.55 (3H, 2d, ³*J*=7Hz, C*H₃*ala).
¹³C-NMR (CDCl₃; 75 MHz): δ 20.9 (*C*H₃ala), 51.3, 51.0 (*C*Hala), 68.2, 68.1 (*C*H₂Ph), 121.4, 120.9 ('ο', O*Ph*), 125.2 (d, J=270Hz, CF₃), 126.6 ('*m*', O*Ph*), 129.1, 128.8, 127.8 (Bn), 130.0 ('*p*',q , J=32Hz, O*Ph*), 135.4 ('*ipso*', CH₂Ph), 153.0 ('*ipso*', OPh), 172.8 (*C*OOCH₂Ph).

### Synthesis of 4-chlorophenyl-(methoxy-L-alaninyl)-phosphorochloridate.

**C₁₀H₁₂Cl₂NO₄P, MW-312.09.**

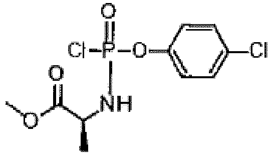

This is synthesised according to ***Standard procedure 4***, using L-alanine methyl ester hydrochloride (1.0 g, 7.16 mmol), 4-chlorophenylphosphorodichloridate (1.757 g, 7.16 mmol), and TEA (1,449 g, 14.32 mmol, 1995 µL) in DCM (30 mL), to yield 1.621 g (72,5%) of crude product used without further purification.
³¹P-NMR (CDCl₃, 121 MHz): δ 9.36, 9.07.
¹H-NMR (CDCl₃; 300 MHz): δ 7.35-7.15 (4H, m, O*Ph*), 4.48-4.36 (1H, bs, N*H*), 4.12-4.04 (1H, m, C*H*CH₃), 3.76-3.74 (3H, 2s, O*H₃*O), 1.49-1.46 (3H, m, CHC*H₃*).
¹³C-NMR (CDCl₃; 75 MHz): δ 21.0 (*C*H₃CH), 50.8, 51.1 (*C*HCH₃), 53.4 (*C*H₃O), 121.9, 122.1, 122-1, 122.4 ('*o*', O*Ph*), 130.6, 130.4, 130.2 ('*m*', O*Ph*), 132.0 ('*p*', OPh), 148.6 ('*ipso*', O*Ph*), 173.5 (*C*OOCH₃).

### Synthesis of 4-chlorophenyl-(ethoxy-L-alaninyl)-phusphorochloridate.

**C₁₁H₁₄Cl₂NO₄P, MW=326.11.**

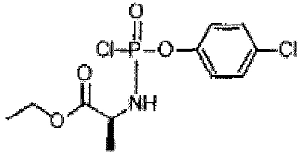

This is synthesized according to ***Standard procedure 4***, using L-alanine ethyl ester hydrochloride (1.000 g, 6.50 mmol), 4-chlorophenylphosphorodichloride (1.595 g, 6.50 mmol), and TEA (1.315 g, 13.00 mmol, 1810 µL) in DCM (20 mL), to yield 1.794 mg (yield 84.7%) of product.
³¹P-NMR (CDCl₃, 121 MHz): δ 9.54, 9.25.
¹H-NMR (CDCl₃; 300 MHz): δ 7.44-7.21 (4H, m, O*Ph*), 4.59 (1H, bs, N*H*), 4.33-4.13 (3H, m, OC*H₂*Ch₃+ C*H*CH₃), 1.57-1.56 (3H, m, C*H₃*CH), 1.43-1.21 (3H, m, OCH₂C*H₃*).
¹³C-NMR (CDCl₃; 75 MHz): δ 14.5, 14.6 (OCH₂*C*H₃), 21.0, 21.5 (*C*H₃CH), 50.9, 51.2 (*C*HCH₃), 62.4, 62.5 (O*C*H₂CH₃), 122.04, 122.3, 122.4 ('*o*', O*Ph*), 130.4 ('*m*', O*Ph*), 131.9 ('*p*', O*Ph*), 148.5, 148.6('*ipso'*, O*Ph*), 173.0, 173.1 (*C*OOCH₂CH₃).

### Synthesis of 4-nitrophenyl-(benzyl-2-amino-2-methylpropanoate)-phosphorochloridate.

**C₁₆H₁₆Cl₂NO₄P, MW=388.18.**

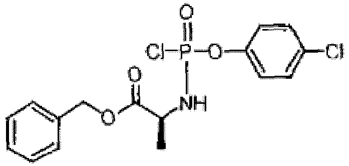

This is synthesized according to ***Standard procedure 4*,** using L-alanine benzyl ester hydrochloride (1.000 g, 4.63 mmol), 4-chlorophenylphosphodichloride (1.136 g, 4.63 mmol), and TEA (937.0 mg, 9.26 mmol, 1290 µL) in DCM (40 mL), to yield 1534 mg (yield 86.5%) of crude product used without further purification.
³¹P-NMR (CDCl₃, 121 MHz): δ 9.43, 9.16.
¹H-NMR (CDCl₃; 300 MHz): δ 7.42-7.08 (9H, m, O*Ph*+ CH₂Ph), 5.19 (2H, s, C*H₂*Ph), 4.61-4.54 (1H, bs, N*H*), 4.26-4.10 (1H, m, C*H*CH₃), 1.42-1.38 (3H, m, C*H₃*CH).
¹³C-NMR (CDCl₃; 75 MHz): δ 20.9, 21.0 (CH₃CH), 51.0, 51.2 (*C*HCH₃), 68.1, 68.2 (O*C*H₂Ph), 122.3, 122.4 ('*o*', O*Ph*), 128.8, 129.1, 130.4 ('*ο*', '*m*', '*p*', CH₂*Ph*+O*Ph*), 131.9 ('*ipso*', CH₂*Ph*), 135.3 ('*p*', O*Ph*), 148.5 ('*ipso*', O*Ph*), 172.7, 172.8 (*C*OOCH₂Ph).

### Synthesis of phenyl-(methyl-2-amino-2-methylpropanoate)-phosphorochloridate.

**C₁₁H₁₅ClNO₄P, MW=291.67.**

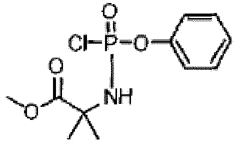

This is synthesised according to ***Standard procedure 4**,* using 2-aminoisobutyrate methyl ester hydrochloride (583.5 mg, 3.75 mmol), phenyl dichlorophosphate (791.1 mg, 3.75, 560 µL), and TEA (758.9 mg, 7.5 mmol, 1045 µL) in DCM (20 mL), to yield 1.041 g (95.2%) of crude product used without further purification.
³¹P-NMR (CDCl₃, 121 MHz): δ 6.99 (s).
¹H-NMR (CDCl₃; 300 MHz): δ 7.41-7.17 (5H, m, O*Ph*), 4.98 (1H, bs, N*H*), 3.80 (3H, s, OC*H₃*), 1.71-1.69 (6H, 2s, [C*H₃*]₂C).
¹³C-NMR (CDCl₃; 75 MHz): δ 27.3, 27.2, 27.0 ([*C*H₃]₂C), 53.6 (O*C*H₃), 58.8 (*C*[CH₃]₂), 120.0, 121.1 ('*o*' O*Ph*), 126.2 ('*p*', O*Ph*), 130.3 ('*m*', O*Ph*) 145.7 ('*p*', O*Ph*), 150.2, 150.3 ('*ipso*', O*Ph*), 175.6, 175.7 (*C*OOCH₃).

### Synthesis of phenyl-(ethyl-2-amino-2-methylpropanoate)-phosphorochloridate.

**C₁₂H₁₇ClNO₄P, MW=305.69.**

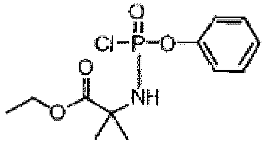

This is synthesised according to ***Standard procedure 4***, using 2-aminoisobutyrate ethyl ester hydrochloride (628.6 mg, 3.75 mmol), phenyl dichlorophosphate (791.1 mg, 3.75, 560 µL), and TEA (758.9 mg, 7.5 mmol, 1045 µL) in DCM (20 mL), to yield 1.018 g (88.8%) of crude product used without further purification,
³¹P-NMR (CDCl₃, 121 MHz): δ 7.02 (s)
¹H-NMR (CDCl₃; 300 MHz): δ 7.23-7.37 (5H, m, O*Ph*), 4.98 (1H, bs, N*H*), 4.24 (2H, q, ³*J*=7.1 Hz, OC*H₂*CH₃), 1.70, 1.68 (6H, 2s, [C*H₃*]₂C), 1.30(3H, t, ³*J*=7,1 Hz, OCH₂C*H₃*).
¹³C-NMR (CDCl₃; 75 MHz): δ 14.5 (*C*H₃CH₂O), 27.3, 26.9 ([*C*H₃]₂C), 58.7 (*C*[CH₃]₂), 62.7 (O*C*H₂CH₃), 121.1, 121.0 ('*o*', OPh), 127.6 ('*p*', OPh), 130.7 ('*m*', OPh), 150.4 ('*ipso*', O*Ph*), 175.2, 175.1 (*C*OOCH₂CH₃).

### Synthesis of phenyl-(benzyl-2-amino-2-methylpropanoate)-phosphorochloridate.

**C₁₇H₁₉ClNO₄P, MW=367.76.**

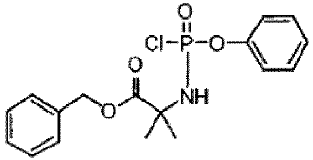

This is synthesised according to ***Standard procedure 4***, using 2-aminoisobutyrate benzyl ester hydrochloride (861.4 mg, 3.75 mmol), phenyl dichlorophosphate (791.1 mg, 3.75, 560 µL), and TEA (758.9 mg, 7.5 mmol, 1045 µL) in DCM (30 mL). The crude was purified by flash chromatography (ethyl acetate/petroleum ether 6:4) affording 580 mg (42.2%) of oil.
³¹P-NMR (CDCl₃, 121 MHz): δ 6.79 (s)
¹H-NMR (CDCl₃; 300 MHz): δ 7.45-7.27 (10H, m, O*Ph*+CH₂*Ph*), 5.28 (2H, s, *CH₂*Ph), 4.81, 4.78 (1H, 2bs, N*H*), 1.78, 1.75 (6H, 2s, [C*H₃*]C).
¹³C-NMR (CDCl₃; 75 MHz): δ 27.3, 26.9 ([*C*H₃]C), 53.9 (*C*[CH₃]₂); 60.9 (*C*H₂Ph), 121.0, 126.3, 129-6, 129.0, 129.1, 130.3, 135.5 (O*Ph*, CH₂*Ph*), 135.5 ('*ipso*', CH₂Ph), 150.3, 150.2 ('*ipso*', OPh), 175.0, 175.2 (*C*OOCH₂Ph).

### Synthesis of 4-nitrophenyl-(methyl-2-amino-2-methylprepanoate)-phosphorochloridate.

**C₁₁H₁₄ClN₂O₆P, MW=336.67.**

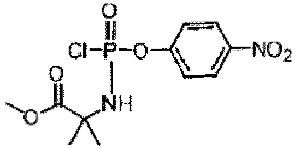

This is synthesised according to ***Standard procedure 4***, using 2-aminoisobutyrate methyl ester hydrochloride (290.0mg, 1.89 mmol), 4-nitrophenylphosphodichloride (483.3 mg, 1.89 mmol), and TEA (382.5 mg, 3.78 mmol, 526,9 µL) in DCM (15 mL), to yield 486 mg (yield 76.4%) of crude product used without further purification.
³¹P-NMR (CDCl₃, 121 MHz): δ 6.61 (s)
¹H-NMR (CDCl₃; 300 MHz): δ 8.25 (2H, d , ³*J*=9.0 Hz, O*Ph*), 7.43 (2H, d, ³*J*=9.0 Hz, O*Ph*), 4.91-4.87 (1H, 2bs, N*H*), 3.79 (3H, s, OC*H₃*), 1.69-1.66 (6H, 2s, [C*H₃*]₂C).
¹³C-NMR (CDCl₃; 75 MHz): δ 27.0, 27.1, 27.3 ([*C*H₃]₂C), 53.8 (O*C*H₃), 59.2 (C[*C*H₃]₂), 121.7, 121.8 ('*o*' O*Ph*), 126.2 ('*m*', O*Ph*), 145.7 ('*p*', O*Ph*), 154.8, 154.7 ('*ipso*', O*Ph*), 175.4, 175.6 (*C*OOCH₃).

### Synthesis of 4-nitrophenyl-(ethyl-2-amino-2-methypropanoate)-phosphorochloridate.

**C₁₂C₁₆ClN₂O₆P, MW=350.69.**

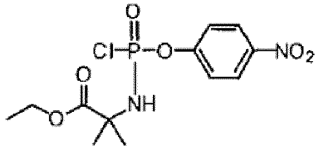

This is synthesised according to ***Standard procedure 4**,* using 2-aminoisobutyrate ethyl ester hydrochloride (270.0 mg, 1.61 mmol), 4-nitrophenylphodichloride (412.3 mg, 1.61 mmol), and TEA (325.8 mg, 3.22 mmol, 448.8 µL) in DCM (15 mL), to yield 500 mg (yield 88.5%) of crude product used without further purification.
³¹P-NMR (CDCl₃, 121 MHz): δ 6.64 (s)
¹H-NMR (CDCl₃; 300 MHz): δ 8.35 (2H, d, ³*J*=9.0 Hz, O*Ph*), 7.53 (2H, d, ³*J*=9.0 Hz, O*Ph*), 4.99-4.96 (1H, 2bs, N*H*), 4.34 (2H, q, ³*J*=7.1 Hz, OC*H₂*CH₃), 1.79-1.76 (6H, 2s, [C*H₃*]₂C), 1.40 (3H, t, ³*J*=7.1 Hz, OCH₂C*H₃*).
¹³C-NMR (CDCl₃; 75 MHz): δ 14.5 (OCH₂*C*H₃), 27.0, 27.3 ([*C*H₃]₂C), 59.1, 59.2 (*C*[CH₃]₂), 62.9, 63.0 (O*C*H₂CH₃), 121.7, 121.8 ('*o*' O*Ph*), 126.2 ('*m*', O*Ph*), 145.7 ('*p*', O*Ph*), 154.7, 154.8 ('*ipso*', O*Ph*), 175.4, 175.6 (*C*OOCH₂CH₃).

### Synthesis of 4-nitrophenyl-(benzyl-2-amino-2-methylpropanoate)-phosphorochloridate.

**C₁₇H₁₈ClN₂O₆P, MW=412.76.**

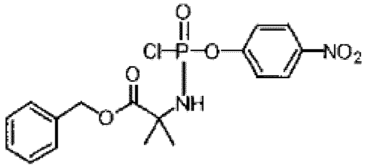

This is synthesised according to ***Standard procedure 4***, using 2-aminoisobutyrate benzyl ester hydrochloride (578 mg, 2.52 mmol), 4-nitrophenylphosphodichloride (645 mg, 2.52 mmol), and TEA (510 mg, 5.04 mmol, 702.5 µL) in DCM (20 mL), to yield 936 mg (yield 90.0%) of crude product used without further purification.
³¹P-NMR (CDCl₃, 121 MHz): δ 6.56 (s)
¹H-NMR (CDCl₃; 300 MHz): δ 8.29 (2H, d, ³*J*=9.0 Hz, O*Ph*), 7.47 (2H, d , ³*J*=9.0 Hz, O*Ph*), 7.40-7.37 (5H, m, CH₂*Ph*), 5.27 (2H, s, C*H₂*Ph), 5.04-5.01 (1H, 2bs, N*H*), 1.77-1.74 (6H, 2s, [C*H₃*]₂C).
¹³C-NMR (CDCl₃; 75 MHz): δ 27.0, 27.3, ([*C*H₃]₂C), 59.2 (*C*[CH₃]₂), 68.5 (O*C*H₂Ph), 121.6, 121.7, 126.2, 128.6, 129.1, ('*o*', '*m*', '*p*', CH₂*Ph*+ OPh), 135.7 ('*ipso*', *C*H₂Ph), 145.7 ('*p*', O*Ph*), 154.7, 154.8 ('*ipso*', O*Ph*), 175.8, 175.9 (*C*OOCH₂Ph).

### Synthesis of 4-chlorophenyl-(methyl-2-amino-2-methylpropanoate)-phosphorochloridate.

**C₁₁H₁₄Cl₂NO₄P, MW=326.11**

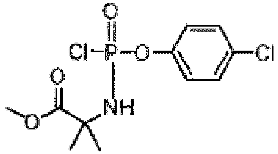

This is synthesized according to ***Standard procedure 4***, using 2-aminoisobutyrate methyl ester hydrochloride (280.0 mg, 1.82 mmol), 4-chlorophenylphosphodichloride (447.4 mg, 1-82 mmol), and TEA (368.3 mg, 3.64 mmol, 507.3 µL) in DCM (20 mL), to yield 554 mg (yield 91.1%) of crude product used without further purification.
³¹P-NMR (CDCl₃, 121 MHz): δ 7.05 (s)
¹H-NMR (CDCl₃; 300 MHz): δ 7.38 (2H, d, ³*J*=9.0 Hz, O*Ph*), 7.28-7.24 (2H, 2d, ³*J*=9.0 Hz, O*Ph*), 4.87-4.83 (1H, 2bs, N*H*), 3.84 (3H, s, OC*H₃*), 1,73.1-71 (6H, 2s, [C*H₃*]₂C).
¹³C-NMR (CDCl₃; 75 MHz): δ 27.0, 27.3, ([*C*H₃]₂C), 53.7 (O*C*H₃), 58.9 (*C*[CH₃]₂), 122.5 ('*o*', O*Ph*), 129.7 ('*m*', O*Ph*), 131.8 ('*p*', O*Ph*) 148.7, 148.9 ('*ipso*', O*Ph*), 175.5, 175.7 (*C*OOCH₃).

### Synthesis of 4-chlorophenyl-(ethyl-2-amino-2-methylpropanoate)-phosphorochloridate.

**C₁₂H₁₆Cl₂NO₄P, MW=340.14.**

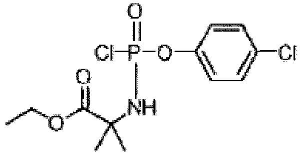

This is synthesised according to ***Standard procedure 4***, using 2-aminoisobutyrate ethyl ester hydrochloride (293.4 mg, 1.75 mmol), 4-chlorophenylphosphodichloride (430.0 mg, 1.75 mmol), and TEA (354.2 mg, 3.50 mmol, 488.0 µL) in DCM (15 mL), to yield 571.7 mg (yield 96.1%) of crude product used without further purification.
³¹P-NMR (CDCl₃, 121 MHz): δ 7.09 (s)
¹H-NMR (CDCl₃; 300 MHz): δ 7.38 (2H, d, ³*J*=9.1 Hz, O*Ph*), 7.26 (2H, d, ³*J*=9.1 Hz, O*Ph*), 4.88-4.84 (1H, 2bs, N*H*), 4.29 (2H, q, ³*J*=7.1 Hz, OC*H₂*CH₃), 1.74-1-70 (6H, 2s, [C*H₃*]C), 1.35 (3H, t, ³*J*=7.1 Hz, OCH₂C*H₃*).
¹³C-NMR (CDCl₃; 75 MHz): δ 14.5 (OCH₂*C*H₃), 27.0, 27.3 ([*C*H₃]C), 58.9 (*C*[CH₃]₂), 62.8 (O*C*H₂CH₃), 122.5 ('*o*', O*Ph*), 130.4 ('*m*', O*Ph*), 131.8 ('*p*', O*Ph*), 148.7, 148.8 ('*ipso*', O*Ph*), 175.1, 175.3 (*C*OOCH₂CH₃).

### Synthesis of 4-chlorophenyl-(benzyl-2-amino-2-methylpropanoate)-phosphorochloridate.

**C₁₇H₁₈Cl₂NO₄P, MW=402.21.**

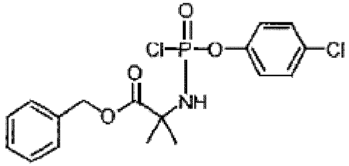

This is synthesised according to ***Standard procedure 4***, using 2-aminoisobutyrate benzyl ester hydrochloride (402.0 mg, 1.75 mmol), 4-chlorophenylphosphodichloride (430 mg, 1.75 mmol), and TEA (354.2 mg, 3.50 mmol, 488,0 µL) in DCM (15 mL), to yield 657.9 mg (yield 93.5%) of crude product used without further purification.
³¹P-NMR (CDCl₃, 121 MHz): δ 7.00 (s)
¹H-NMR (CDCl₃; 300 MHz): δ 7.39-7.12 (9H, m, CH₂*Ph*+ O*Ph*), 5.18 (2H, s, *CH₂*Ph), 4.75-4.72 (1H, 2bs, N*H*), 1.68-1.65 (6H, 2s, [C*H₃*]2C).
¹³C-NMR (CDCl₃; 75 MHz): δ 27.0, 27.3, ([*C*H₃]₂C), 59.0 (*C*[CH₃]₂), 68.4 (O*C*H₂Ph), 122.5, 128.6, 129.1, 130.7 ('*o*', '*m*', '*p*', CH₂*Ph*+ O*Ph*), 131.8 ('*p*', *C*H₂Ph), 135.4 ('*p*', O*Ph*), 148.6, 148.7 ('*ipso*', O*Ph*), 174.9, 175.1 (*C*OOCH₂Ph).

### Synthesis of 4-(trifluoromethyl)phenyl-(benzyl-2-amino-2-methylpropanoate)-phosphorochloridate.

**C₁₈H₁₈ClF₃NO₄P, MW=435.76.**

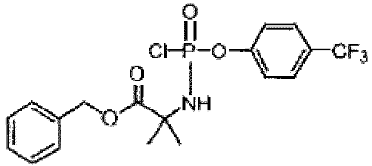

This is synthesised according to ***Standard procedure 4***, using 2-aminoisobutyrate benzyl ester hydrochloride (341.0 mg, 1.49 mmol), 4-(trifluoromethyl)-phenyl-phosphodichloridate (414.3 mg, 1.49 mmol), and TEA (300.5 mg, 2.97 mmol, 413.9 µL) in DCM (15 mL), to yield 623.9 mg (96.4%) of crude product used without further purification.
³¹P-NMR (CDCl₃, 121 MHz): δ 6.74 (s)
¹H-NMR (CDCl₃; 300 MHz): δ 7.66 (2H, d, ³*J*=8.8 Hz, O*Ph*), 7.42-7.30 (7H, m, O*Ph*+*CH*₂*Ph*), 5.25 (2H, s, C*H₂*Ph), 4.95-4.91 (1H, 2bs, N*H*), 1.75-1.72 (6H, 2s, [C*H₃*]₂C).
¹³C-NMR (CDCl₃; 75 MHz): δ 26.9, 27.0, 27.3 ([*C*H₃]₂C), 59.1 (*C*[CH₃]₂), 68.4 (*C*H₂Ph), 121.1, 121.4, 127.7, 128.4, 128.5, 128.6, 128.9 ('*o*', '*m*', '*p*', O*Ph*+CH₂*Ph*), 124.2 (CF₃, *J*=265 Hz), 135.4 ('*ipso*', CH₂*Ph*), 152.6, 152.7 ('*ipso*', O*Ph*), 174.9, 175.0 (*C*OOCH₂Ph).

### Synthesis of Phenyl-(methoxy-α,α-cycloleucinyl)-phosphorochloridate.

**C₁₃H₁₇ClNO₄P, MW=317.70.**

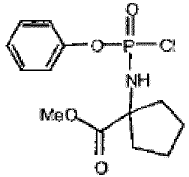

This is synthesised according to ***Standard procedure 4***, using methyl-1-amino-1-cyclopentanoate hydrochloride salt (0.885 g, 5.01 mmol), phenyldichlorophosphate (1.12 g, 0.749 ml, 5.01 mmol), and TEA (1.4 ml, 10 mmol) in DCM (40 mL), to yield 1.266 g (81%) of crude product used without further purification.
³¹P-NMR CCDCl₃, 121 MHz): δ 7.90.
¹H-NMR (CDCl₃; 300 MHz): δ 7.4-7.2 (5H, m, O*Ph*), 4.3 (1H, bs, N*H*), 3.75 (3H, 2s, OC*H₃*), 2.15 (4H, m, 4H cyclopentane), 1.9-1.7 (4H, m, 4H cyclopentane)..
¹³C-NMR (CDCl₃; 75 MHz): δ 24.4 (2CH₂ cyclopent), 38.8, 38.7, 38.6 (2CH₂ cyclopent), 53.3, 53.2 (*C*H₃O), 66.6 (*Cq* cyclopentane), 121.1, 121.0 ('*o*' OPh), 126.3 ('*p*', OPh), 130.3, 130.2 ('*m*', OPh), 150.2 ('*ipso*', OPh), 174.8 (*C*OOCH₃).

### Synthesis of Phenyl-(ethoxy-α,α-cycloleucinyl)-phosphorochloridate.

**C₁₄H₁₉ClNO₄P, MW-331.73.**

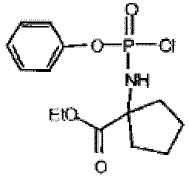

This is synthesised according to ***Standard procedure 4***, using ethyl-1-amino-1-cyclopentanoate hydrochloride salt (955 mg, 5.01 mmol), phenyldichlorophosphate (1.12g, 5.01 mmol, 749 µL), and TEA (1.4 mL, 10.02 mmol) in DCM (40 mL). The crude was purified by flash chromatography (ethyl acetate/petroleum ether 7:3) affording 1.457 g (89%) of oil.
³¹P-NMR (CDCl₃), 121 MHz): δ 8.04, 7.97.
¹H-NMR (CDCl₃; 300 MHz): δ 7.4-7.1 (5H, m, O*Ph*), 4.7 (1H, bs, N*H*), 4.2 (2H, 2q, ³*J*=7.1 Hz, O*CH₂*CH₃), 2.15 (4H, m, 4H cyclopentane), 1.9-1.7 (4H, m, 4H cyclopentane), 1.30 (3H, t, ³*J*=7.1 Hz, OCH₂*CH₃*).
¹³C-NMR (CDCl₃; 75 MHz): δ 14.5 (*C*H₃CH₂), 24.5 (2CH₂ cyclopent), 38.8, 38.7, 38.6, 38.5 (2CH₂ cyclopent), 62.0_CH3*C*H₂), 68.3 (*Cq* cyclopentane), 120.9 ('*o*' OPh), 126.3 ('*p*', OPh), 130.3 ('*m*', OPh), 150.3-150.2 ('*ipso*', OPh), 174.9-174.8 (*C*OOCH₂CH₃).

### Synthesis of Phenyl-(benzoxy-α,α-cycloleucinyl)-phosphorochloridate.

**C₁₉H₂₁ClNO₄P, MW=393.80.**

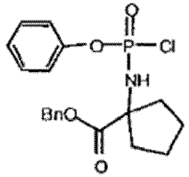

This is synthesised according to ***Standard procedure 4***, using benzyl-1-amino-1-cyclopentanoate hydrochloride salt (0.984 g, 3.84 mmol), phenyl-dichlorophosphate (0.577 ml, 3.84 mmol), and TEA (1.08 mL, 7.69 mmol) in DCM (30 mL), to yield 1.485 g (98%) of crude product used without further purification.
³¹P-NMR (CDCl₃, 121 MHz): δ 7.85.
¹H-NMR (CDCl₃; 300 MHz): δ 7.3-7.0 (10H, m, O*Ph*+*CH₂Ph*), 5.2 (2H, s, *CH₂*Ph), 4.95-4.65 (1H, bs, N*H*), 2.25-2.1 (4H, m, 4H cyclopentane), 1.9-1.7 (4H, m, 4H cyclopentane).
¹³C-NMR (CDCl₃; 75 MHz): δ 24.4, 24,3 (2CH₂ cyclopent), 38.8, 38.7, 38.5 (2CH₂ cyclopent), 67.3 (*Cq* cyclopentane), 68.0 (*C*H₂Ph), 121.0 ('*o*' OPh), 126.4 ('*p*', OPh), 130.1, 129.0, 128.8 ('*m*'OPh, CH₂*Ph*), 135.4 ('*ipso*', CH₂Ph), 150.1 ('*ipso*', OPh), 173.4 ( *C*OOCH₂Ph).

### Synthesis of p-fluorophenyl-(methoxy-α,α-cycloleucinyl)-phosphorochloridate.

**C₁₃H₁₆ClNO₄P, MW=335.70.**

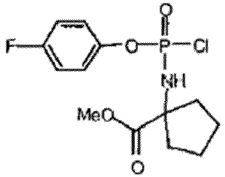

This is synthesised according to ***Standard procedure 4,*** using methyl-1-amino-1-cyclopentanoate hydrochloride salt (0.885 g, 5.01 mmol), para-fluorophenyldichlorophosphate (1.21 g, 5.01 mmol), and TEA (1.4 ml, 10 mmol) in DCM (40 mL), to yield 1.65 g (99%) of crude product used without further purification.
³¹P-NMR (CDCl₃, 121 MHz): δ 8.61.
¹H-NMR (CDCl₃; 300 MHz): δ 7.3-7.2 (2H, m, O*Ph*), 7.1-7.0 (2H, m,O*Ph*), 4.7 (1H, bs, N*H*), 3.78 (3H, 2s, O*CH₃*), 2.25-2.15 (4H, m, 4H cyclopentane), 2,0-1.8 (4H, m, 4H cyclopentane)..
¹³C-NMR (CDCl₃; 75 MHz): δ 24.4 (2CH₂ cyclopent), 38.7, 38.6, 38.5 (2CH₂ cyclopent), 53.3 (*C*H₃O), 66.3-66-2 (*Cq* cyclopentane), 117.1-116.8 ('*o*' OPh), 122.6-122.5 ('*m*', OPh), 146.1-145.9 ('*ipso*', OPh), 159.0 ('*p*', OPh), 175.3-175.2 (*C*OOCH₃).

### Synthesis of p-fluorophenyl-(ethoxy-α,α-cycloleucinyl)-phosphorochloridate.

**C₁₄H₁₈ClFNO₄P, MW=349.72.**

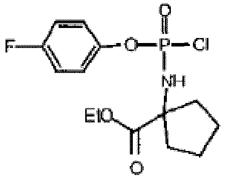

This is synthesised according to ***Standard procedure 4***, using ethyl-1-amino-1-cyclopentanoate hydrochloride salt (955 mg, 5.01 mmol), para-fluorophenyldichlorophosphate (1.21g, 5.01 mmol), and TEA (1.4 mL, 10.02 mmol) in DCM (40 mL), to yield 1.64 g (94%) of crude product used without further purification.
³¹P-NMR (CDCl₃, 121 MHz): δ 8.70.
¹H-NMR (CDCl₃; 300 MHz): δ 7.3-7.2 (2H, m, O*Ph*), 7.1-7.0 (2H, m, O*Ph*), 4.8 (1H, bs, N*H*), 4.2 (2H, 2q, ³*J*=7.1 Hz, O*CH₂*CH₃), 2.25-2.1 (4H, m, 4H cyclopentane), 2.0-1.8 (4H, m, 4H cyclopentane), 1.4 (3H, t, ³*J*=7.1 Hz, OCH₂*CH*₃).
¹³C-NMR (CDCl₃; 75 MHz): δ 14.4 (*C*H₃CH₂), 24.4 (2CH₂ cyclopent), 38.8, 38.7, 38.6, 38.5 (2CH₂ cyclopent), 62.3_CH₃*C*H₂), 68.3 (*Cq* cyclopentane), 117.4, 117.0 ('*o*' OPh), 122.7, 122.6 ('*m*', OPh), 146.1, 146.0 ('*ipso*', OPh), 159.0 ('*p*', OPh), 174.9 (*C*OOCH₂CH₃).

### Synthesis of p-fluorophenyl-(benzoxy-α,α-cycloleucinyl)-phosphorochloridate.

**C₁₉H₂₀ClFNO₄P, MW=4011.79.**

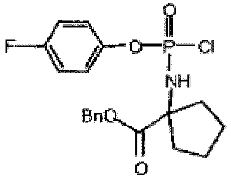

This is synthesized according to ***Standard procedure 4*,** using benzy]-1-amino-1-cyclopentanoate hydrochloride salt (1.281 g, 5.01 mmol), para-fluorophenyl-dichlorophosphate (1.21 g, 5.01 mmol), and TEA (1.4 mL, 10 mmol) in DCM (40 mL), to yield 1.85 g (90%) of crude product used without further purification.
³¹P-NMR (CDCl₃, 121 MHz): δ 7.85.
¹H-NMR (CDCl₃; 300 MHz): δ 7.65-7.4 (5H, m, *CH₂Ph*), 7.3-7.2 (2H, m, O*Ph*), 7,1-7,0 (2H, m, O*Ph*), 5.2 (2H, s, *CH₂*Ph), 4.6 (1H, bs, N*H*), 2.2-2.1 (4H, m, 4H cyclopentane), 2.0-1.8 (4H, m, 4H cyclopentane).
¹³C-NMR (CDCl₃; 75 MHz): δ 24.5 (2CH₂ cyclopent), 38.9, 38.8, 38.6, 38.5 (2CH₂ cyclopent), 68.1 (*Cq* cyclopentane), 68.4 (*C*H₂Ph), 117.0, 116.8 ('*o*' OPh), 122.6, 122.5 ('*m*'OPh) 129.1, 129.0, 128.8, 128.7 (CH₂*Ph*), 135.7 ('*ipso*', CH₂Ph), 146.1, 145.9 ('*ipso*', OPh), 159.0 ('*p*', OPh), 174.6 (*C*OOCH₂Ph).

### Synthesis of p-nitrophenyl-(methoxy-α,α-cycloleucinyl)-phosphorochloridate.

**C₁₃H₁₆ClN₂O₆P, MW-362.70.**

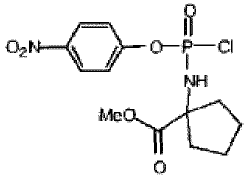

This is synthesised according to ***Standard procedure 4*,** using methyl-1-amino-1-cyclopentanoate hydrochloride salt (0.885 g, 5.01 mmol), para-nitrophenyldichlorophosphate (1.632 g. 5.01 mmol), and TEA (1.4 ml, 10 mmol) in DCM (40 mL), to yield 1.601 g (90%) of crude product used without further purification.
³¹P-NMR (CDCl₃, 121 MHz): δ 8.02.
¹H-NMR (CDCl₃; 300 MHz): δ 8.2 (2H, 2d, ³*J*=8 Hz, O*Ph*), 7.32 (2H, 2d, ³*J*=8 Hz, O*Ph*), 4.9 (1H, bs, N*H*), 3.71 (3H, s, OC*H₃*), 2.25-2.00 (4H, m, 4H cyclopentane), 1.95-1.7 (4H, m, 4H cydopentane)..
¹³C-NMR (CDCl₃; 75 MHz): δ 24.3 (2CH₂ cyclopent), 38.7, 38.6 (2CH₂ cyclopent), 53.3 (*C*H₃O), 68.6 (*Cq* cyclopentane), 121.8, 121.7 ('*o*' OPh), 126.0 ('*m*', OPh), 145.6 ('*ipso*', OPh), 154.8, 154.7 ('*p*', OPh), 175.1-175.0 (*C*OOCH₃).

### Synthesis of p-nitrophenyl-(ethoxy-α,α-cycloleucinyl)-phosphorochloridate,

**C₁₄H₁₈ClN₂O₆P, MW=376.73.**

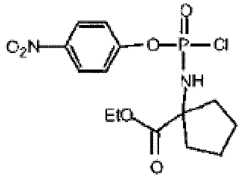

This is synthesised according to ***Standard procedure 4,*** using ethyl-1-amino-1-cyclopentanoate hydrochloride salt **(955** mg, 5.01 mmol), para-nitrophenyldichlorophosphate (1.362 g, 5.01 mmol), and TEA (1.4 mL, 10.02 mmol) in DCM (40 mL), to yield 1.669 g (90%) of crude product used without further purification.
³¹P-NMR (CDCl₃, 121 MHz): δ 7.95.
¹H-NMR (CDCl₃; 300 MHz): δ 8.1 (2H, 2d, ³*J*=8 Hz, O*Ph*), 7.28 (2H, 2d, ³*J*=8 Hz, O*Ph*), 4.8 (1H, bs, N*H*), 4.2 (2H, 2q, ³*J*=7.1 Hz, O*CH₂*CH₃), 2.2-2.0 (4H, m, 4H cyclopentane), 1.95-1.7 (4H, m, 4H cyclopentane), 1.27 (3H, t, ³*J*=7.1 Hz, OCH₂*CH₃*).
¹³C-NMR (CDCl₃; 75 MHz): δ 14.4 (*C*H₃CH₂), 24.4 (2CH₂ cyclopent), 38.8, 38.7 (2CH₂ cyclopent), 62.4_CH₃*C*H₂), 68.5 (*Cq* cyclopentane), 121.8, 121.1 ('*o*' OPh), 126.1, 125.9 ('*m*', OPh), 145.6 ('*ipso*', OPh), 154.8 ('*p*', OPh), 174.9 (*C*OOCH₂CH₃).

### Synthesis of p-nitrophenyl-(benzoxy-α,α-cycloleucinyl)-phosphorochloridate.

**C₁₉H₂₀CIN₂O₆P, MW=438.80.**

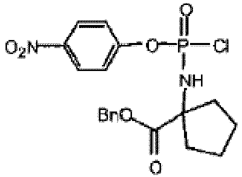

This is synthesised according to ***Standard procedure 4*,** using benzyl-1-amino-1-cyclopentanoate hydrochloride salt (0.835 g, 3.25 mmol), para-nitrophenyl-dichlorophosphate (0.85 g, 3.25 mmol), and TEA (0.91 mL, 6.7 mmol) in DCM (30 mL), to yield 1.215 g (85%) of crude product used without further purification.
³¹P-NMR (CDCl₃, 121 MHz); δ 7.99, 7.90.
¹H-NMR (CDCl₃; 300 MHz): δ 8.1 (2H, 2d, ³*J*=8 Hz, O*Ph*), 7.4-7.2 (7H, m, O*Ph*+ CH₂*Ph*), 5,18 (2H, s, *CH*₂Ph), 5.0 (1H, bs, N*H*), 2.2-2.0 (4H, m, 4H cyclopentane), 1.95-1.75 (4H, m, 4H cyclopentane).
¹³C-NMR (CDCl₃; 75 MHz): δ 24.4 (2CH₂ cyclopent), 38.8, 38.7, 38.6, 38.5 (2CH₂ cyclopent), 68.0 (*C*H₂Ph), 68.6 (*Cq* cyclopentane), 121.8, 121.7 ('*o*' OPh), 126.1, 125.9 ('*m*' OPh) 129.1, 129.0, 128.8, 128.6 (CH₂*Ph*), 135.7 ('*ipso*', CH₂Ph), 145.6 ('*ipso*', OPh), 154.8, 154.7 ('p', OPh), 174.5, 174.4 (*C*OOCH₂Ph),

### Synthesis of p-chlorophenyl-(methoxy-α,α-cycloleucinyl)-phosphorochloridate.

**C₁₃H₁₆Cl₂NO₄P, MW=352.15.**

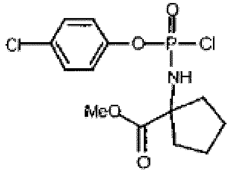

This is synthesised according to ***Standard procedure 4*,** using methyl-1-amino-1-cyclopentanoate hydrochloride salt (0.443 g, 2.5 mmol), para-chlorophenyldiclorophosphate (0.613 g, 2.5 mmol), and TEA (0.7 ml, 5 mmol) in DCM (20 mL), to yield 0.852 g (98%) of crude product used without further purification.
³¹P-NMR (CDCl₃, 121 MHz): δ 9.55,9.5.
¹H-NMR (CDCl₃; 300 MHz): δ 7.35-7.15 (4H, m, O*Ph*), 4.95 (1H, bs, N*H*), 3.78 (3H, s, OC*H*₃), 2.2-2.00 (4H, m, 4H cyclopentane), 1.95-1.7 (4H, m, 4H cyclopentane).
¹³C-NMR (CDCl₃; 75 MHz): δ 24.3 (2CH₂ cyclopent), 38.7 (2CH₂ cyclopent), 53.3 (*C*H₃O), 68.6 (*Cq* cyclopentane). 122.0 ('*o*' OPh), 130.1 ('*m',* OPh), 133.2 ('*p*', OPh), 149.9 (*'ipso'*, OPh), 175.1-175.0 (*C*OOCH₃).

### Synthesis of p-chlorophenyl-(ethoxy-α,α-cycloleucinyl)-phosphorochloridate.

**C₁₄H₁₈Cl₂NO₄P, MW=366.18.**

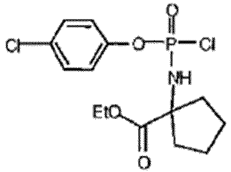

This is synthesised according to ***Standard procedure 4*,** using ethyl-1-amino-1-cyclopentanoate hydrochloride salt (0.477 g, 2.5 mmol), para-chlorophenyldichlorophosphate (0.613 g, 2.5 mmol), and TEA (0.7 mL, 5 mmol) in DCM (20 mL), to yield 0.880 g (97%) of crude product used without further purification.
³¹P-NMR (CDCl₃, 121 MHz): δ 9.85,9.70.
¹H-NMR (CDCl₃; 300 MHz): δ 7.35-7.15 (4H, m, O*Ph*), 4.9 (1H, bs, N*H*), 4.22 (2H, 2q, ³*J*=7.1 Hz, O*CH₂*CH₃), 2.2-2.0 (4H, m, 4H cyclopentane), 1.95-1.7 (4H, m, 4H cyclopentane), 1.27 (3H, t, ³*J*=7 Hz, OCH₂*CH₃*).
¹³C-NMR (CDCl₃; 75 MHz): δ 14.4 (*C*H₃CH₂), 24.4 (2CH₂ cyclopent), 38.8, 38.7 (2CH₂ cyclopent), 62.5, 62.4 CH₃*C*H₂), (68.) (*Cq* cyclopentane), 122.2, 122.1 ('*o*' OPh), 130.1 ('*m*', OPh), 133.2 ('*p*', OPh), 149.8 (*'ipso',* OPh), 174.8 (*C*OOCH₂CH₃).

### Synthesis of p-chlorophenyl-(benzoxy-α,α-cycloleucinyl)-phosphorochloridate.

**C₁₉H₂₀Cl₂NO₄P, MW=428.25.**

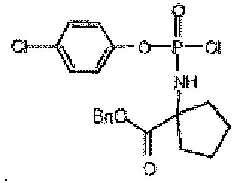

This is synthesised according to ***Standard procedure 4*,** using benzyl-1-amino-1-cyclopentanoate hydrochloride salt (0.640 g, 2.5 mmol), para-chlorophenyldichlorophosphate (0.613 g, 2.5 mmol), and TEA (0.7 mL, 5 mmol) in DCM (20 mL), to yield 1.041 g (97%) of crude product used without further purification.
³¹P-NMR (COCl₃, 121 MHz): δ 9.39, 8.95.
¹H-NMR (CDCl₃; 300 MHz): δ 7.4-7.15 (9H, m, O*Ph* + CH₂*Ph*), 5.20 (2H, s, *CH₂*Ph), 5.0 (1H, bs, N*H*), 2.2-2.0 (4H, m, 4H cyclopentane), 1.95 -1.75 (4H, m, 4H cyclopentane).
¹³C-NMR (CDCl₃; 75 MHz): δ 24.4 (2CH₂ cyclopent), 38.8, 38.7, 38.6 (2CH₂ cyclopent), 68.1, 68.0 (*C*H₂Ph), 68.2 (*Cq* cyclopentane), 121.9, 121.8 ('*o*' OPh), 130.5, 130.4, 129.3, 129,2 ('*m*'OPh, CH₂*Ph*), 133.2 ('*p*', OPh), 135.7 (*'ipso',* CH₂Ph), 149.9 ('*ipso*', OPh), 174.3, 174.2 (*C*OOCH₂Ph).

### Synthesis of p-trifluorophenyl-(methoxy-α,α-cycloleucinyl)-phosphorochloridate.

**C₁₄H₁₆CIF₃NO₄P, MW=385.70.**

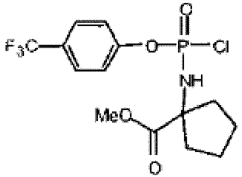

This is synthesised according to ***Standard procedure 4*,** using methyl-1-amino-1-cyclopentanoate hydrochloride salt (0.443 g, 2.5 mmol), paratrifluorophenyldichlorophosphate (0.700 g, 2.5 mmol), and TEA (0.7 ml, 5 mmol) in DCM (20 mL), to yield 0.931 g (97%) of crude product used without further purification.
³¹P-NMR (CDCl₃, 121 MHz): δ 8.80, 8.62.
¹H-NMR (CDCl₃; 300 MHz): δ 7.65 (2H. 2d, ³*J*=8 Hz, O*Ph*), 7.35 (2H, 2d, ³*J*=8 Hz *OPh*), 5.02 (1H, bs, N*H*), 3.78 (3H, s, OC*H₃*), 2.25-2.05 (4H, m, 4H cyclopentane), 1.95-1,7 (4H, m, 4H cyclopentane).
¹³C-NMR (CDCl₃; 75 MHz): δ 22.8 (2CH₂ cyclopent), 37.5, 37.2 (2CH₂ cyclopent), 51.5 (*C*H₃O), 68.4 (*Cq* cyclopentane), 120,0 ('*o*', O*Ph*), 124.8 (d, J=270Hz, CF₃), 126.6 ('*m*', O*Ph*), 129.5 ('*p*',q, J=32Hz, O*Ph*), 152.8 ('*ipso',* OPh), 175.2 (*C*OOCH₃).

### Synthesis of p-trifluorophenyl-(ethoxy-α,α-cycloleucinyl)-phosphorochloridate.

**C₁₅H₁₈CIF₃NO₄P, MW=399.73.**

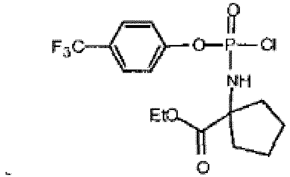

This is synthesised according to ***Standard procedure 4*,** using ethyl-1-amino-1-cyclopentanoate hydrochloride salt (0.477 g, 2.5 mmol), para-trifluorophenyldichlorophosphate (0.700 g, 2.5 mmol), and TEA (0.7 mL, 5 mmol) in DCM (20 mL), to yield 0.950 g (89%) of crude product used without further purification.
³¹P-NMR (CDCl₃, 121 MHz): δ 8.49.
¹H-NMR (CDCl₃; 300 MHz): δ 7.45 (2H, m, O*Ph*), 7.2 (2H, m, O*Ph*), 5.12 (1H, bs, N*H*), 4.05 (2H, m, O*CH*₂CH₃), 2.15-2.0 (4H, m, 4H cyclopentane), 1.9-1.65 (4H, m, 4H cyclopentane), 1.2 (3H, 2t, ³*J*=7 Hz, OCH₂*CH₃*).
¹³C-NMR (CDCl₃; 75 MHz): δ 14.3 (*C*H₃CH₂), 24.2, 24.1 (2CH₂ cyclopent), 38.6, 38.5, 38.4 (2CH₂ cyclopent), 62.0 CH₃*C*H₂), 68,4 (*Cq*_cyclopentane), 121.5 ('o', O*Ph*), 125.0 (d, J=270Hz, CF₃), 127.5 ('*m*', O*Ph*), 129.9 (*'p'*,q, J=32Hz, O*Ph*), 152.8, 152.7 (*'ipso',* OPh), 174.9, 174.6 (*C*OOCH₂CH₃).

### Synthesis of p-trifluorophenyl-(benzoxy-α,α-cycloleucinyl)-phosphorochloridate.

**C₂₀H₂₀CIF₃NO₄P, MW= 461.80.**

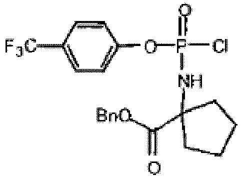

This is synthesized according to ***Standard procedure 4*,** using henzyl-1-amino-1-cyclopentanoate hydrochloride salt (0.700 g, 2.73 mmol), para-trifluorophenyldichlorophosphate (0.75 g, 2.73 mmol), and TEA (0.75 mL, 5.47 mmol) in DCM (25 mL), to yield 1.089 g (86%) of crude product used without further purification.
¹⁴P-NMR (CDCl₃, 121 MHz): δ 9.39, 8.95.
¹H-NMR (CDCl₃; 300 MHz): δ 7.50 (2H, m, O*Ph*, 7.4-7.15 (7H, m, O*Ph*+CH₂*Ph*), 5.20 (2H, s, *CH₂*Ph), 4.95 (1H, bs, N*H*), 2.2-2.0 (4H, m. 4H cyclopentane), 1.95-1.75 (4H, m, 4H cyclopentane),
¹³C-NMR (CDCl₃; 75 MHz): δ 24.3 (2CH₂ cyclopent), 38.8, 38.7, 38.6 (2CH₂ cyclopent), 68.1, 68.0 (*C*H₂Ph), 68.2 (*Cq* cyclopentane), 121.4, 121.3 ('ο', *OPh*), 125.1 (d, J=270Hz, CF₃), 126.6 ('*m*' O*Ph*) 129.2, 128.8, 127.8 (Bn), 129.8 ('*p*',q , J=32Hz, O*Ph*), 135.7 (*'ipso*', CH₂Ph), 151.5 ('*ipso'*, OPh), 174.5, 174.4 (*C*OOCH₂Ph).

### Synthesis of Phenyl-(methoxy-L-phenylalaninyl)-phosphorochloridate.

**C₁₆H₁₇ClNO₄P, MW=353.14.**

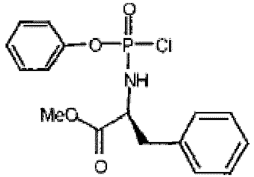

This is synthesised according to ***Standard procedure 4,*** using L-phenylalamine methyl ester hydrochloride (1.08 g, 5 mmol), phenyldichlorophosphate (1.12 g, 0.75 ml, 5 mmol), and TEA (1.4ml, 10 mmol) in DCM (40 mL), to yield 1.626 g (92%) of crude product used without further purification.
³¹P-NMR (CDCl₃, 121 MHz): δ 9.1, 8.95.
¹H-NMR (CDCl₃; 300 MHz): δ 7.3-7.1 (10H, m, CH₂Ph+ O*Ph*), 5.00 (1H, bs, N*H*), 4.35 (1H, m, CHphenylala), 3.79 (3H, 2s, CH₃O), 3.00 (2H, m, CH₂Ph)
¹³C-NMR (CDCl₃; 75 MHz): δ 36.3 (CH₂phenylalanine), 53.0 (*C*H₃O), 56.6, 56.5 (CHpkenylala), 121.0 ('*o*' OPh), 126.4 ('*p*' OPh), 130.2 ('*m*', OPh), 150.2 ('*ipso*', OPh), 174.1 (*CO*OCH₃),

### Synthesis of Phenyl-(methoxy-L-leucinyl)-phosphorochloridate

**C₁₃H₁₉CINO₄P, MW=319.72.**

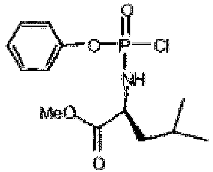

This is synthesised according to ***Standard procedure 4*,** using L-leucine methyl ester hydrochloride (0.91 g, 5 mmol), phenyldichlorophosphate (1.12 g, 0.75 ml, 5 mmol), and TEA (1.4 ml, 10 mmol) in DCM (40 mL), to yield 1.58 g (99%) of crude product used without further purification.
³¹P-MMR (CDCl₃, 121 MHz): δ 9.45. 9.35.
¹H-NMR (CDCl₃; 300 MHz): δ 7.4-7.2 (5H, m, O*Ph*), 4.90 (1H, bs, N*H*), 3.95 (1H, m, C*H*CH₂CH(CH₃)₂), 3.78 (3H, s, OC*H₃*), 1.8 (1H, m, CHCH₂C*H*(CH₃)₂), 1.8-1.5 (2H, m, CHC*H*₂CH(CH₃)₂), 1.0-0.9 (6H. m, CHCH₂CH(*CH*₃)₂).
¹³C-NMR (CDCl₃; 75 MHz): δ 23.2, 23-1, 22.4, 22.3 (2C, CHCH₂CH(*CH₃*)₂), 24.9, 24.8 (CHCH₂*CH*(*CH₃*)₂), 43.6 (CH*CH₂*CH(CH₃)₂), 53.2 (*C*H₃O), 53.7, 53.6 (*CH*CH₂*CH*(CH₃)₂), 120.9 ('*o*' OPh), 126.4 ('*p',* OPh), 130.2 ('*m*', OPh), 150.1 ('*ipso*', OPh), 173.6 (*C*OOCH₃).

### Synthesis of Phenyl-(benzoxy-L-leucinyl)-phosphorochloridate

**C₁₉H₂₃CINO₄P, MW=395.82.**

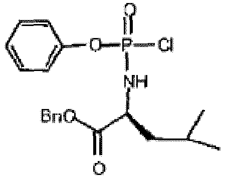

This is synthesised according to ***Standard procedure 4*,** using L-leucine benzyl ester hydrochloride (1.29 g, 5.0 mmol), phenyl-dichlorophosphate (1.12 g, 0.75 ml, 5.0 mmol), and TEA (1.4 mL, 10.0 mmol) in DCM (40 mL), to yield 1.88 g (95%) of crude product used without further purification.
³¹P-NMR (CDCl₃, 121 MHz): δ 9.93, 9.57.
¹H-NMR (CDCl₃; 300 MHz): δ 7.5-7.2 (10H, m, O*Ph*+*CH₂Ph*), 5.2 (2H, 2s, *CH*₂Ph), 4.95 (1H, bs, N*H*), 4.2-4.1 (1H, m, C*H*CH₂CH(CH₃)₂), 1.95.1.80 (1H, m, CHCH₂C*H*(CH₃)₂), 1.7 (2H, m, CHC*H₂*CH(CH₃)₂), 1.0-0.9 (6H, m, CHCH₂CH(*CH₃*)*₂*).
¹³C-NMR (CDCl₃; 75 MHz): δ 23.2, 23.1, 22.4, 22.3 (2C, CHCH₂CH(*CH₃*)₂), 24.9 (CHCH₂*CH*(*CH₃*)*₂*), 43.5 (CH*CH₂*CH(CH₃)₂), 53.8, 53.3 (*CH*CH₂*CH*(CH₃)₂), 67.8, 67.7 (*C*H₂Ph), 120.7 ('*o*' OPh), 126.4 ('*p*', OPh), 130.2, 129,1, 128.8, 128.7 ('*m'*OPh, CH₂*Ph*), 135.8 ('*ipso',* CH₂Ph), 150.2 (*'ipso',* OPh), 174.1 (*C*OOCH₂Ph).

### Synthesis of p-nitrophenyl-(benzoxy-L-leucinyl)-phosphorochloridate.

**C₁₉H₂₂CIN₂O₆P, MW=440.81.**

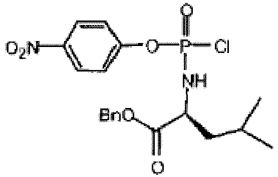

This is synthesized according to ***Standard procedure 4*,** using L-leucine benzyl ester hydrochloride (1.08 g, 5.01 mmol), para-nitrophenyl-dicloro phosphate (1.362 g, 5.01 mmol), and TEA (1.4 mL, 1.4 mmol) in DCM (40 mL), to yield 2.08g (95%) of crude product used without further purification.
³¹P-NMR (CDl₃, 121 MHz): δ 9.87, 9.38.
¹H-NMR (CDCl₃; 300 MHz): δ 8-25-8.10 (2H, m , O*Ph*), 7.35-7.25 (7H, m, O*Ph* + *CH₂Ph*), 5.15 (2H, 2s, *CH*₂Ph), 4.95 (1H, bs, N*H*), 4.15 (1H, m, C*H*CH₂CH(CH₃)₂), 1.95 (1H, m, CHCH₂C*H*(CH₃)₂), 1.7 (2H, m, CHC*H₂*CH(CH₃)₂), 1.0-0.9 (6H, m, CHCH₂CH(*CH₃*)₂).
¹³C-NMR (CDCl₃; 75 MHz): δ 23.2, 23.1, 22.1, 22.0 (2C, CHCH₂CH(*CH₃)₂*), 24.8 (CHCH₂*CH*(*CH₃)₂*)*,* 43.4, 43.3 (CH*CH₂*CH(CH₃)₂), 54.2, 53.9 (*CH*CH₂C*H*(CH₃)₂), 68.0, 67.9 (*C*H₂Ph), 121.6 ('*o*' OPh), 126.2, 126.1 ('*m*' OPh), 129.2, 129.0 (CH₂*Ph*), 135.4, 135.3 ('*ipso*', CH₂Ph), 145.8, 145.7 ('*ipso*', OPh), 154.7, 154.5 ('*p*', OPh), 173.0,172.8( *C*OOCH₂Ph).

### Synthesis of p-chlorophenyl-(benzoxy-L-leucinyl)-phosphorochloridate.

**C₁₉H₂₂Cl₂NO₄P, MW=430.26.**

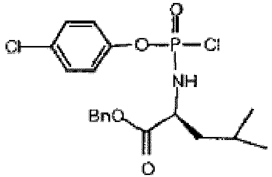

This is synthesised according to ***Standard procedure 4***, using L-leucine benzyl ester hydrochloride (0.644 g, 2.5 mmol), para-chlorophenyl-dichlorophosphate (0.613 g, 2.5 mmol), and TEA (0.7 mL, 5 mmol) in DCM (20 mL), to yield 0.968 g (90%) of crude product used without further purification.
³¹P-NMR (CDCl₃, 121 MHz): δ 9.71, 9.55.
¹H-MMR (CDCl₃; 300 MHz): δ 7.4-7.0 (9H, m, O*Ph* + CH₂*Ph*), 5.15 (2H, s, *CH₂*Ph), 4.5 (1H, d, ³*J*=Hz, N*H*), 4.0 (1H, m, C*H*CH₂CH(CH₃)₂), 1.9-1.8 (1H, m, CHCH₂C*H*(CH₃)₂), 1.7 (2H, m, CHC*H₂*CH(CH₃)₂), 0.85 (6H, m, CHCH₂CH(*CH₃)₂*).
¹³C-NMR (CDCl₃; 75 MHz): δ 23.4, 23.3, 22.5, 22.4 (2C, CHCH₂CH*(CH₃)₂*), 25,0 (CHCH₂*CH*(CH*₃)₂*), 45.8, 43.7 (CH*CH₂*CH(CH₃)₂), 54.0, 53.8 (CHCH₂*CH*(CH₃)₂), 68.2 (*C*H₂Ph), 122.5 ('*o*' OPh), 130.5, 130.4, 129.3, 129.2 ('*m*'OPh, CH₂*Ph*), 133.2 ('*p*', OPh), 135.7 ('*ipso*', CH₂Ph), 149.9, 149.8 ('*ipso*', OPh), 173.4, 173.2 (*C*OOCH₂Ph).

### Synthesis of 4-chlorophenyl-(methyl-2-amine-2-methylpropanoate)-phosphorochloridate.

**C₁₁H₁₄Cl₂NO₄P, MW=326.11.**

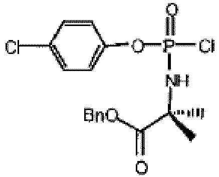

This is synthesised according to ***Standard procedure 4***, using 2-aminoisobutyrate methyl ester hydrochloride (280.0mg, 1.82 mmol), 4-chlorophenylphosphodichloride (447.4 mg, 1.82 mmol), and TEA (368.3 mg, 3.64 mmol, 507.3 µL) in DCM (20 mL), to yield 554 mg (yield 91.1%) of crude product used without further purification.
³¹P-NMR (CDCl₃, 121 MHz): δ 7.05 (s)
¹H-MMR (CDCl₃; 300 MHz): δ 7.38 (2H, d, ³*J*=9.0 Hz, *OPh*), 7.29-7.24 (2H, 2d, ³*J*=9.0 Hz, O*Ph*), 4.87-4.83 (1H, 2bs, N*H*), 3.84 (3H, s, OC*H*₃), 1.73-1.71 (6H, 2s, [C*H₃*]₂C).
¹³C-NMR (CDCl₃; 75 MHz): δ 27.0, 27.3, ([*C*H₃]₂C), 53.7 (O*C*H₃), 58.9 (*C*[CH₃]₂), 122.5 ('*o*', O*Ph*), 129.7 ('*m*', O*Ph*), 131.8 ('*p*', *OPh*) 148.7, 148.9 ('*iρsο*', O*Ph*), 175.5, 175.7 (*C*OOCH₃).

### Synthesis of 4-chlorophenyl-phosphodichloridate.

**C₆H₄Cl₃O₂P, MW=245.43.**

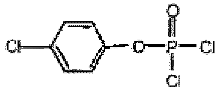

This was synthesised according to ***Standard procedure 3***, using phosphorus-oxychloride (1533 mg, 10.00 mmol, 932 µL), 4-chlorophenol (1.285 g, 10.00 mmol) and TEA (1,011 g, 10.00 mmol, 1394 µL) in ethylether (100 mL) to give an oil (1.897 g, 77.3 % yield).
³¹P-NMR (CDCl₃, 121 MHz): δ 5.18.
¹H-NMR (CDCl₃; 300 MHz): δ 7.45 (2H, d, ³*J*=9.0 Hz, O*Ph*), 7.30 (2H, d, ³*J*=9.0 Hz, O*Ph*).
¹³C-NMR (CDCl₃; 75 MHz): δ 122.5 ('*o*', O*Ph*), 130.6 ('*m*', OPh), 133.2 ('*p*', O*Ph*), 148.5 ('*ipso*', O*Ph*).

### Synthesis of 4-(trifluoromethyl)-phenyl-phosphodichloridate.

**C₇H₄ClF₃O₃P, MW=278.99.**

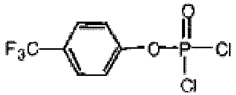

This was synthesised according to ***Standard procedure 3***, using phosphorus-oxychloride (1.570 mg, 10.24 mmol, 954.5 µL), 4-trifluoromethylphenol (I660 g, 10.24 mmol) and TEA (1.036 g, 10.24 mmol, 1427 µL in ethylether (100 mL) to give an oil (2.521 g, 88.2% yield).
³¹P-NMR (CDCl₃, 121 MHz): δ 4.75.
¹H-NMR (CDCl₃; 300 MHz): δ 7.77 (2H, d, ³*J*=8.4 Hz, O*Ph*), 7.49 (2H, d, ³*J*=8.4 Hz, O*Ph*).
¹³C-NMR (CDCl₃; 75 MHz): δ 121.6 ('*ο*', O*Ph*), 123.6 (*C*F₃, *J*=271 Hz, O*Ph*), 128.2 ('*m*', OPh), 129.7 ('*p*', *J*=33 Hz), 152.7 ('*ipso*', O*Ph*).

### Synthesis of 4-fluorophenyl-phosphodichloridate.

**C₆H₄Cl₂FO₂P, MW=228.97.**

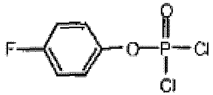

This was synthesised according to ***Standard procedure 3***, using phosphorus-oxychloride (1.395 mL, 15.00 mmol), 4-chlorophenol (1.68 g, 15.00 mmol) and TEA (2.1 mL, 15.00 mmol) in ethylether (140 mL) to give an oil (3.96 g, 96 % yield).
³¹P-NMR (CDCl₃, 121 MHz); δ 5.52.
¹H-NMR (CDCl₃; 300 MHz): δ 7.15 (2H, d, ³*J*=8.0 Hz, O*Ph*), 7.05 (2H, d, ³*J*=8.0 Hz, O*Ph*).
¹³C-NMR. (CDCl₃; 75 MHz): δ 116.8 ('*ο*', O*Ph*), 122.1 ('*m*', OPh), 146.7 ('*p*', O*Ph*), 158.7 ('*ipso*', O*Ph*).

Experimental data are given in Table I illustrating the activity of compounds embodying the present invention, and of some comparaive compounds, with respect to human breast cancer cell line MDA MB231, human colon cancer cell line HT115 and human prostrate cancer cell line PC-3. The compounds include those whose preparations are described above and compounds made by preparative methods corresponding to the methods described above.

The experimental procedures used human colon cancer cell line (HT115), human prostate cancer cell line (PC-3), human breast cancer cell line (MDA MB 231) and normal human umbilical vein endothelial cell (HUVEC). Compounds were diluted over a range of concentrations and added to cells over 1 to 3 days. The cytotoxity was determined using a MTT assay at the end of each experiment.

In the Table:
ArO refers to Ar as defined above with respect to formula I:
J refers to the moiety of the present compounds represented by, respectively, ROCOCR'R"NH-, as defined above with respect to formula I, or, with respect to Examples 51, 52 and 53, HOCOCR'R"NH-, as defined above with respect to formula II; and
B refers to the base moiety of the compounds:
   BVU stands for 2-bromovinyl uridine.
   5-(C=CC[O]O)MeU stands for methyl propenoate-2'-deoxyuridine.
   GemCyt stands for Gemcitabine.
Examples A, 1, 67 and G are further comparative examples.
Example A is 5-(2-Bromovinyl)-2'-deoxyuridine.
Example 1 is Example 1 above corresponding to compound (7) above.
Example 67 is propenate-2'-deoxyuridine.
Example G is gemcitabine.

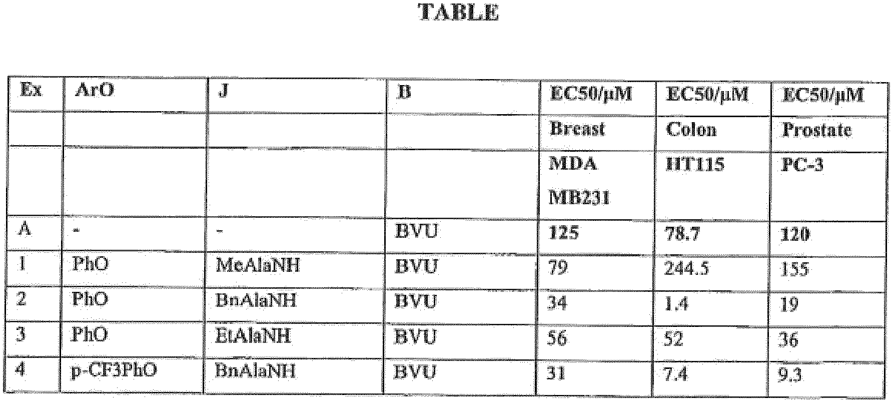

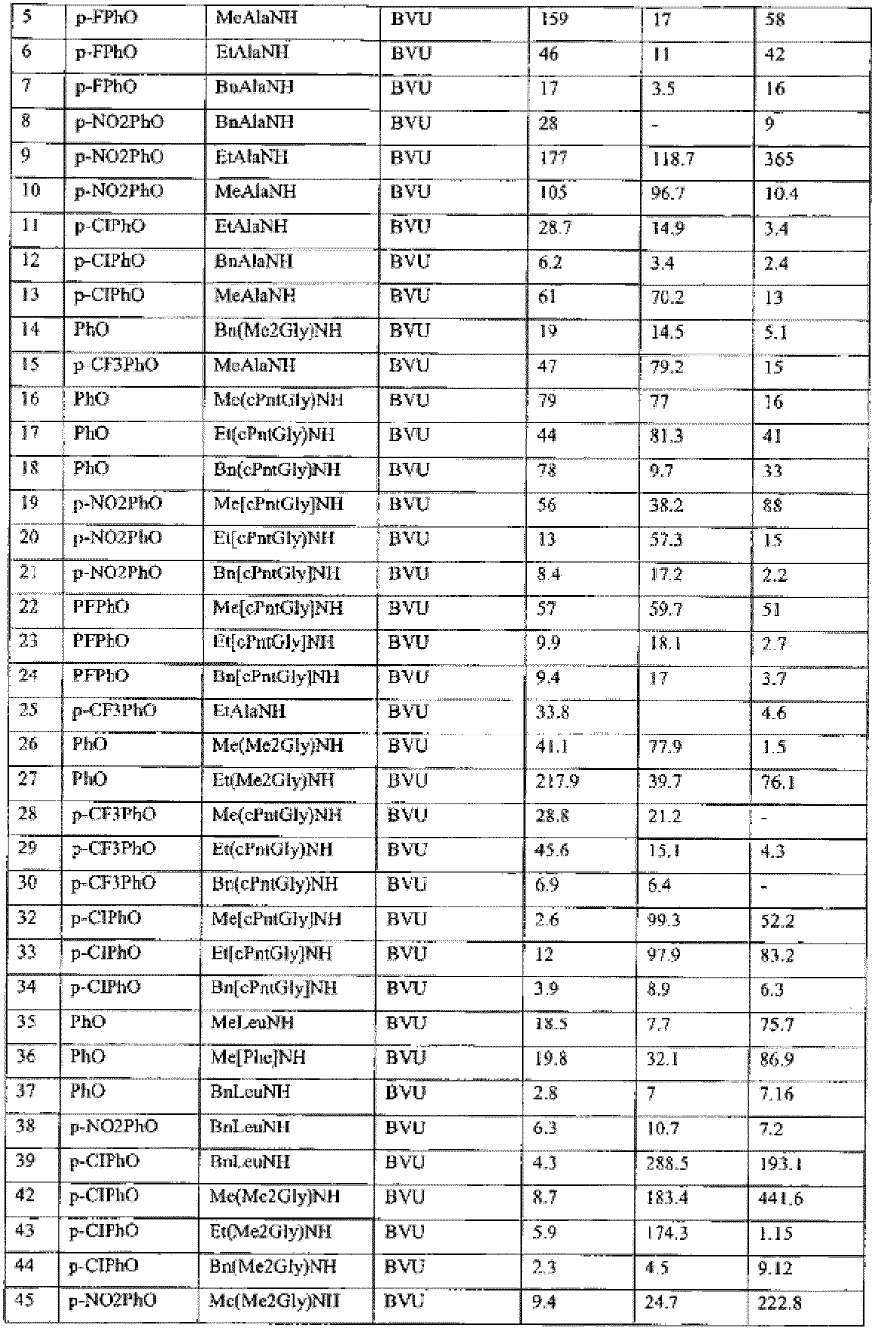

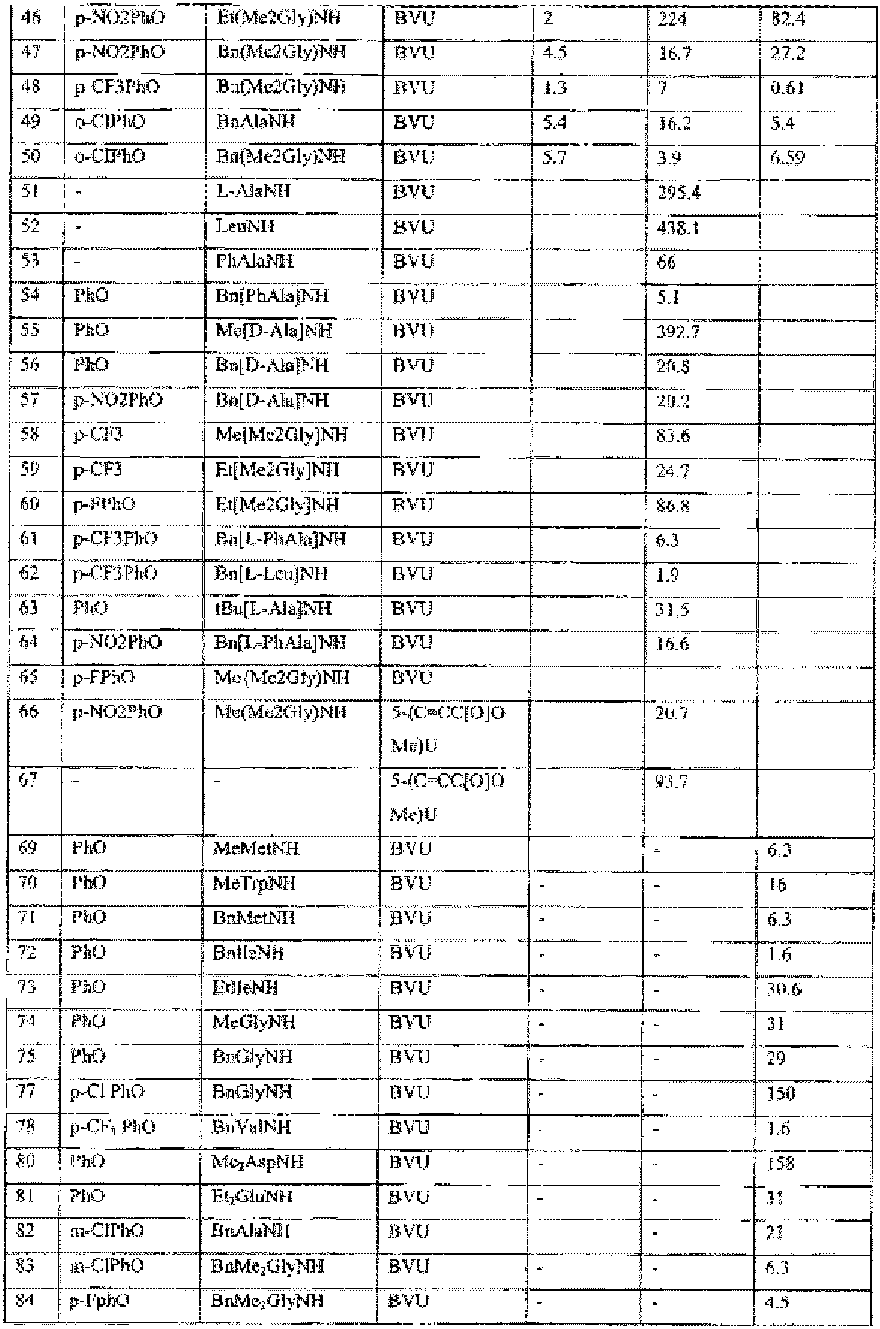

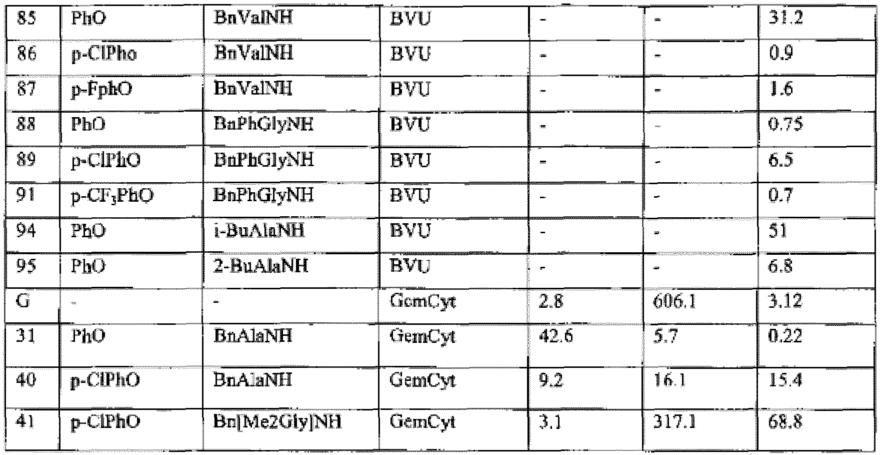

Gemcitabine (Example G in the Table) and compound CPF31 (Example 31 in the Table: gemcitabine-[phenyl-(benzoxy-L-alaninyl)]-phosphate) were compared in a mouse model with xenografts of human cancer (colon HT115 and prostrate PC3).

Mice were dosed daily at a range of concentrations (0.01-10µM) and tumour volume assessed versus control.

Kaplan-Meier statistics were computed regarding incident-free survival.

In the attached drawings:
Figure 1 shows for the mouse xenograft the tumour volume for prostate data at day 13 using Gemzar™ (gemcitabine available ex. Lilly);
Figure 2 shows for the mouse xenograft the tumour volume for prostate data at day 13 using CPF31;
Figure 3 shows the incident free survival functions v. day for each of CPF31 and gemcitabine; and
Figure 4 shows for the mouse xenograft the tumour volume for colon data at day 24 using, respectively, Gemzar and compound CPF31.

Referring to the drawings, CPF31 can be seen to be significantly less toxic than gemcitabine.

CPF31 was significantly effective at reducing prostate and colon tumour volume relative to control at daily dosing of 5 and 10 µM (3 and 6 µg/ml). Gemcitabine was not effective at the highest non-toxic concentration.

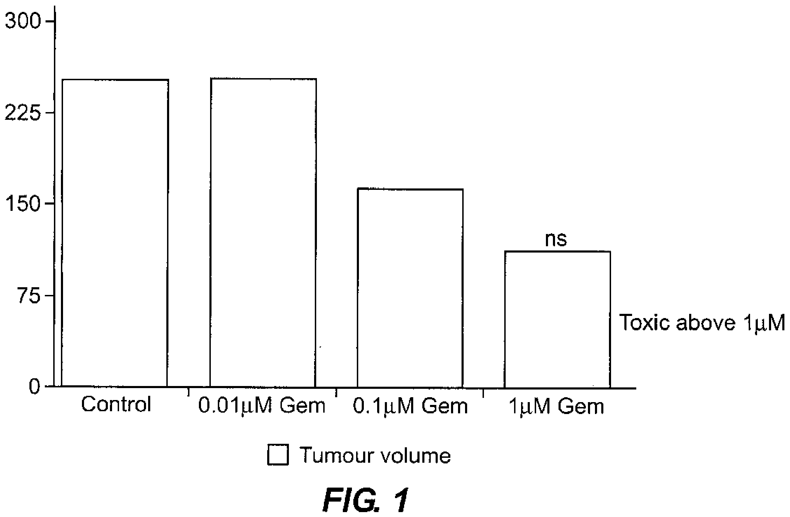

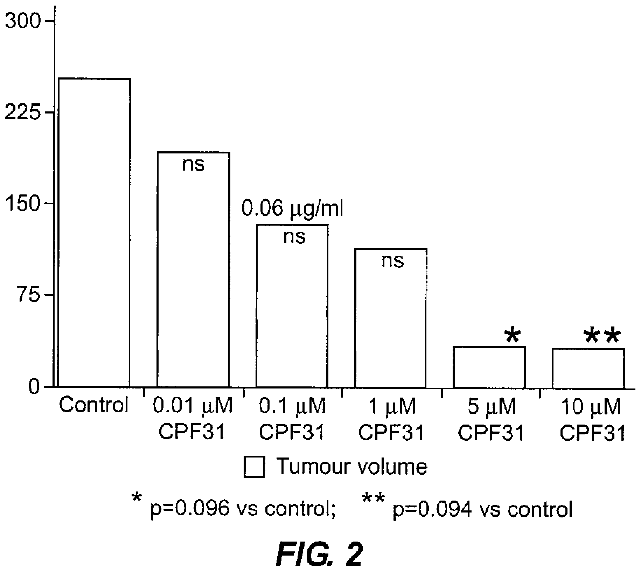

Gemzar is seen from Figure 1 to be toxic above 1µM. In contrast, CPF31 is seen from Figure 2 to have substantially lower toxicity.

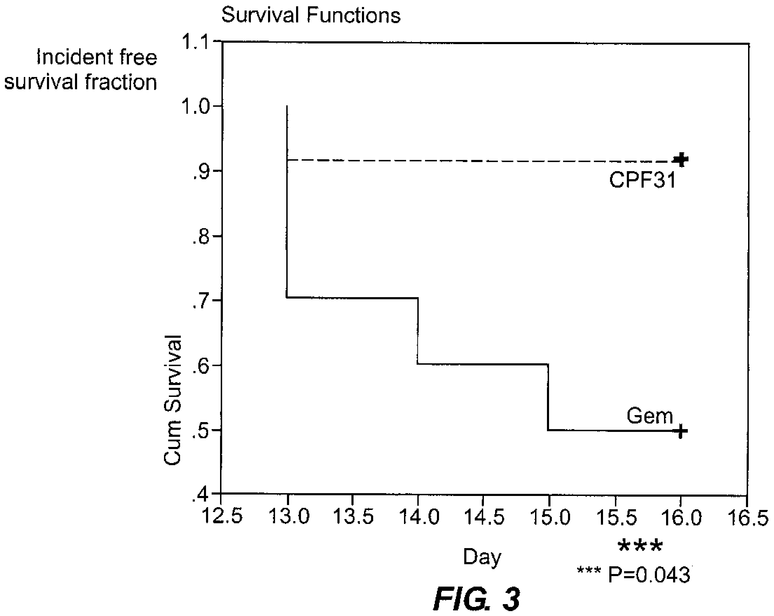

Figure 3 shows that CPF31 has significantly lower side effects on a comparable basis: 3 animals show serious toxicity (10% body mass loss) in GMZ and in CPF31 on day 10, collectively 4 in GMZ and 1 in CPF31 on day 11 and 5 in GMZ and 1 in CPF on day 13. Using Chi square analysis by combining 5 and 10µM groups, the significance is p=0.193, 0,078 and 0.0289 on day 10, 11 and 13. It is clear that by day 13, CPF31 displayed signficantly less side effects, and the anti-cancer effects continue to exceed that of Gemzar,

Figure 3 shows the Kaplan-Meier survival curve, incidence free survival: based on the loss according to weight loss. A Cox proportion analysis shows that CPF31 is far less toxic than GMZ based on the weight-loss calculated loss (p-0.043).

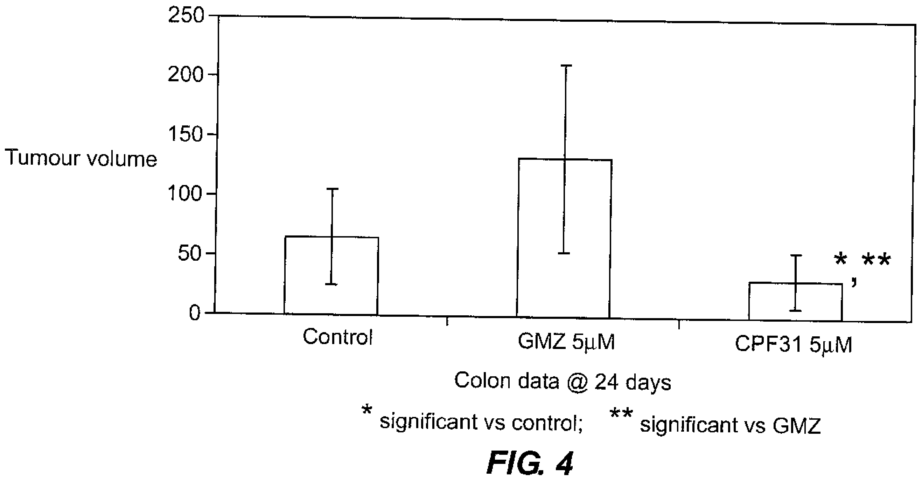

CPF31 was found to be active at 5µM *in vitro*, whereas Gemzar was found to be active at 600µM, with respect to the same colon cell line. Figure 4 shows the results of testing both *in vivo* at 5µM. The greater activity of CPF31 in reducing tumour volume is shown in Figure 4.

## Claims

1. A chemical compound having formula I: 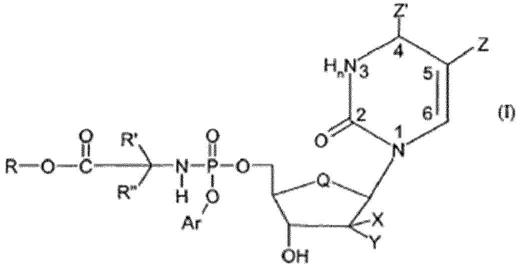 wherein:
R is selected from a C₁-C₁₆ branched or unbranched acyclic alkyl group, a C₃-C₁₂ cyclic alkyl group, a phenyl group or a naphthyl group and a benzyl group; and wherein R is unsubstituted;
R' and R" are independently selected from H, methyl (-CH₃), benzyl (-CH₂C₆H₅) and secondary butyl (-CH₂CH(CH₃)₂, or, R' and R" together with the C atom to which they are attached, provide a C₅-₆ ring;
Q is -O-;
X and Y are both F;
Ar is naphthyl or phenyl; and one, two, three or four substituents, which may be the same or different, may be present on Ar and are selected from the group comprising halogen,-NO₂; -NH₂; -C₁₋₃alkyl; -C₁₋C₃alkoxy; -SC₁₋₃alkyl; -CN; -COC₁₋₃alkyl; and -CO₂C₁₋₃alkyl;
Z is H;
n is 0, Z' is -NH₂ and a double bond exists between position 3 and position 4; or a pharmaceutically acceptable salt, ester or salt of such ester of a compound on formula I.

2. A compound according to claim 1, wherein R is a C₁-C₆ branched or unbranched acyclic alkyl group.

3. A compound according to claim 1, wherein R is C₅-C₇ cyclic alkyl group.

4. A compound according to claim 1, wherein R is a benzyl group.

5. A compound according to any one of claims 1 to 4, wherein one of R' and R" is H and one of R' and R" is methyl.

6. A compound of any one of claims 1 to 5, wherein Ar is naphthyl.

7. A compound of any one of claims 1 to 6, wherein Ar is phenyl.

8. A compound of any one of claims 1 to 7, wherein Ar is unsubstituted.

9. A compound of any one of claims 1 to 7, wherein the substituents on Ar may be selected from F, Cl, CF₃ and NO₂.

10. A compound according to any one of claims 1 to 9 for use in a method of prophylaxis or treatment of cancer.

11. A pharmaceutical composition comprising a compound according to any one of claims 1 to 9 in combination with a pharmaceutically acceptable carrier, diluent or excipient.

12. A method of preparing a pharmaceutical composition comprising the step of combining a compound according to any one of claims 1 to 9 with a pharmaceutically acceptable excipient, carrier or diluent.

13. A process for the preparation of a compound of formula I according to claim 1, the process comprising reacting of a compound of formula (III): 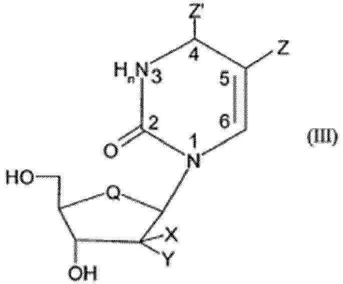 with a compound of formula (IV) 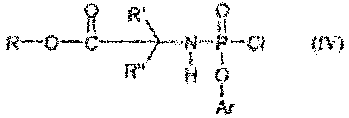 wherein Ar, n, Q, R, R', R", X, Y, Z and Z' have the meanings described in claim 1 and a double bond exists between position 3 and position 4.

## Patentansprüche

1. Chemische Verbindung mit der Formel I: 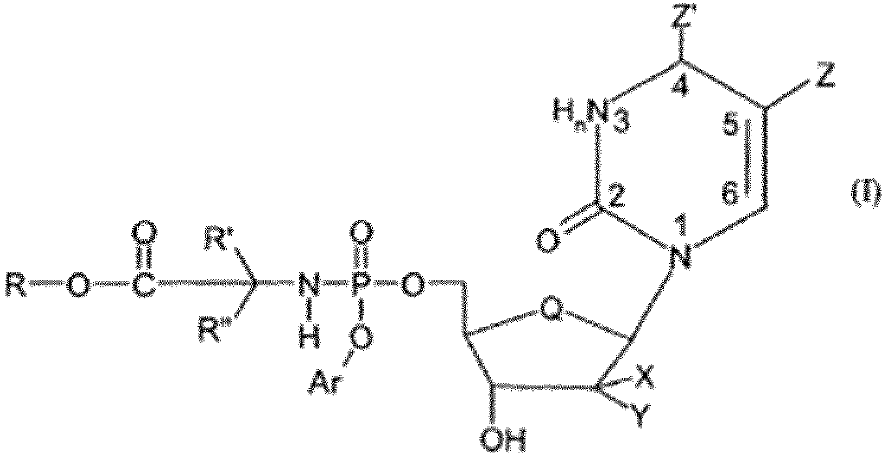 wobei:
R aus einer verzweigten oder unverzweigten acyclischen C₁-C₁₆-Alkylgruppe, einer cyclischen C₃-C₁₂-Alkylgruppe einer Phenylgruppe oder einer Naphthylgruppe und einer Benzylgruppe ausgewählt ist; und wobei
R unsubstituiert ist;
R' und R" unabhängig voneinander aus H, Methyl (-CH₃), Benzyl (-CH₂C₆H₅) und sekundärem Butyl (-CH₂CH(CH₃)₂) ausgewählt sind oder R' und R" zusammen mit dem C-Atom, an das sie gebunden sind, einen C₅₋₆-Ring bereitstellen;
Q -O- ist;
X und Y beide F sind;
Ar Naphthyl oder Phenyl ist; und
ein, zwei, drei oder vier Substituenten, die gleich oder unterschiedlich sein können, an Ar vorhanden sein können und aus der Gruppe bestehend aus Halogen, -NO₂; -NH₂; -C₁₋₃-Alkyl; -C₁-C₃-Alkoxy; -SC₁₋₃-Alkyl; -CN; -COC₁₋₃-Alkyl; und -CO₂C₁₋₃-Alkyl ausgewählt sind;
Z H ist;
n 0 ist, Z'-NH₂ ist und eine Doppelbindung zwischen Position 3 und Position 4 besteht; oder
pharmazeutisch verträgliches/r Salz, Ester oder Salz eines derartigen Esters einer Verbindung der Formel I.

2. Verbindung nach Anspruch 1, wobei R eine verzweigte oder unverzweigte acyclische C₁-C₆-Alkylgruppe ist.

3. Verbindung nach Anspruch 1, wobei R eine cyclische C₅-C₇-Alkylgruppe ist.

4. Verbindung nach Anspruch 1, wobei R eine Benzylgruppe ist.

5. Verbindung nach einem der Ansprüche 1 bis 4, wobei entweder R' oder R" H ist und entweder R' oder R" Methyl ist.

6. Verbindung nach einem der Ansprüche 1 bis 5, wobei Ar Naphthyl ist.

7. Verbindung nach einem der Ansprüche 1 bis 6, wobei Ar Phenyl ist.

8. Verbindung nach einem der Ansprüche 1 bis 7, wobei Ar unsubstituiert ist.

9. Verbindung nach einem der Ansprüche 1 bis 7, wobei die Substituenten an Ar aus F, Cl, CF₃ und NO₂ ausgewählt sein können.

10. Verbindung nach einem der Ansprüche 1 bis 9 zur Verwendung in einem Verfahren zur Prophylaxe oder Behandlung von Krebs.

11. Pharmazeutische Zusammensetzung, umfassend eine Verbindung nach einem der Ansprüche 1 bis 9 in Kombination mit einem pharmazeutisch verträglichen Träger, Verdünnungsmittel oder Hilfsstoff.

12. Verfahren zum Herstellen einer pharmazeutischen Zusammensetzung, umfassend den Schritt des Kombinierens einer Verbindung nach einem der Ansprüche 1 bis 9 mit einem pharmazeutisch verträglichen Hilfsstoff, Träger oder Verdünnungsmittel.

13. Verfahren zum Herstellen einer Verbindung der Formel I nach Anspruch 1, wobei das Verfahren das Reagieren einer Verbindung der Formel (III): 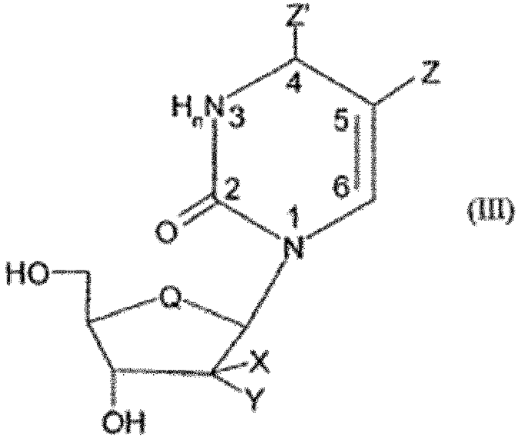 mit einer Verbindung der Formel (IV) 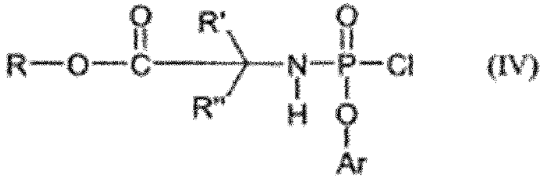 umfasst, wobei Ar, n, Q, R, R', R", X, Y, Z und Z' die in Anspruch 1 beschriebenen Bedeutungen aufweisen und eine Doppelbindung zwischen Position 3 und Position 4 besteht.

## Revendications

1. Composé chimique représenté par la formule I: 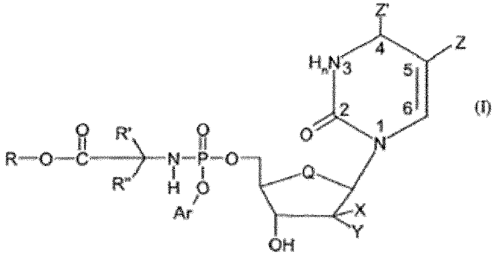 dans laquelle :
R est choisi parmi un groupe alkyle acyclique ramifié ou non ramifié en C₁ à C₁₆, un groupe alkyle cyclique en C₃ à C₁₂, un groupe phényle ou un groupe naphtyle et un groupe benzyle ; et dans laquelle R est non substitué ;
R' et R" sont indépendamment choisis parmi un atome H, un groupe méthyle (-CH₃), un groupe benzyle (-CH₂C₆H₅) et un groupe butyle secondaire (-CH₂CH(CH₃)₂) ou, R' et R" conjointement avec l'atome C auquel ils sont liés, donnent un noyau en C₅ à C₆ ;
Q représente -O- ;
X et Y représentent tous les deux un atome F ;
Ar représente un groupe naphtyle ou phényle ; et un, deux, trois ou quatre substituants, qui peuvent être identiques ou différents, peuvent être présents sur le groupe Ar et sont choisis dans le groupe comprenant un atome d'halogène, un groupe -NO₂; un groupe -NH₂; un groupe -(C₁ à C₃)alkyle ; un groupe -(C₁ à C₃)alcoxy ; un groupe -S(C₁ à C₃)alkyle ; un groupe -CN; un groupe -CO(C₁ à C₃)alkyle ; et un groupe -CO₂(C₁ à C₃)alkyle ;
Z représente un atome H ;
n vaut 0, Z' représente un groupe -NH₂ et une double liaison existe entre la position 3 et la position 4; ou sel, ester ou sel de cet ester pharmaceutiquement acceptable d'un composé de formule I.

2. Composé selon la revendication 1, R représentant un groupe alkyle acyclique ramifié ou non ramifié en C₁ à C₆.

3. Composé selon la revendication 1, R représentant un groupe alkyle cyclique en C₅ à C₇.

4. Composé selon la revendication 1, R représentant un groupe benzyle.

5. Composé selon l'une quelconque des revendications 1 à 4, l'un des groupes R' et R" représentant un atome H et l'un des groupes R' et R" représentant un groupe méthyle.

6. Composé selon l'une quelconque des revendications 1 à 5, Ar représentant un groupe naphtyle.

7. Composé selon l'une quelconque des revendications 1 à 6, Ar représentant un groupe phényle.

8. Composé selon l'une quelconque des revendications 1 à 7, Ar étant non substitué.

9. Composé selon l'une quelconque des revendications 1 à 7, lesdits substituants sur le groupe Ar pouvant être choisis parmi un atome F, un atome Cl, un groupe CF₃ et un groupe NO₂.

10. Composé selon l'une quelconque des revendications 1 à 9 pour utilisation dans une méthode de prophylaxie ou de traitement d'un cancer.

11. Composition pharmaceutique comprenant un composé selon l'une quelconque des revendications 1 à 9 en combinaison avec un vecteur, diluant ou excipient pharmaceutiquement acceptable.

12. Procédé de préparation d'une composition pharmaceutique comprenant l'étape de combinaison d'un composé selon l'une quelconque des revendications 1 à 9 avec un excipient, vecteur ou diluant pharmaceutiquement acceptable.

13. Procédé de préparation d'un composé de formule I selon la revendication 1, le procédé comprenant la réaction d'un composé de formule (III) : 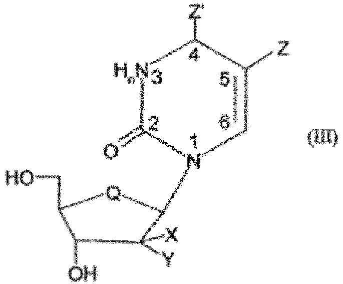 avec un composé de formule (IV) 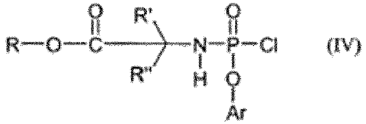 dans lesquelles Ar, n, Q, R, R', R", X, Y, Z et Z' ont les significations décrites dans la revendication 1 et une double liaison existe entre la position 3 et la position 4.
